(19) Europäisches Patentamt
European Patent Office
Office européen des brevets

(11) EP 4 644 874 A1

(12) **EUROPEAN PATENT APPLICATION**
published in accordance with Art. 153(4) EPC

(43) Date of publication:
**05.11.2025 Bulletin 2025/45**

(21) Application number: **23912043.9**

(22) Date of filing: **25.12.2023**

(51) International Patent Classification (IPC):
***G01N 21/78*** *(2006.01)*

(52) Cooperative Patent Classification (CPC):
**G01N 21/78**

(86) International application number:
**PCT/JP2023/046400**

(87) International publication number:
**WO 2024/143263 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.12.2022 JP 2022209083**

(71) Applicant: **FUJIFILM Corporation**
**Tokyo 106-8620 (JP)**

(72) Inventors:
- **SHIROKANE, Kenji**
  **Ashigarakami-gun, Kanagawa 258-8577 (JP)**
- **NAKATA, Hiyoku**
  **Ashigarakami-gun, Kanagawa 258-8577 (JP)**
- **NAKABAYASHI, Jun**
  **Ashigarakami-gun, Kanagawa 258-8577 (JP)**

(74) Representative: **HGF**
**HGF Limited**
**1 City Walk**
**Leeds LS11 9DX (GB)**

(54) **FLUORESCENCE INTENSITY ENHANCING AGENT, METHOD FOR ENHANCING FLUORESCENCE INTENSITY OF FLUORESCENTLY LABELED TARGET BIOLOGICAL MATERIAL, AND FLUORESCENCE DETECTION KIT**

(57) A fluorescence intensity enhancer containing a water-soluble compound having a melting point of 50°C or higher, a fluorescence intensity enhancing method of a fluorescently labeled target biological substance using the fluorescence intensity enhancer, and a kit for fluorescence detection containing the fluorescence intensity enhancer.



Processed by Luminess, 75001 PARIS (FR)

## Description

### BACKGROUND OF THE INVENTION

1. Field of the Invention

The present invention relates to a fluorescence intensity enhancer, a fluorescence intensity enhancing method of a fluorescently labeled target biological substance, and a kit for fluorescence detection.

2. Description of the Related Art

A bioimaging technique for analyzing the dynamics, function, or the like of a substance constituting a living body, such as a biomolecule, a cell, or a tissue is known. For the purpose of observing or analyzing the dynamics, interaction, concentration change, function change, or the like of a substance such as a protein, a nucleic acid, or a low-molecular-weight compound, or a complex thereof, fluorescence imaging is frequently used in which information is obtained by visualizing with a fluorescence label using a fluorescent dye-labeled biomolecule (a fluorescent dye-labeled antibody or the like) obtained by labeling a biomolecule (an antibody or the like) that is bindable to a target substance to be detected with a fluorescent dye. The fluorescence imaging using the fluorescent dye-labeled biomolecule is used, for example, in Western blotting in which a specimen derived from a living body is transferred onto a membrane and a specific protein is detected from a protein mixture, cell staining in which the dynamics of a target substance in a cell is observed in a state of the cell, and the like.

In the fluorescence imaging, an organic fluorescent dye molecule is generally used. The brightness (fluorescence intensity) is increased generally to a desired level by using a fluorescent dye-labeled biomolecule in which a plurality of organic fluorescent dye molecules are bonded to one biomolecule. However, since most of the organic dyes exhibiting fluorescence, such as a cyanine dye and a rhodamine dye, have an aromatic chromophore having high planarity, an interaction between the dyes easily occurs, and as a result, a decrease in the fluorescence intensity after labeling due to an interaction such as self-association between the dyes easily occurs. In addition, as the number of molecules (degree of fluorescence labeling: DOL) of the organic fluorescent dye per one molecule of the biological molecule increases, the fluorescence intensity due to self-association or the like tends to further decrease.

In addition, a technique of performing fluorescence imaging by allowing coexistence of a fluorescent dye-labeled biomolecule and another molecule is known. For example, JP2016-534147A describes that a conjugate compound of a chlorotoxin peptide and a cyanine dye is used for imaging applications of tumors, and that the composition used for tableting the conjugate compound contains mannitol. JP2022-501402A describes that a method of administering an $^{89}$Zr-labeled antigen binding construct is used for imaging applications of tumors, and describes that the composition used for tableting the antigen binding construct contains sucrose. JP1993-133957A(JP-H05-133957A) describes dried mammalian cells that are useful as a control or standard in an immunoassay, which are obtained by fixing cells with a fixative, reducing the cells with a Schiff base reducing agent, and drying the cells in the presence of trehalose as a compound for stabilizing a membrane.

### SUMMARY OF THE INVENTION

The present inventors have repeatedly studied, as a technique of suppressing self-association between fluorescent dyes in a fluorescent dye-labeled biomolecule, a technique of suppressing the self-association by adjusting the chemical structure of an organic fluorescent dye molecule to be introduced into the fluorescent dye-labeled biomolecule. However, in this technique, self-association can be suppressed by an organic fluorescent dye molecule having a specific chemical structure, but the technique has no general-purpose properties. Therefore, in fluorescence imaging, there is a demand for the development of a technique that suppresses self-association and enhances fluorescence intensity by acting on a fluorescently labeled target biological substance with a fluorescent dye-labeled biomolecule, as a technique that can be used more generally.

That is, an object of the present invention is to provide a fluorescence intensity enhancer capable of enhancing the fluorescence intensity of a fluorescently labeled target biological substance by acting on the fluorescently labeled target biological substance. In addition, another object of the present invention is to provide a fluorescence intensity enhancing method of a fluorescently labeled target biological substance using the fluorescence intensity enhancer, and a kit for fluorescence detection containing the fluorescence intensity enhancer.

As a result of repeated studies, the present inventors have found that the fluorescence intensity of a fluorescently labeled target biological substance can be enhanced by allowing a water-soluble compound having a melting point of 50°C or higher, such as mannitol, sucrose, or trehalose, to act on the fluorescently labeled target biological substance.

That is, the above-described objects of the present invention have been achieved by the following methods.

[1] A fluorescence intensity enhancer comprising a water-soluble compound having a melting point of 50°C or higher.
[2] The fluorescence intensity enhancer according to [1], in which a content of the water-soluble compound is 4.5% by mass or more.
[3] The fluorescence intensity enhancer according to [1] or [2], in which the water-soluble compound does not contain a salt structure.
[4] The fluorescence intensity enhancer according to any one of [1] to [3], in which the water-soluble compound is a polyol compound or a betaine compound.
[5] The fluorescence intensity enhancer according to any one of [1] to [4], in which the content of the water-soluble compound is 20% by mass or more.
[6] The fluorescence intensity enhancer according to any one of [1] to [5], in which the melting point is 80°C or higher.
[7] The fluorescence intensity enhancer according to any one of [1] to [6], in which the fluorescence intensity enhancer is in a solution form.
[8] The fluorescence intensity enhancer according to any one of [1] to [7], in which the fluorescence intensity enhancer is used for fluorescence imaging.
[9] A fluorescence intensity enhancing method of a fluorescently labeled target biological substance, the fluorescence intensity enhancing method comprising allowing the fluorescence intensity enhancer according to any one of [1] to [8] to act on a fluorescently labeled target biological substance.
[10] A fluorescence intensity enhancing method of a fluorescently labeled target biological substance, the fluorescence intensity enhancing method comprising allowing the fluorescence intensity enhancer according to any one of [1] to [8] to act on a fluorescently labeled target biological substance to enhance a fluorescence intensity from a fluorescence label.
[11] A kit for fluorescence detection, comprising:

(a) a fluorescence intensity enhancer containing a water-soluble compound having a melting point of 50°C or higher; and
(b) a reagent for producing a fluorescent dye-labeled biological molecule, which contains a fluorescent dye, or a fluorescent dye-labeled biomolecule.

In the present invention, in a case where there is a plurality of substituents, linking groups, structural units, or the like (hereinafter, referred to as substituents or the like), which are represented by a specific symbol or Formula, or in a case where a plurality of substituents or the like are regulated at the same time, the substituents or the like may be the same or different from each other, unless otherwise specified. The same applies to the regulation of the number of substituents or the like. In addition, in a case where a plurality of substituents or the like come close to each other (particularly in a case where they are adjacent to each other), they may be linked to each other to form a ring, unless otherwise specified. In addition, unless otherwise specified, rings such as an alicyclic ring, an aromatic ring, and a heterocyclic ring may be fused to form a fused ring.

For example, in the present invention, a structure represented by General Formula (I) described below means that n (n is an integer of 2 or more) pieces of structures represented by General Formula (i) are connected. In this case, the n pieces of structures represented by General Formulae (i) may be the same or different from each other. It is noted that $X^1$ to $X^3$ in General Formula (i) respectively have the same meanings as $X^1$ to $X^3$ in General Formula (I) described later. The same applies to a structure parenthesized in $( )_s$, a structure parenthesized in ( )t, a structure parenthesized in $( )_u$, a structure parenthesized in $( )_m$, a structure parenthesized in $( )_{n1}$, a structure parenthesized in $( )_{na}$, and a structure parenthesized in $( )_{nb}$, where s pieces of structures may be the same or different from each other, t pieces of structures may be the same or different from each other, u pieces of structures may be the same or different from each other, m pieces of structures may be the same or different from each other, n1 pieces of structures may be the same or different from each other, na pieces of structures may be the same or different from each other, and nb pieces of structures may be the same or different from each other.

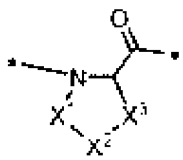

General Formula (i)

In the present invention, in a case where an E type double bond and a Z type double bond are present in a molecule, the double bond may be any one thereof or may be a mixture thereof, unless otherwise specified. In addition, unless otherwise specified, in a case where a chiral carbon atom or a chiral center is present in a compound, the steric conformation may be any of R or S in the R-S notation, and a mixture thereof may be may be allowed.

In the present invention, the denotation of a compound or substituent is meant to include not only the compound itself but also a salt thereof, and an ion thereof. For example, the dissociable anionic group such as the carboxy group, the sulfo group, and the phosphono group ($-P(=O)(OH)_2$) may have an ionic structure by a hydrogen ion being dissociated therefrom, or they may have a salt structure. That is, in the present invention, the "carboxy group" is meant to include a group of an ion or salt of a carboxylic acid, the "sulfo group" is meant to include a group of an ion or salt of a sulfonic acid, and the "phosphono group" is meant to include a group of an ion or salt of a phosphonic acid. The monovalent or polyvalent cation in forming the salt structure is not particularly limited. Examples thereof include an inorganic cation and an organic cation, and specific examples thereof include alkali metal cations such as $Na^+$, $Li^+$, and $K^+$, alkaline earth metal cations such as $Mg^{2+}$, $Ca^{2+}$, and $Ba^{2+}$, and organic ammonium cations such as a trialkylammonium cation and a tetraalkylammonium cation.

In a case of a salt structure, the type of the salt may be one type, two or more types thereof may be mixed, a salt-type group and a group having a free acid structure may be mixed in a compound, and a compound having a salt structure and a compound having a free acid structure compound may be mixed.

All of the compounds described in the present invention and the present specification are neutral compounds. In the present invention and the present specification, the compound being neutral means that the compound is electrically neutral. Specifically, the charge of the compound as a whole is adjusted to be 0 by a group having a charge or by a counterion in the compound. For example, in the cyanine dye represented by General Formula ($\alpha$), which is exemplified as an example of the dye that constitutes the phosphor moiety, the formal charge of the nitrogen atom to which $R^{42}$ is bonded is +1, and the dissociable group such as a sulfo group in another structure of the cyanine dye or the fluorescent dye (F) has an ionic structure such as a sulfonate ion to be paired with this formal charge, whereby the fluorescent dye (F) is to be a compound having a charge of 0 as a whole.

In each general formula pertaining to the cyanine dye, which is defined in the present invention, the positive charge possessed by the compound is specified and indicated, for convenience, as a structure of a specific nitrogen atom. However, since the cyanine dye defined in the present invention has a conjugated system, another atom other than the nitrogen atom actually may be capable of being positively charged, and thus any cyanine dye capable of adopting a structure represented by each general formula as one of the chemical structures is included in the cyanine dye represented by each general formula. This also applies to the negative charge.

In addition, it is meant to include those in which a part of the structure is changed within the scope that does not impair the effect of the present invention. Furthermore, it is meant that a compound, which is not specified to be substituted or unsubstituted, may have any substituent within the scope that does not impair the effect of the present invention. The same applies to a substituent (for example, a group represented by "alkyl group", "methyl group", "methyl") and a linking group (for example, a group represented by "alkylene group", "methylene group", "methylene"). Among such substituents, a preferred substituent in the present invention is a substituent selected from a substituent group T described later.

In the present invention, in a case where the number of carbon atoms of a certain group is specified, this number of carbon atoms means the number of carbon atoms of the entire group thereof unless otherwise specified in the present invention or the present specification. That is, in a case where this group has a form of further having a substituent, it means the total number of carbon atoms, to which the number of carbon atoms of this substituent is included.

In addition, in the present invention, the numerical range indicated by using "to" means a range including the numerical values before and after "to" as the lower limit value and the upper limit value, respectively.

By allowing the fluorescence intensity enhancer according to the embodiment of the present invention on the fluorescently labeled target biological substance, the fluorescence intensity of a fluorescently labeled target biological substance can be enhanced.

In addition, the fluorescence intensity enhancing method of a fluorescently labeled target biological substance according to the embodiment of the present invention can enhance the fluorescence intensity of the fluorescently labeled target biological substance.

In addition, the kit for fluorescence detection according to the embodiment of the present invention can enhance the fluorescence intensity of the fluorescently labeled target biological substance.

## DESCRIPTION OF THE PREFERRED EMBODIMENTS

<<Fluorescence intensity enhancer>>

The fluorescence intensity enhancer according to the embodiment of the present invention contains a water-soluble compound having a melting point of 50°C or higher. Hereinafter, the water-soluble compound having a melting point of 50°C or higher will also be referred to as a "water-soluble compound (W)".

In the present invention, the "water-soluble compound" means a compound having a solubility of 1 g/100 mL-$H_2O$ or more in water at 25°C, that is, a compound that dissolves 1 g or more in 100 mL of water at 25°C. Here, "dissolve" means that the liquid becomes uniformly transparent.

The solubility of the water-soluble compound (W) in water at 25°C is preferably 5 to 500 g/100 mL-$H_2O$, more preferably 10 to 400 g/100 mL-$H_2O$, and still more preferably 30 to 300 g/100 mL-$H_2O$.

In the present invention, the melting point of a compound is a value obtained by a method described in Examples later, using a simultaneous thermogravimetric and differential thermal measurement device. It is noted that as long as the melting point of the compound is measured, the decomposition of the water-soluble compound (W) may occur at a low temperature as compared with the melting of the water-soluble compound (W).

The melting point of the water-soluble compound (W) is 50°C or higher, and from the viewpoint of further enhancing the fluorescence intensity, the melting point is preferably 80°C or higher and more preferably 90°C or higher. The upper limit value is not particularly limited as long as the effect of the present invention is exhibited, and it can be set to, for example, 600°C or lower and is preferably 500°C or lower and more preferably 400°C or lower. That is, the melting point of the water-soluble compound (W) is preferably 50°C to 600°C, more preferably 80°C to 500°C, and still more preferably 90°C to 400°C.

It is noted that in a case where the fluorescence intensity enhancer according to the embodiment of the present invention contains two or more types of water-soluble compounds (W), the melting point of the water-soluble compound (W) is the lowest value among the melting points of the water-soluble compounds (W) contained in the fluorescence intensity enhancer according to the embodiment of the present invention.

By allowing the fluorescence intensity enhancer according to the embodiment of the present invention to act on a fluorescently labeled target biological substance, the water-soluble compound having a melting point of 50°C or higher functions as an active ingredient and the fluorescence intensity of the fluorescently labeled target biological substance can be enhanced. The reason is not clear, but it is considered that the water-soluble compound having a melting point of 50°C or higher, which is contained in the fluorescence intensity enhancer according to the embodiment of the present invention, is interposed between the fluorescent dye-labeled biomolecules in the fluorescently labeled target biological substance and/or between the fluorescent dyes, and thus the interaction between the fluorescent dyes is suppressed, whereby the quenching due to the association of the fluorescent dyes is suppressed, and the fluorescence intensity can be enhanced. On the other hand, in case of a water-soluble compound having a melting point of lower than 50°C, the water-soluble compound cannot be interposed in the form of a film to suppress the interaction between the fluorescent dyes, and the effect of enhancing the fluorescence intensity cannot be obtained. In addition, in a case of a compound that is not a water-soluble compound, such as an oil-soluble compound, phase separation occurs in a case of acting on a fluorescently labeled target biological substance, and the compound cannot be interposed between the fluorescent dyes to suppress the interaction between the fluorescent dyes, and the effect of enhancing the fluorescence intensity cannot be obtained.

The water-soluble compound (W) preferably contains a water-soluble group, and for example, preferably contains at least one of a monovalent water-soluble group such as a hydroxy group, a carboxy group, a sulfo group, a phosphono group, a phosphonooxy group, an amino group, an ammonio group, or a phosphonio group, or a divalent water-soluble group such as an ether bond or an amide bond.

As described above, the carboxy group, the sulfo group, the phosphono group, and the phosphonooxy group may have an ionic structure in which a hydrogen ion is dissociated, or may have a salt structure.

The water-soluble compound (W) is more preferably a compound having a betaine structure, which contains at least one anionic group of a carboxy group, a sulfo group, a phosphono group, or a phosphonooxy group, and at least one cationic group of an ammonio group or a phosphonio group, or a compound containing a hydroxy group.

The water-soluble compound (W) may be a low-molecular-weight compound or a polymer compound. The molecular weight of the water-soluble compound (W) is preferably 10 to 300,000, more preferably 20 to 200,000, and still more preferably 30 to 100,000. In a case where the water-soluble compound is a compound having a repeating structure, such as a polymer compound, the molecular weight means a weight-average molecular weight.

From the viewpoint of further enhancing the fluorescence intensity, it is preferable that the water-soluble compound (W) does not contain a salt structure. The salt structure refers to a compound consisting of an anionic group and a cationic group, and means a structure in which the anionic group and the cationic group are present as separate molecules. Specifically, the description regarding the salt structure at the beginning can be applied.

It is noted that a betaine structure having an anionic group and a cationic group in the same molecule does not correspond to the salt structure.

By using the fluorescence intensity enhancer according to the embodiment of the present invention, the fluorescence intensity of the fluorescently labeled target biological substance is enhanced, and the fluorescence intensity in a region other than the fluorescence region exhibited by the fluorescently labeled target biological substance may be increased. From the viewpoint of suppressing an increase in fluorescence intensity in a region other than the fluorescence region exhibited by the fluorescently labeled target biological substance, the water-soluble compound (W) is preferably a polyol compound or a betaine compound.

The polyol compound means a compound having two or more hydroxy groups in the molecule. Examples of the polyol compound include sugar compounds such as glucose, sucrose, maltose, lactose, trehalose, ribitol, sorbitol, mannitol, maltitol, lactitol, xylitol, fruit sugar (fructose), 1-kestose, nystose trihydrate, fucose, dulcitol, galactooligosaccharide, 4'-galactosyl lactose, isomalto-oligosaccharide, lactulose, palatinit, palatinose monohydrate, raffinose pentahydrate, arabinose, dihydroxyacetone dimer, galactose, glyceryl aldehyde dimer, mannose, ribose, xylose, lactosyl fructoside, erythritol, dulcoside A, isosteviol, rebaudioside A, rebaudioside B, rebaudioside C, rebaudioside D, rebaudioside F, rubusoside, stevioside, 1-deoxynojirimycin, fucoidan, rhamnose, threose, arabinose, lyxose, allose, altrose, gulose, idose, talose, 1,3-dihydroxyacetone, erythrose, xylulose, ribulose, psicose, sorbose, tagatose, polysucrose, and fructooligosaccharide, and polyoxyalkylene glycol. It is noted that the sugar compound may be any of a monosaccharide, a polysaccharide in which two or more sugars are bonded, a sugar alcohol, or a chemically modified sugar obtained by copolymerizing a sugar with epichlorohydrin or the like.

The betaine compound means a compound having a betaine structure, preferred examples thereof include a compound having a betaine structure, which contains at least one anionic group of a carboxy group, a sulfo group, a phosphono group, or a phosphonooxy group, and at least one cationic group of an ammonio group or a phosphonio group, and a compound having a betaine structure, which contains a carboxy group as the anionic group and an ammonio group as the cationic group is more preferable.

Examples of the betaine compound include trimethylglycine.

In addition, preferred examples of the water-soluble compound (W) also include water-soluble polymers such as polyvinylpyrrolidone, in addition to the above-described polyol compound and betaine compound.

The content of the water-soluble compound (W) is not particularly limited as long as the effect of the present invention is exhibited, and for example, it can be set to 1% by mass or more and it is preferably 2.5% by mass or more, and from the viewpoint of further enhancing the fluorescence intensity, it is more preferably 4.5% by mass or more, still more preferably 9% by mass or more, even still more preferably 12% by mass or more, yet even still more preferably 15% by mass or more, particularly preferably 20% by mass or more, and most preferably 25% by mass or more. The upper limit value thereof is not particularly limited, and may be 100% by mass or less.

It is noted that the content of the water-soluble compound (W) means the content of the water-soluble compound (W) in the fluorescence intensity enhancer of the present invention. Furthermore, in a case of allowing the fluorescence intensity enhancer according to the embodiment of the present invention in a solution form to act on a fluorescently labeled target biological substance, it is preferable that the content of the water-soluble compound (W) in the fluorescence intensity enhancer (solution form) according to the embodiment of the present invention satisfies the content of the water-soluble compound (W) from the viewpoint of further enhancing the fluorescence intensity.

In addition, the water-soluble compound (W) used in the present invention is preferably colorless, and it is more preferably a compound that does not absorb excitation light of a fluorescent dye.

The fluorescence intensity enhancer according to the embodiment of the present invention may be in either a solid or a solution form.

Examples of the solid form of the fluorescence intensity enhancer according to the embodiment of the present invention include a fine particle powder, a freeze-dried powder, and the like of the fluorescence intensity enhancer according to the embodiment of the present invention.

In a case where the fluorescence intensity enhancer according to the embodiment of the present invention is in a solid form, components such as a buffering agent and a surfactant constituting a buffer solution described later may be further contained in a solid form. It is noted that a part of the fluorescence intensity enhancer according to the embodiment of the present invention may be present in an aqueous medium such as a buffer solution described later in a solution form, and the remainder may be present therein in a solid form.

Examples of the solution form of the fluorescence intensity enhancer according to the embodiment of the present invention include a solution obtained by dissolving the fluorescence intensity enhancer according to the embodiment of the present invention in an aqueous medium.

The aqueous medium may contain an organic solvent in addition to water within a range in which the effects of the present invention are exhibited. Examples of the organic solvent which may be contained include water-miscible organic solvents such as methanol, ethanol, dimethyl sulfoxide, dimethylformamide, tetrahydrofuran, acetonitrile, and acetic acid.

In addition, the aqueous medium may contain an additive within a range in which the effects of the present invention are exhibited. Examples of the additives which may be contained include a compound contained in a buffer, such as a buffering agent such as tris-buffered saline (TBS), phosphate-buffered saline (PBS), tris-acetate EDTA (TAE), tris-borate EDTA (TBE), tris EDTA (TE), and Good's Buffer (for example, available from Nippon Gene Co., Ltd. and Dojindo Laboratories), a surfactant such as sodium dodecyl sulfate, Triton X (trade name, polyoxyethylene (10) octylphenyl ether, for example, available from Sigma-Aldrich Co., LLC), or Tween-20 (trade name, polyoxyethylene sorbitan monolaurate, for example, available from FUJIFILM Wako Pure Chemical Corporation). In addition, a blocking agent such as bovine serum albumin (BSA) may be contained. That is, in a case where the fluorescence intensity enhancer according to the embodiment of the present invention is in a solution form, examples thereof include a buffer solution such as TBS, PBS, Tween-20-containing TBS (TBS-T), Tween-20-containing PBS (PBS-T), TAE, TBE, TE, or Good's Buffer, which contains the water-soluble compound (W).

The form of the fluorescence intensity enhancer according to the embodiment of the present invention is

preferably a solution form, and more preferably a form of a buffer solution containing the water-soluble compound (W).

The fluorescence intensity enhancer according to the embodiment of the present invention is preferably used for fluorescence imaging applications.

The fluorescence imaging means bioimaging in which a targeted biological substance (also referred to as a "target biological substance" in the present invention) is visualized and observed by fluorescence labeling using a fluorescent dye-labeled biomolecule. Specifically, the fluorescence of a target biological substance (in the present invention, also referred to as a "fluorescently labeled target biological substance") labeled with a fluorescent dye-labeled biomolecule is observed. Details of the fluorescent dye used for the fluorescent dye-labeled biomolecule, the fluorescent dye-labeled biomolecule, and the fluorescence detection using the fluorescent dye-labeled biomolecule will be described later.

The fluorescence imaging application is not particularly limited, and the present invention can be applied to the fluorescence imaging applications that is generally used. For example, the present invention can be used for applications such as Western blotting (hereinafter, also abbreviated as WB), multicolor WB, cell staining, and dot blotting.

In particular, in WB, a protein band can be detected by separating a target biological substance (protein) by the sodium dodecyl sulfate-polyacrylamide gel electrophoresis (SDS-PAGE) method according to a difference in molecular mass, then transferring the protein to a membrane, specifically binding a fluorescent dye-labeled antibody to the protein transferred to the membrane to obtain a fluorescently labeled target biological substance, and then evaluating the fluorescently labeled target biological substance. In WB, after obtaining the fluorescently labeled target biological substance, the fluorescence intensity can be further increased by drying the membrane and evaluating the membrane. Therefore, the band of the protein can be detected even in a case where the amount of the protein is small.

In the WB, since by allowing the fluorescence intensity enhancer according to the embodiment of the present invention to act on the fluorescently labeled target biological substance, the effect of quenching due to the interaction between the fluorescent dyes in the fluorescently labeled target biological substance can be suppressed in a case where the membrane is dried, and the fluorescence intensity can be enhanced, it is considered that a band with a lower amount of protein can be detected with higher sensitivity as compared with that in the ordinary WB.

It is noted that the effect of enhancing the fluorescence intensity by the fluorescence intensity enhancer according to the embodiment of the present invention is not limited to a case where the membrane is dried, and the effect is also obtained in a case where the membrane is observed without being dried.

<<Fluorescence intensity enhancing method of fluorescently labeled target biological substance>>

The fluorescence intensity enhancing method of a fluorescently labeled target biological substance according to the embodiment of the present invention is a method including allowing the fluorescence intensity enhancer according to the embodiment of the present invention to act on a fluorescently labeled target biological substance.

Specifically, allowing the fluorescence intensity enhancer according to the embodiment of the present invention to act on the fluorescently labeled target biological substance is preferably allowing the fluorescence intensity enhancer according to the embodiment of the present invention to act on the fluorescently labeled target biological substance to enhance the fluorescence intensity from the fluorescence label.

It is noted that in a case of acting on a fluorescently labeled target biological substance, it is preferable that the fluorescence intensity enhancer according to the embodiment of the present invention is allowed to act as the above-described solution.

To enhance the fluorescence intensity by allowing the fluorescence intensity enhancer according to the embodiment of the present invention to act on a fluorescently labeled target biological substance, the fluorescence intensity enhancer according to the embodiment of the present invention may be allowed to act after the fluorescently labeled target biological substance is obtained and before the fluorescence detection is performed, such that a function of suppressing an interaction between fluorescent dyes in the fluorescently labeled target biological substance is developed. For example, it is preferable that the fluorescence intensity enhancer according to the embodiment of the present invention is allowed to act in a washing step of the fluorescently labeled target biological substance, and it is more preferable that the fluorescently labeled target biological substance is washed with the buffer solution containing the above-described water-soluble compound (W).

For example, in the WB, in a step of obtaining a fluorescently labeled target biological substance by binding a fluorescent dye-labeled antibody to a protein transferred to a membrane by an antibody reaction and then washing the fluorescently labeled target biological substance with a washing buffer solution, it is preferable that the fluorescently labeled target biological substance is washed with a buffer solution containing the above-described water-soluble compound (W).

As the washing buffer solution, a washing buffer solution generally used for WB can be used without particular limitation, and examples thereof include washing buffer solutions such as TBS, PBS, TBS-T, PBS-T, TAE, TBE, TE, and Good's Buffer.

In addition, examples of the buffer solution containing the water-soluble compound (W) include a buffer solution such as TBS, PBS, TBS-T, PBS-T, TAE, TBE, TE, or Good's Buffer, which contains the water-soluble compound (W).

It is noted that as washing of the fluorescently labeled target biological substance, a plurality of times of washing by replacing the washing buffer solution are generally performed, but by performing washing with the buffer solution containing the water-soluble compound (W) at least as the last washing, the fluorescence intensity from the fluorescent label can be enhanced.

The washing conditions such as the washing time (shaking time) and the content of the water-soluble compound (W) in the buffer solution in the washing step with the buffer solution containing the water-soluble compound (W) are not particularly limited as long as the effect of enhancing the fluorescence intensity is obtained. For example, the washing time (shaking time) can be set to 5 to 15 minutes, and the description of the content of the water-soluble compound (W) in the above-described fluorescence intensity enhancer according to the embodiment of the present invention can be applied to the content of the water-soluble compound (W) in the buffer solution.

<<Kit for fluorescence detection>>

The kit for fluorescence detection according to the embodiment of the present invention is a kit containing the following (a) and (b).

(a) A fluorescence intensity enhancer according to the embodiment of the present invention.

(b) A reagent for producing a fluorescent dye-labeled biological molecule, which contains a fluorescent dye, or a fluorescent dye-labeled biomolecule.

The fluorescence intensity enhancer according to the embodiment of the present invention in the (a) may be in either the above-described solid or solution form.

In a case of being in a solid form, in addition to the (a), a buffer solution such as TBS, PBS, TBS-T, PBS-T, TAE, TBE, TE, or Good's Buffer may be contained, and a component (a buffering agent, a surfactant, or the like) other than the aqueous medium constituting the buffer solution such as TBS, PBS, TBS-T, PBS-T, TAE, TBE, TE, or Good's Buffer may be contained. In a case of containing a component (a buffering agent, a surfactant, or the like) other than the aqueous medium constituting the buffer solution such as TBS, PBS, TBS-T, PBS-T, TAE, TBE, TE, or Good's Buffer, it is preferable that the fluorescence intensity enhancer is allowed to act as a solution form by adding the aqueous medium thereto in a case of acting on a fluorescently labeled target biological substance.

In a case of being in a solution form, a buffer solution such as TBS, PBS, TBS-T, PBS-T, TAE, TBE, TE, or Good's Buffer is preferable, and Tween-20 may be separately added.

In a case where the (b) is a reagent for producing a fluorescent dye-labeled biomolecule containing a fluorescent dye, in addition to the fluorescent dye, other components used for producing a fluorescent dye-labeled biomolecule, and examples thereof include a biological substance (preferably an antibody) that binds to a fluorescent dye to produce a fluorescent dye-labeled biomolecule.

In a case where the (b) is a fluorescent dye-labeled biomolecule, the fluorescent dye-labeled biomolecule may function as either a primary antibody or a secondary antibody for the target biological substance to be applied. It is noted that in a case where the fluorescent dye-labeled biomolecule functions as a secondary antibody, a primary antibody against the target biological substance to be applied may be further contained.

The kit for fluorescence detection according to the embodiment of the present invention may be any kit as long as it is used for fluorescence detection of a fluorescently labeled target biological substance, and it is preferably used for fluorescence detection in fluorescence imaging applications.

The kit for fluorescence detection according to the embodiment of the present invention may further contain, in addition to the (a) and the (b), a protein extraction and treatment liquid, a gel, an electrode solution, a blotting agent, a blocking agent, a treatment liquid, a washing solution, a buffer solution, and the like, which are usually used for producing a fluorescently labeled target biological substance.

Hereinafter, a fluorescent dye used for a fluorescent dye-labeled biomolecule, a fluorescent dye-labeled biomolecule, and fluorescence detection using the fluorescent dye-labeled biomolecule will be described in detail.

<Fluorescent dye>

The fluorescent dye-labeled biomolecule on which the fluorescence intensity enhancer according to the embodiment of the present invention is allowed to act may be any biological substance labeled with a fluorescent dye.

As the fluorescent dye, a fluorescent dye commonly used for labeling a biological substance can be used without particular limitation. Examples thereof include a xanthene dye, a rhodamine dye, a coumarin dye, a cyanine dye, a pyrene

dye, an oxazine dye, a squarylium dye, a phthalocyanine dye, a porphyrin dye, a pyridyloxazole dye, and a pyrromethene dye.

Specific examples thereof include fluorescent compounds (compounds exhibiting fluorescence) described in WO2019/230963A, WO2021/100814A, WO2021/125295A, WO2021/215514A, WO2022/025210A, WO2022/191123A, JP2019-172826A, WO2019/230963A, JP2022-131357A, and the like.

In addition, a commercially available fluorescent dye can also be used without particular limitation. Examples thereof include Alexa Fluor 350, Alexa Fluor 405, Alexa Fluor 430, Alexa Fluor 488, Alexa Fluor 514, Alexa Fluor 532, Alexa Fluor 546, Alexa Fluor 555, Alexa Fluor 568, Alexa Fluor 594, Alexa Fluor 633, Alexa Fluor 635, Alexa Fluor 647, Alexa Fluor 660, Alexa Fluor 680, Alexa Fluor 700, Alexa Fluor 750, Alexa Fluor 790, DyLight 350, DyLight 405, DyLight 425Q, DyLight 488, DyLight 510-LS, DyLight 515-LS, DyLight 550, DyLight 594, DyLight 633, DyLight 650, DyLight 680, DyLight 730-B1, DyLight 747-B4, DyLight 800, DyLight 650-4xPEG, DyLight 800-4xPEG, and the like, all of which are trade names of products manufactured by Thermo Fisher Scientific Inc.; ATTO 390, ATTO 425, ATTO 465, ATTO 488, ATTO 495, ATTO 514, ATTO 520, ATTO 532, ATTO RHO6G, ATTO 540Q, ATTO 550, ATTO 565, ATTO RHO11, ATTO 580Q, ATTO 590, ATTO 594, ATTO 610, ATTO 612Q, ATTO 620, ATTO 633, ATTO RHO14, ATTO 647, ATTO 647N, ATTO 655, ATTO 665, ATTO 680, ATTO 700, ATTO 725, ATTO 740, and the like, all of which are trade names of products manufactured by ATTO-TEC GmbH; Cy3, Cy3B, Cy5, Cy5.5, Cy7, and the like, all of which are trade names of products manufactured by Global Life Science Technologies Japan, Inc.; and CF 350, CF 405S, CF 405M, CF 405L, CF 430, CF 440, CF 450, CF 488A, CF 503R, CF 514, CF 532, CF 535ST, CF 543, CF 550R, CF 555, CF 568, CF 570, CF 583, CF 583R, CF 594, CF 594ST, CF 597R, CF 620R, CF 633, CF 640R, CF 647, CF 660C, CF 660R, CF 680, CF 680R, CF 700, CF 750, CF 770, CF 790, CF 800, CF 820, CF 850, CF 870, and the like, all of which are trade names of products manufactured by Biotium, Inc.

The fluorescence intensity enhancer according to the embodiment of the present invention can more effectively enhance the fluorescence intensity of a biological substance labeled with a fluorescent dye having two or more phosphor moieties in one molecule. Preferred examples of the fluorescent dye having two or more phosphor moieties in one molecule include the following fluorescent dye (F).

<Fluorescent dye (F)>

The fluorescent dye (F) is a compound containing two or more phosphor moieties of which light absorption characteristics are equivalent to each other, where it is a compound in which the phosphor moieties adjacent to each other are each linked through a group having a structure represented by General Formula (I). The structure represented by General Formula (I) has a repeating unit (the repetition number is 2 or more) including a nitrogen-containing saturated 5-membered ring such as a ring structure derived from proline and functions as a linker that is rigid with respect to the two phosphor moieties linked by a group including a structure represented by General Formula (I), and thus it is possible to effectively suppress the quenching due to the intramolecular or intermolecular association of the phosphor moieties. Therefore, it is conceived that a fluorescent dye-labeled biomolecule obtained by using the fluorescent dye (F) can exhibit an excellent fluorescence intensity.

The fluorescent dye (F) may be a compound classified into a polymer or an oligomer.

In the present invention, the phrase "phosphor moieties of which light absorption characteristics are equivalent to each other" means those that satisfy a relationship in which a difference in the maximum absorption wavelength between the respective phosphor moieties in the absorption spectra is within 15 nm.

In the present invention, the compound is preferably such that all the phosphor moieties contained in the fluorescent dye (F) satisfy a relationship in which a difference between the maximum absorption wavelength on the lowest wavelength side and the maximum absorption wavelength on the highest wavelength side among the maximum absorption wavelengths in the absorption spectra of the respective phosphor moieties is within 15 nm

As the compound having two phosphor moieties in the fluorescent dye, a compound that causes a fluorescence resonance energy transfer phenomenon (FRET) is known. In this compound, a difference in maximum absorption wavelength generally exceeds 15 nm between an absorption spectrum of a phosphor moiety I (an energy donor) that is excited with excitation light and an absorption spectrum of another phosphor moiety II (an energy acceptor) that receives energy from the phosphor moiety I and emits light or is quenched. In such a compound, the energy is transferred to the phosphor moiety II instead of emitting fluorescence from the phosphor moiety I, and thus the fluorescence intensity of the compound is decreased.

On the other hand, as described above, the fluorescent dye (F) has phosphor moieties of which light absorption characteristics are equivalent to each other, and thus the FRET phenomenon does not occur, and it is possible to exhibit a fluorescence intensity proportional to the number of phosphor moieties.

The chemical structures of the above-described phosphor moieties of which the light absorption characteristics are equivalent to each other are not particularly limited as long as the above-described difference in maximum absorption wavelength is satisfied, and they preferably have the same structure in terms of the main skeleton of the phosphor moiety. However, the steric conformation, chain length, and the like of the substituent may be different from each other, and in a

case where an anionic group or a cationic group is contained, a counter ion thereof may be different from each other. The difference in the maximum absorption wavelength is preferably within 10 nm and more preferably within 5 nm.

It is noted that the absorption spectrum of the phosphor moiety is a spectrum that is obtained measuring, using a spectrophotometer, a simple body of a phosphor constituting the phosphor moiety, which is diluted with a PBS buffer solution.

In addition, it is preferable that "the compound in which phosphor moieties adjacent to each other are each linked through a group having a structure represented by General Formula (I)" is a compound in which two structures having a phosphor moiety as a substituent are linked through a group including a structure represented by General Formula (I).

It is noted that the structure having the above-described phosphor moiety as a substituent is not particularly limited as long as the phosphor moieties adjacent to each other are each linked through a group having a structure represented by General Formula (I). For example, the structure may have, as a substituent, a group in which a 5-membered ring contains a phosphor moiety, where the 5-membered ring described in the structure represented by General Formula (I) is formed to have a carbon atom, a nitrogen atom, and $X^1$ to $X^3$ as ring-constituting atoms by a combination of $L^1$ and $L^2$, a combination of $L^1$ and $L^3$, a combination of $L^4$ and $L^5$, or a combination of $L^4$ and $L^6$, as described in detail in General Formula (II) described later. That is, the structure having a phosphor moiety as a substituent may be a structure in which the 5-membered ring described in the structure represented by General Formula (I), which has a carbon atom, a nitrogen atom, and $X^1$ to $X^3$ as ring-constituting atoms, has a group in which a phosphor moiety is contained as a substituent as long as the phosphor moieties adjacent to each other are each linked through a group having a structure represented by General Formula (I). Examples of such a compound include a compound represented by General Formula (VI) or (VII) described later.

In the fluorescent dye (F), the number of the phosphor moieties is 2 or more. The upper limit value thereof is not particularly limited and can be set to, for example, 30 or less, and it is preferably 20 or less and more preferably 15 or less.

In addition to the above, the description, the specific examples, and the like of the phosphor moiety in General Formula (II) described later can be applied to the phosphor moiety contained in the fluorescent dye (F).

(Structure represented by General Formula (I))

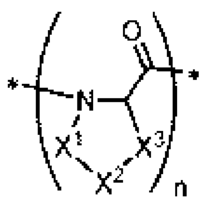

General Formula (I)

In the formula, $X^1$ to $X^3$ represent -O-, -S-, $>NR^1$, or $>CR^2R^3$.

$R^1$ to $R^3$ represent a hydrogen atom, an alkyl group, an alkenyl group, an alkynyl group, an aryl group, a heteroaryl group, an acyl group, $-NR^8R^9$, $-OR^{10}$, or an anionic group.

$R^8$ to $R^{10}$ represent a hydrogen atom, an alkyl group, an alkenyl group, an alkynyl group, an acyl group, an aryl group, a heteroaryl group, or an anionic group.

n is an integer of 2 or more.

**[0096]** * represents a bonding site.

The structure represented by General Formula (I) is preferably a structure represented by any one of General Formula (IA) or (IB) in consideration of stereoisomers. It is noted that $X^1$ to $X^3$ and n in the following general formula respectively have the same meanings as $X^1$ to $X^3$ and n in General Formula (I).

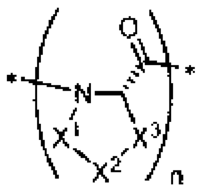

General Formula (IA)

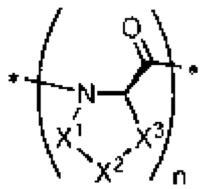

General Formula (IB)

$X^1$ to $X^3$ represent -O-, -S-, $>NR^1$, or $>CR^2R^3$, and $R^1$ to $R^3$ represent a hydrogen atom, an alkyl group, an alkenyl group, an alkynyl group, an acyl group, an aryl group, a heteroaryl group, $-NR^8R^9$, $-OR^{10}$, or an anionic group.

The alkyl group, the alkenyl group, the alkynyl group, the acyl group, the aryl group, the heteroaryl group, and the anionic group, which can be adopted as $R^1$ to $R^3$, respectively have the same meanings as the alkyl group, the alkenyl

group, the alkynyl group, the acyl group, the aryl group, the heteroaryl group, and the anionic group in the substituent group T which will be described later, and the same also applies to the preferred ranges thereof.

The alkyl group, the alkenyl group, the alkynyl group, the acyl group, the aryl group, and the heteroaryl group, which can be adopted as $R^1$ to $R^3$, may be unsubstituted or may have a substituent.

Examples of the substituent which may be contained in the alkyl group, the alkenyl group, the alkynyl group, the acyl group, the aryl group, and the heteroaryl group, as $R^1$ to $R^3$, include substituents in the substituent group T which will be described later. For example, a halogen atom or an anionic group is preferable.

In $-NR^8R^9$ and $-OR^{10}$, which can be adopted as $R^1$ to $R^3$, $R^8$ to $R^{10}$, represent a hydrogen atom, an alkyl group, an alkenyl group, an alkynyl group, an acyl group, an aryl group, a heteroaryl group, or an anionic group.

The alkyl group, the alkenyl group, the alkynyl group, the acyl group, the aryl group, the heteroaryl group, and the anionic group, which can be adopted as $R^8$ to $R^{10}$, respectively have the same meanings as the alkyl group, the alkenyl group, the alkynyl group, the acyl group, the aryl group, the heteroaryl group, and the anionic group in the substituent group T which will be described later, and the same also applies to the preferred ranges thereof.

The alkyl group, the alkenyl group, the alkynyl group, the acyl group, the aryl group, the heteroaryl group, which can be adopted as $R^8$ to $R^{10}$, may be unsubstituted or may have a substituent.

Examples of the substituent which may be contained in the alkyl group, the alkenyl group, the alkynyl group, the acyl group, the aryl group, and the heteroaryl group, as $R^8$ to $R^{10}$, include substituents in the substituent group T which will be described later. For, example, a halogen atom, a carbamoyl group, an acylamino group, an alkoxy group (preferably, an alkoxy group having an anionic group), or an anionic group is preferable, and a carbamoyl group, an acylamino group, an alkoxy group (preferably, an alkoxy group having an anionic group), or an anionic group is more preferable.

$R^1$ is preferably a hydrogen atom, an alkyl group, $-NR^8R^9$, $-OR^{10}$, or an anionic group.

$R^2$ and $R^3$ are preferably a hydrogen atom, $-NR^8R^9$, $-OR^{10}$, or an anionic group, where it is more preferable that $R^2$ is a hydrogen atom, $-NR^8R^9$, $-OR^{10}$, or an anionic group, and $R^3$ is a hydrogen atom.

$R^8$ to $R^{10}$ are preferably a hydrogen atom, an alkyl group, or an acyl group. The alkyl group may be an alkyl group having an anionic group, and the acyl group may be an acyl group having an anionic group (preferably, an acyl group having an anionic group or an acyl group substituted with an alkoxy group having an anionic group). The above-described alkyl group and acyl group may have a substituent other than the anionic group, and examples thereof include substituents in the substituent group T which will be described later. For example, a carbamoyl group or an acylamino group is preferable.

Regarding $X^1$ to $X^3$, it is preferable that at least any one thereof is $>CR^2R^3$, and it is more preferable that at least two thereof are $>CR^2R^3$ and the remaining one is -O-, -S-, or $>CR^2R^3$.

From the viewpoint of making the structure represented by General Formula (I) a more rigid structure, it is preferable that the structure represented by General Formula (I) includes a structure in which $X^1$ to $X^3$ are $>CR^2R^3$. That is, "the structure represented by General Formula (I) includes a structure in which $X^1$ to $X^3$ are $>CR^2R^3$" means that all of $X^1$ to $X^3$ are $>CR^2R^3$ in at least one structure represented by General Formula (i) among n pieces of the consecutive structures represented by General Formula (i) described above.

The proportion of the number of the structures in which $X^1$ to $X^3$ are $>CR^2R^3$ among the structures represented by General Formula (I) is preferably 30% or more, more preferably 60% or more, and still more preferably 80%. The upper limit of the ratio is not particularly limited and may be 100% or less. It is noted that it is also preferable that all of the structures represented by General Formula (I) are structures in which $X^1$ to $X^3$ described above are $>CR^2R^3$.

From the viewpoint of suppressing the intermolecular interaction and furthermore, the intramolecular interaction between structures represented by General Formula (I) in a case where a plurality of structures are present, at least one $R^2$ in the above-described structure in which $X^1$ to $X^3$ is $>CR^2R^3$ is preferably $-NR^8R^9$, $-OR^{10}$, or an anionic group, and it is more preferably $-NR^8R^9$, $-OR^{10}$, or an anionic group, where at least one of $R^8$, ..., or $R^{10}$ is a group including an anionic group.

It is noted that the phrase "at least one $R^2$ in structure in which $X^1$ to $X^3$ are $>CR^2R^3$ is $-NR^8R^9$, $-OR^{10}$, or an anionic group, where at least one of $R^8$, ..., or $R^{10}$ is a group including an anionic group" means that, in other words, at least one of $R^8$ or $R^9$ is a group including an anionic group in a case where $R^2$ is $-NR^8R^9$, and $R^{10}$ is a group including an anionic group in a case where $R^2$ is $-OR^{10}$.

In addition, the description that $R^{10}$ is a group including an anionic group means that $R^{10}$ is an anionic group or is a group having an anionic group as a substituent. The same applies to a case where at least one of $R^8$ or $R^9$ is a group including an anionic group.

n is an integer of 2 or more. Since n is an integer of 2 or more, the fluorescent dye (F) can impart the rigidity that is effective for suppressing the association of dyes (phosphor moieties), to a structure represented by General Formula (I), and a decrease in the fluorescence intensity can be suppressed.

In a case where the number of phosphor moieties is 2, the lower limit value of n is preferably an integer of 3 or more, more preferably an integer of 7 or more, still more preferably an integer of 9 or more, and particularly preferably an integer of 12 or more, from the viewpoint of further improving the fluorescence intensity. The upper limit value thereof is not

particularly limited and can be set to, for example, an integer of 72 or less, and it is preferably an integer of 36 or less, more preferably an integer of 24 or less, and still more preferably an integer of 18 or less. That is, n is preferably an integer of 3 to 72, more preferably an integer of 7 to 36, still more preferably an integer of 9 to 24, and particularly preferably an integer of 12 to 18.

On the other hand, in a case where the number of phosphor moieties is 3 or more, a certain degree of association of dyes can be allowed since the number of phosphor moieties (the number of dyes) per one molecule is large as compared with a case where the number of phosphor moieties is 2, and in a case where the number of phosphor moieties is an integer of 3 or more, the fluorescence intensity can be further improved, which is preferable. Among the above, in a case where n is an integer of 6, there is a certain degree of effect of suppressing the association, which is insufficient. Therefore, n is more preferably an integer of 7 or more. In a case where the number of phosphor moieties is 3 or more, the upper limit value of n is, for example, preferably an integer of 72 or less, more preferably an integer of 36 or less, and still more preferably an integer of 24 or less. That is, in a case where the number of phosphor moieties is 3 or more, n is preferably an integer of 3 to 72, more preferably an integer of 7 to 36, and still more preferably an integer of 7 to 24.

<Compound represented by General Formula (II)>

The fluorescent dye (F) is preferably represented by General Formula (II).

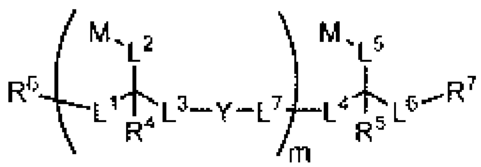

General Formula (II)

In the formula, $R^4$ and $R^5$ represent a hydrogen atom, an alkyl group, an alkenyl group, an alkynyl group, an acyl group, an amino group, a hydroxy group, an alkoxy group, a sulfanyl group, an aryl group, or a heteroaryl group.

$R^6$ and $R^7$ represent a hydrogen atom, a hydroxy group, a sulfanyl group, an alkyl group, an alkenyl group, an alkynyl group, an aryl group, an alkoxy group, an amino group, an acyl group, a heteroaryl group, an anionic group, a cationic group, or Q.

Q represents a carboxy group, a substituent capable of being bonded to a biological substance, or a substituent capable of being bonded to a solid support.

$L^1$ to $L^7$ represents a single bond or a divalent linking group.

M represents a phosphor moiety, a physiologically active substance moiety, a prodrug moiety, or a radioactive isotope-containing moiety.

Y represents the structure represented by General Formula (I) described above.

m is an integer of 1 or more.

However, at least two of M's represent the above-described phosphor moieties of which the light absorption characteristics are equivalent to each other. In other words, this means that at least two of M's are phosphor moieties, where individual phosphor moieties adjacent to each other are the above-described phosphor moieties of which the light absorption characteristics are equivalent to each other.

$R^4$ and $R^5$ represent a hydrogen atom, an alkyl group, an alkenyl group, an alkynyl group, an acyl group, an amino group, a hydroxy group, an alkoxy group, a sulfanyl group, an aryl group, or a heteroaryl group.

The alkyl group, the alkenyl group, the alkynyl group, the acyl group, the amino group, the alkoxy group, the aryl group, and the heteroaryl group, which can be adopted as $R^4$ or $R^5$, respectively have the same meanings as the alkyl group, the alkenyl group, the alkynyl group, the acyl group, the amino group, the alkoxy group, the aryl group, and the heteroaryl group in the substituent group T which will be described later, and the same also applies to the preferred ranges thereof.

The alkyl group, the alkenyl group, the alkynyl group, the acyl group, the amino group, the alkoxy group, the aryl group, and the heteroaryl group, which can be adopted as $R^4$ or $R^5$, may be unsubstituted or may have a substituent, and examples of the substituent which may be contained therein include substituents in the substituent group T which will be described later. For example, a halogen atom is preferable.

From the viewpoint of ease of synthesis, $R^4$ and $R^5$ are preferably a hydrogen atom. $R^4$ and $R^5$ are often a hydrogen atom in a case where an amino acid is used as a raw material; however, the substituents in $R^4$ and $R^5$ do not greatly contribute to the exhibition of the excellent fluorescence intensity by the fluorescent dye (F), and thus $R^4$ and $R^5$ may be another substituent (an alkyl group, an alkenyl group, an alkynyl group, an acyl group, an amino group, a hydroxy group, an alkoxy group, a sulfanyl group, an aryl group, or a heteroaryl group) other than the hydrogen atom.

$R^6$ and $R^7$ represent a hydrogen atom, a hydroxy group, a sulfanyl group, an alkyl group, an alkenyl group, an alkynyl group, an aryl group, an alkoxy group, an amino group, an acyl group, a heteroaryl group, an anionic group, a

cationic group, or Q.

The alkyl group, the alkenyl group, the alkynyl group, the aryl group, the alkoxy group, the amino group, the acyl group, the heteroaryl group, the anionic group, and the cationic group, which can be adopted as $R^6$ or $R^7$, respectively have the same meanings as the alkyl group, the alkenyl group, the alkynyl group, the aryl group, the alkoxy group, the amino group, the acyl group, the heteroaryl group, the anionic group, and the cationic group in the substituent group T which will be described later, and the same also applies to the preferred ranges thereof.

The alkyl group, the alkenyl group, the alkynyl group, the aryl group, the alkoxy group, the amino group, the acyl group, and the heteroaryl group, which can be adopted as $R^6$ or $R^7$, may be unsubstituted or may have a substituent.

Examples of the substituent which may be contained in the alkyl group, the alkenyl group, the alkynyl group, the aryl group, the alkoxy group, the amino group, the acyl group, and the heteroaryl group, as $R^6$ or $R^7$, include substituents in the substituent group T which will be described later, where an alkyl group, an acyl group, an alkoxy group, an amino group, an anionic group, a cationic group, $-(L-O)_gR^E$, or Q, or a substituent obtained by combining two or more thereof is preferable, and an alkyl group, an acyl group, an alkoxy group, an amino group, $-(L-O)_gR^E$, or Q, or a substituent obtained by combining two or more of these substituents is more preferable.

Q, which can be adopted as $R^6$ or $R^7$, represents a carboxy group, a substituent capable of being bonded to a biological substance, or a substituent capable of being bonded to a solid support.

As the substituent capable of being bonded to a biological substance, the description of the substituent capable of being bonded to a biological substance described later can be applied, and as the substituent capable of being bonded to a solid support, the description of the substituent capable of being bonded to a solid support described later can be applied.

For $R^6$, the description of the substituent represented by $-L^9R^{6A}$ or $-L^{13}R^{6A}$ described later is preferably described, and For $R^7$, the description of the substituent represented by $-L^8R^{7A}$ or $-L^{12}R^{7A}$ described later is preferably described. Specifically, the description of the substituent represented by $-L^9R^{6A}$ means a substituent obtained by a combination of a group that can be adopted as $L^9$ and a group that can be adopted as $R^{6A}$. The same applies to $-L^{13}R^{6A}$, $-L^8R^{7A}$, and $-L^{12}R^{7A}$.

In the present invention, from the viewpoint that a proper hydrophilicity and a proper excluded volume effect can be provided and an excellent fluorescence intensity can be obtained, it is preferable that any one of $R^6$ or $R^7$ preferably includes a structure represented by $-(L-O)_gR^E$, and it is more preferable that $R^7$ includes a structure represented by $-(L-O)_gR^E$.

$L^2$ to $L^5$ and $L^7$ represent a single bond or a divalent linking group and are preferably a single bond or a linking group obtained by combining one or two or more of an alkylene group, an alkenylene group, an alkynylene group, an arylene group, a heteroarylene group, -O-, -S-, >C=O, $>NR^A$, >S=O, $>S(=O)_2$, and $>P(=O)OR^B$. $R^A$ and $R^B$ respectively have the same meanings as $R^A$ and $R^B$ described later and represent a hydrogen atom or a substituent.

$L^1$ and $L^6$ represent a single bond or a divalent linking group and are preferably a single bond, an alkylene group, an alkenylene group, an alkynylene group, an arylene group, a heteroarylene group, -O-, -S-, >C=O, $>NR^A$, >S=O, $>S(=O)_2$, or $>P(=O)OR^B$. $R^A$ and $R^B$ respectively have the same meanings as $R^A$ and $R^B$ described later and represent a hydrogen atom or a substituent.

The alkylene group that can constitute $L^1$ to $L^7$ has the same meaning as the group in which one hydrogen atom is further removed from the alkyl group selected from the substituent group T, which is described later, and the same applies to the preferred one thereof.

The alkenylene group that can constitute $L^1$ to $L^7$ has the same meaning as the group in which one hydrogen atom is further removed from the alkenyl group selected from the substituent group T, which is described later, and the same applies to the preferred one thereof.

The alkenylene group that can constitute $L^1$ to $L^7$ has the same meaning as the group in which one hydrogen atom is further removed from the alkynyl group selected from the substituent group T, which is described later, and the same applies to the preferred one thereof.

The arylene group that can constitute $L^1$ to $L^7$ has the same meaning as the group in which one hydrogen atom is further removed from the aryl group selected from the substituent group T, which is described later, and the same applies to the preferred one thereof.

The heteroarylene group that can constitute $L^1$ to $L^7$ is synonymous with the group in which one hydrogen atom is further removed from the heteroaryl group selected from the substituent group T, which is described later, and the same applies to the preferred one thereof.

The alkylene group, the alkenylene group, the alkynylene group, the arylene group, and the heteroarylene group, which can constitute $L^1$ to $L^7$, may be an unsubstituted group or a group having a substituent.

The substituent which may be contained in the alkylene group, the alkenylene group, the alkynylene group, the arylene group, and the heteroarylene group, which can constitute $L^1$ to $L^7$, is not particularly limited, and it is preferably selected from a substituent group T described later. More preferred examples thereof include a halogen atom, an alkyl group, an acylamino group, and a carbamoyl group. In addition, the substituent which may be contained in the alkylene group, the alkenylene group, the alkynylene group, the arylene group, and the heteroarylene group, which can constitute

$L^1$ to $L^7$, may be substituted with a substituent selected from the substituent group T which will be described later, and it is, for example, preferably an amino group.

In addition, the number of substituents which may be contained in the alkylene group, the alkenylene group, the alkynylene group, the arylene group, and the heteroarylene group, which can constitute $L^1$ to $L^7$, is not particularly limited as long as it can be adopted as a structure. The number thereof can be set to at least one or more, the upper limit thereof is not particularly limited, and for example, all hydrogen atoms in the alkylene group, the alkenylene group, the alkynylene group, the arylene group, and the heteroarylene group may be substituted with a substituent.

The substituent which can be adopted as $R^A$ in $>NR^A$ and as $R^B$ in $>P(=O)OR^B$, which can constitute $L^1$ to $L^7$, is not particularly limited, and it is preferably selected from the substituent group T which will be described later. $R^A$ is preferably a hydrogen atom, an alkyl group, an alkenyl group, an alkynyl group, an aryl group, a heteroaryl group, or an anionic group, more preferably a hydrogen atom or an alkyl group, and still more preferably a hydrogen atom. $R^B$ is preferably a hydrogen atom, an alkyl group, an alkenyl group, an alkynyl group, an aryl group, or a heteroaryl group, more preferably a hydrogen atom or an alkyl group, and still more preferably a hydrogen atom.

It is noted that all the alkyl group, the alkenyl group, the alkynyl group, the aryl group, and the heteroaryl group, which can be adopted as $R^A$ and $R^B$, may be an unsubstituted group or a group having a substituent.

In the linking group obtained by combining two or more of an alkylene group, an alkenylene group, an alkynylene group, an arylene group, a heteroarylene group, -O-, -S-, >C=O, $>NR^A$, >S=O, $>S(=O)_2$, and $>P(=O)OR^B$, which can constitute $L^2$ to $L^5$ and $L^7$, the type of the group to be combined is not particularly limited as long as the group to be combined has a reasonable chemical structure; however, it is preferably 2 to 6 types and more preferably 2 to 4 types. It is noted that in a case of counting, as one type, each of the alkylene group, the alkenylene group, the alkynylene group, the arylene group, the heteroarylene group, -O-, -S-, >C=O, $>NR^A$, >S=O, $>S(=O)_2$, and $>P(=O)OR^B$, the maximum number of types is 12.

It is noted that in the linking group obtained by combining two or more of an alkylene group, an alkenylene group, an alkynylene group, an arylene group, a heteroarylene group, -O-, -S-, >C=O, $>NR^A$, >S=O, $>S(=O)_2$, and $>P(=O)OR^B$, which can constitute $L^2$ to $L^5$ and $L^7$, the number of groups to be combined is not particularly limited; however, examples thereof preferably include 2 to 10 groups, more preferably include 2 to 6 groups, and still more preferably 2 to 4 groups.

(i) $L^1$ and $L^6$

$L^1$ is more preferably >C=O, $>NR^A$, an arylene group, an alkylene group, -O-, or -S-, still more preferably >C=O, $>NR^A$, an arylene group, or an alkylene group, and particularly preferably >C =O or $>NR^A$.

$L^6$ is more preferably a single bond, >C=O, $>NR^A$, or an arylene group, and still more preferably a single bond, >C=O, or $>NR^A$.

(ii) $L^3$, $L^4$, and $L^7$

$L^3$, $L^4$, and $L^7$ are more preferably a single bond, >C =O, $>NR^A$, an alkylene group, an alkenylene group, an alkynylene group, an arylene group, or a heteroarylene group, or a combination of at least one of an alkylene group, an alkenylene group, an alkynylene group, an arylene group, or a heteroarylene group, and >C=O and $>NR^A$, and still more preferably a single bond, >C =O, $>NR^A$, an alkylene group, an alkenylene group, an alkynylene group, an arylene group, or a heteroarylene group, or a group that links $-C(=O)NR^A-$ to >C=O, or $>NR^AC(=O)-$ to $>NR^A$ by at least one of an alkylene group, an alkenylene group, an alkynylene group, an arylene group, or a heteroarylene group.

(iii) $L^2$ and $L^5$

$L^2$ and $L^5$ are more preferably a group obtained by combining one or two or more of an alkylene group, an alkenylene group, an alkynylene group, an arylene group, a heteroarylene group, -O-, -S-, >C=O, and $>NR^A$, and are still more preferably a group represented by * $-L^x-L^y-$**.

$L^x$ is a single bond or a group obtained by combining one or two or more of an alkylene group, an alkenylene group, an alkynylene group, an arylene group, and a heteroarylene group, and $L^y$ is a single bond, -O, -, -S-, >C=O, or $>NR^A$. * represents a bonding site to a carbon atom to which $L^1$ and $L^3$ are bonded or a carbon atom to which $L^4$ and $L^6$ are bonded, and ** represents a bonding site to M. However, in a case where $L^y$ is a single bond, $L^x$ is a heteroarylene group or a group consisting of a group obtained by combining one or two or more of an alkylene group, an alkenylene group, an alkynylene group, and an arylene group, which are located on the * side, and a heteroarylene group which is located on the ** side.

Among the groups represented by * $-L^x-L^y-$**, $L^2$ and $L^5$ are preferably a group in which $L^y$ is a single bond, -S-, >C=O, or $>NR^A$, more preferably a group in which $L^y$ is >C=O or $>NR^A$, and still more preferably a group in which $L^x$ is an alkylene group and $L^y$ is >C=O or $>NR^A$.

It is noted that in the compound represented by General Formula (II), it suffices that the number of shortest-distance atoms in the linking chain (each linking chain of the linking chain including $L^2$ and the linking chain including $L^5$) that connects M to a carbon atom to which $L^1$ and $L^3$ are bonded or a carbon atom to which $L^4$ and $L^6$ are bonded is, for example, 1 to 60, where 1 to 40 is preferable. In a case where M is a phosphor moiety, the above-described number of shortest-distance atoms means the number of atoms that constitute the shortest chain in the linking chain that connects a conjugated structural moiety for exhibiting fluorescence in the phosphor moiety M to a carbon atom to which $L^1$ and $L^3$ are bonded or a carbon atom to which $L^4$ and $L^6$ are bonded.

It is noted that in the compound represented by General Formula (II), it is also preferable that a structure represented by $-(CH_2-CH_2-O)_b-$ described later (b is also as described later) is provided at any portion of the linking chain represented by "-linking group ZZZ-$L^2$-" in the structure represented by "the conjugated structural moiety of M - the linking group ZZZ-$L^2$- described later" and any portion of the linking chain represented by "-linking group ZZZ-$L^5$-" in the structure represented by "the conjugated structural moiety of M - the linking group ZZZ-$L^5$- described later".

In the compound represented by General Formula (II), adjacent groups may be bonded to each other to form a ring. Examples of the combination of adjacent groups which may be bonded to each other to form a ring include a combination of $L^1$ and $L^2$, a combination of $L^1$ and $L^3$, a combination of $L^2$ and $R^4$, a combination of $L^4$ and $L^5$, a combination of $L^4$ and $L^6$, or a combination of $L^5$ and $R^5$.

The above-described ring which may be formed by bonding adjacent groups to each other may be any one of an aromatic ring or an aliphatic ring or may be any one of a hydrocarbon ring or a hetero ring, and it is preferably a 5- or 6-membered ring.

The preferred examples of the aliphatic ring include a cyclopentane ring, a cyclohexane ring, or a 5-membered ring having a carbon atom, a nitrogen atom, and $X^1$ to $X^3$ as ring-constituting atoms, which is described in the structure represented by General Formula (I) described above, where a 5-membered ring having a carbon atom, a nitrogen atom, and $X^1$ to $X^3$ as ring-constituting atoms, which is described in the structure represented by General Formula (I) described above, is more preferable.

The aromatic ring is preferably a benzene ring or a nitrogen-containing aromatic heterocyclic ring, more preferably a benzene ring or a nitrogen-containing aromatic heterocyclic ring in which the ring-constituting atom is composed of a carbon atom and a nitrogen atom, and still more preferably a benzene ring or a pyridine ring.

These rings may have a substituent, and the substituent which may be contained is not particularly limited and is selected from the substituent group T.

The ring formed by the combination of $L^2$ and $R^4$ or the combination of $L^5$ and $R^5$ may be any one of the above-described aliphatic ring or aromatic ring, where the above-described aliphatic ring is preferable.

The ring formed by a combination of $L^1$ and $L^2$, a combination of $L^1$ and $L^3$, a combination of $L^4$ and $L^5$, or a combination of $L^4$ and $L^6$, may be any one of the above-described aliphatic ring or aromatic ring, where a 5-membered ring having a carbon atom, a nitrogen atom, and $X^1$ to $X^3$ as ring-constituting atoms, which is described in the structure represented by General Formula (I) described above, or a benzene ring is preferable.

For example, the following structures surrounded by broken lines in General Formula (II)

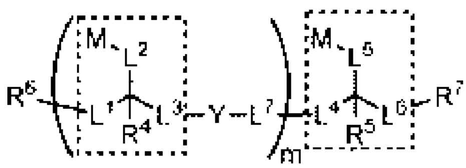

General Formula (II)

can be set to structures including the following structures. In the following structure, * indicates a connecting portion.

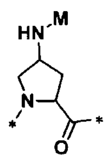

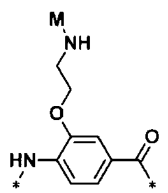

m is an integer of 1 or more. The upper limit value thereof is not particularly limited and can be set to, for example, an integer of 30 or less, and it is preferably an integer of 20 or less, more preferably an integer of 15 or less, and still more preferably an integer of 10 or less. That is, m can be an integer of 1 to 30, and is preferably an integer of 1 to 20, more preferably an integer of 1 to 15, and still more preferably an integer of 1 to 10.

M represents a phosphor moiety, a physiologically active substance moiety, a prodrug moiety, or a radioactive isotope-containing moiety. However, at least two of M's represent phosphor moieties of which the light absorption

characteristics are equivalent to each other.

The phosphor moiety which can be adopted as M (hereinafter, also referred to as the phosphor moiety M) can be used without particular limitation as long as it is a structural moiety consisting of an organic compound that exhibits fluorescence. In addition, the phosphor moiety M may be a structural moiety in which a structural moiety consisting of an organic compound exhibiting fluorescence further has a linking group. Such a linking group is not particularly limited; however, examples thereof include a linking group ZZZ described later. For example, in the compound represented by General Formula (II), preferred examples of the compound include a compound in which the phosphor moiety M is bonded to $L^2$ or $L^5$ by this linking group ZZZ.

Preferred examples of the phosphor moiety M include a structural moiety consisting of at least one dye of a xanthene dye, a rhodamine dye, a coumarin dye, a cyanine dye, a pyrene dye, an oxazine dye, a squarylium dye, a pyridyloxazole dye, or a pyrromethene dye.

As the xanthene dye, the rhodamine dye, the coumarin dye, the cyanine dye, the pyrene dye, the oxazine dye, the squarylium dye, the pyridyloxazole dye, and the pyrromethene dye, dyes that are generally known as these dyes can be used without particular limitation.

As one aspect, the phosphor moiety M is preferably a structural moiety consisting of a pyrromethene dye. Examples of the pyrromethene dye include a dipyrromethene boron complex. As the dipyrromethene boron complex, it is possible to use a fluorescent compound (a dipyrromethene boron complex) represented by General Formula (1) or (4) described in WO2019/230963A, or a compound (a dipyrromethene boron complex) represented by General Formula (1) described in WO2021/100814A, and the description thereof can be incorporated into the present specification by reference.

It is noted that the incorporation is made such that the dye that constitutes the phosphor moiety M does not have a substituent capable of being bonded to a biological substance.

As another aspect, the phosphor moiety M is preferably a structural moiety consisting of a cyanine dye, and it is more preferably a structural moiety consisting of a cyanine dye represented by General Formula (α).

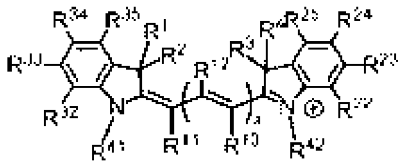

General Formula (α)

In the formula, $R^1$ to $R^4$ represent an alkyl group or $-(CH_2-CH_2-O)_b-R^{21}$. b is 1 to 50, and $R^{21}$ represents an alkyl group.

$R^{11}$ to $R^{13}$ represent a hydrogen atom, an alkyl group, an alkoxy group, an aryloxy group, an alkylthio group, an arylthio group, an amino group, or a halogen atom, where adjacent groups may be bonded to each other to form a 5-membered or 6-membered ring.

$R^{22}$ to $R^{25}$ and $R^{32}$ to $R^{35}$ represent a hydrogen atom, an alkyl group, an alkoxy group, an aryl group, a sulfo group, a sulfamoyl group, a carboxy group, an alkoxycarbonyl group, an aryloxycarbonyl group, an acyloxy group, a carbamoyl group, an acylamino group, a nitro group, or a halogen atom.

$R^{41}$ and $R^{42}$ represent an alkyl group or $-(CH_2-CH_2-O)_b-R^{21}$. $R^{21}$ and b respectively have the same meanings as $R^{21}$ and b described above. $R^{41}$ and $R^{42}$ may be bonded to each other to form a ring.

a is an integer of 1 to 3.

In a case where one hydrogen atom is removed from any of $R^1$ to $R^4$, $R^{11}$ to $R^{13}$, $R^{22}$ to $R^{25}$, $R^{32}$ to $R^{35}$, $R^{41}$, or $R^{42}$ described above, a monovalent structural moiety is provided.

However, the cyanine dye represented by Formula (α) is neutral.

Depending on the length of the methine chain having a repetition number of 2a + 3, which is connected by a conjugated double bond, the cyanine dyes represented by General Formula (α) respectively have an excitation absorption wavelength in a wavelength range of 520 to 600 nm (in the vicinity of 585 nm) in a case of a = 1, in a wavelength range of 620 to 700 nm (in the vicinity of 685 nm) in a case of a = 2, and in a wavelength range of 740 to 830 nm (in the vicinity of 785 nm) in a case of a = 3. As a result, each of the fluorescent dye (F) which has, as the phosphor moiety M, a structural moiety consisting of a cyanine dye represented by General Formula (α) can be used as a compound exhibiting an excellent fluorescence intensity, in the fluorescence labeling in which a light source having any wavelength in a wavelength range of about 500 to 800 nm (for example, in the vicinity of 600 nm, in the vicinity of 700 nm, or in the vicinity of 800 nm) matching with the absorption excitation wavelength of the compound is used as an excitation light source.

In multicolor WB, a plurality of luminescence colors are detected in the range from the visible range to the near infrared range. As a result, it is necessary to select wavelengths so that the absorption and luminescence waveforms of a plurality of dyes have a suitable wavelength relationship so that crosstalk does not occur due to mutual interference in a case where the dyes are excited to emit light. Ideally, it should be adjusted so that only one dye emits light at one excitation

light and the other dyes do not emit light. From this point of view, two types of excitation light sources having wavelengths separated to some extent, for example, in the vicinity of 700 nm and in the vicinity of 800 nm, are used for luminescence in the near infrared range of the multicolor WB.

As compared with the detection by visible light excitation, the fluorescence detection by near-infrared light excitation can suppress the autofluorescence of the membrane, that is, the background fluorescence, and thus it is easy to increase the signal to noise ratio (the S/N ratio) and it is possible to detect a target protein with high sensitivity. As a result, in recent years, there has been an increasing need for fluorescence detection WB using luminescence in the near infrared range in the analytical research on the trace amount of proteins.

However, in the near infrared range, the fluorescence quantum yield of the fluorescent dye is generally low, and thus it is difficult to obtain a high signal amount. Among the fluorescent dyes (F) which has, as the phosphor moiety M, a structural moiety consisting of a cyanine dye represented by General Formula ($\alpha$), the compound in which a = 2 or 3 can be used as a compound that exhibits an excellent fluorescence intensity even in the multicolor WB having the above-described two types of excitation light sources in the vicinity of 700 nm and in the vicinity of 800 nm, and in particular, it can exhibit an excellent fluorescence intensity even with respect to a request for observing and detecting proteins with higher sensitivity, as compared with the fluorescence labeling using cyanine dyes in the related art.

(i) $R^1$ to $R^4$

$R^1$ to $R^4$ represent an alkyl group or $-(CH_2-CH_2-O)_b-R^{21}$.

The alkyl group which can be adopted as $R^1$ to $R^4$ has the same meaning as the alkyl group in the substituent group T which will be described later.

The unsubstituted alkyl group preferably has 1 to 6 carbon atoms, more preferably has 1 to 4 carbon atoms, and still more preferably has 1 or 2 carbon atoms.

In a case where the alkyl group has a substituent, the alkyl group moiety of the alkyl group having a substituent preferably has 1 to 10 carbon atoms, more preferably has 1 to 8 carbon atoms, still more preferably has 2 to 6 carbon atoms, and particularly preferably has 2 to 5 carbon atoms. In addition, the number of atoms that constitute the longest chain of the alkyl group having a substituent is preferably 3 to 35, more preferably 3 to 25, still more preferably 3 to 15, and particularly preferably 3 to 11.

In the present invention, the "number of carbon atoms of the alkyl group moiety of the alkyl group having a substituent" means the number of carbon atoms excluding the substituent moiety contained in the alkyl group.

In the present invention, the "number of atoms that constitute the longest chain of the alkyl group having a substituent" means the number of atoms including the substituent moiety (that is, the number of atoms obtained by subtracting the number of atoms of the molecular chain that does not constitute the longest chain, from the number of total atoms). It is noted that in a case where a substituent having a dissociative hydrogen atom such as a sulfo group or a carboxy group constitutes the longest chain, the calculation is carried out including the hydrogen atom regardless of the presence or absence of dissociation. In addition, the number of atoms in the substituent moiety capable of being bonded to a biological substance described later is not included.

Examples of the substituent which may be contained in the alkyl group which can be adopted as $R^1$ to $R^4$ include an alkoxy group, a carboxy group, an alkoxycarbonyl group, an acyloxy group, a carbonyl group, an acylamino group, a sulfo group, a phosphono group, and - $(CH_2-CH_2-O)_b-R^{21}$, as well as a group consisting of a combination of these substituents.

The alkyl group having a substituent, which can be adopted as $R^1$ to $R^4$, is not particularly limited as long as it is the above-described alkyl group having a substituent.

The alkyl group which can be adopted as $R^1$ to $R^4$ is preferably an unsubstituted alkyl group.

($-(CH_2-CH_2-O)_b-R^{21}$)

In $-(CH_2-CH_2-O)_b-R^{21}$ which can be adopted as $R^1$ to $R^4$, b is 1 to 50, and $R^{21}$ represents an alkyl group.

b means an average repetition number (simply, also referred to as a repetition number), and it is preferably 1 to 24, more preferably 1 to 12, still more preferably 1 to 10, particularly preferably 4 to 10, and most preferably 4 to 8.

The average repetition number can be calculated from the average integrated value obtained by subjecting a compound to $^1$H-NMR measurement. The average repetition number defined in the present invention means a number that is obtained by rounding off the first decimal place of the average repetition number calculated according to the above method.

To the alkyl group as $R^{21}$, the description of the alkyl group which can be adopted as $R^1$ to $R^4$ described above can be applied.

The $-(CH_2-CH_2-O)_b-R^{21}$ which can be adopted as $R^1$ to $R^4$ and $-(CH_2-CH_2-O)_b-R^{21}$ which can be adopted as a substituent by an alkyl group as $R^1$ to $R^4$ are preferably an alkyl group of $-(CH_2-CH_2-O_b$-unsubstituted.

From the viewpoint of further improving the fluorescence intensity of the fluorescent dye itself, it is preferable that at least one of $R^1$, ..., or $R^4$ includes a structure represented by $-(CH_2-CH_2-O)_b-$, and it is more preferable at least one of $R^1$ or $R^2$ and at least one of $R^3$ or $R^4$ include a structure represented by $-(CH_2-CH_2-O)_b-$.

It is still more preferable that the entire phosphor moiety M in the fluorescent dye (F) is a structural moiety consisting of a cyanine dye represented by General Formula ($\alpha$), where at least one of $R^1$ or $R^2$ and at least one of $R^3$ or $R^4$ includes a structure represented by $-(CH_2-CH_2-O)_b-$.

It is preferable that the structure represented by $-(CH_2-CH_2-O)_b-$ is introduced by employing $-(CH_2-CH_2-O)_b-R^{21}$ as $R^1$ to $R^4$.

The b in $-(CH_2-CH_2-O)_b-$ described above has the same meaning as the b in $-(CH_2-CH_2-O)_b-R^{21}$ described above.

Since the substituents of $R^1$ to $R^4$ protrude in a direction perpendicular to the cyanine dye skeleton (plane), it is presumed that in a case of including a structure represented by $-(CH_2-CH_2-O)_b-$ as this substituent, the fused ring portion is difficult to undergo the $\pi$-$\pi$ interaction (the effect of suppressing the association is strengthened), and thus the decrease in the fluorescence intensity due to the association can be suppressed.

(ii) $R^{11}$ to $R^{13}$

$R^{11}$ to $R^{13}$ each independently represent a hydrogen atom, an alkyl group, an alkoxy group, an aryloxy group, an alkylthio group, an arylthio group, an amino group, or a halogen atom. Adjacent groups may be bonded to each other to form a 5- or 6-membered ring.

The alkyl group, the alkoxy group, the aryloxy group, the alkylthio group, the arylthio group, the amino group, and the halogen atom, which can be adopted as $R^{11}$ to $R^{13}$, respectively have the same meanings as the alkyl group, the alkoxy group, the aryloxy group, the alkylthio group, the arylthio group, the amino group, and the halogen atom in the substituent group T described later, and the same applies to the preferred ranges thereof.

Examples of the substituent which may be contained in the alkyl group, the alkoxy group, the aryloxy group, the alkylthio group, the arylthio group, and the amino group, as $R^{11}$ to $R^{13}$, include the substituents in the substituent group T described below.

Among $R^{11}$ to $R^{13}$, the 5- or 6-membered ring formed by bonding adjacent groups to each other may be either aromatic or aliphatic, and it is preferably aliphatic. In addition, it is preferable to form a 6-membered ring. The number of the above-described 5- or 6-membered rings in the compound is not particularly limited; however, it is preferably 1 or 2 and more preferably 1.

In a case of taking a case of a = 3 as an example, preferred examples of the structure having a ring formed by bonding adjacent groups among $R^{11}$ to $R^{13}$ include the following structures. It is noted that in the following examples, $R^{11}$ to $R^{13}$ that do not form a ring structure are a hydrogen atom, and the ring structure is described as a structure that does not have a substituent, which are not limited thereto. It is noted that, hereinafter, the structure beyond the wavy line will be omitted.

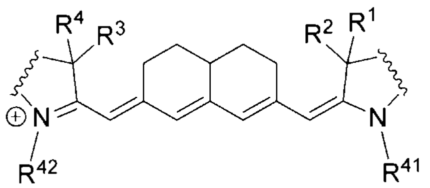
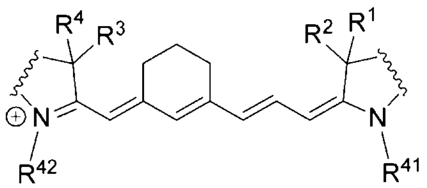
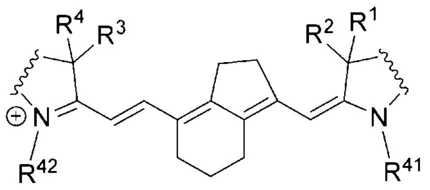
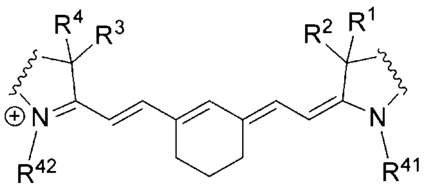

$R^{11}$ and $R^{13}$ possessed by the carbon atom bonded to the indolenine ring are preferably a hydrogen atom.

$R^{12}$, and $R^{13}$ other than those described are preferably a hydrogen atom or an alkyl group.

Among $R^{11}$ to $R^{13}$, adjacent groups in $R^{12}$ and $R^{13}$ other than $R^{11}$ and $R^{13}$ possessed by the carbon atom bonded to the indolenine ring (that is, adjacent groups of $R^{13}$ and $R^{12}$ other than $R^{13}$ possessed by the carbon atom bonded to the indolenine ring) are preferably bonded to each other to form a 5- or 6-membered ring and more preferably to form a 6-membered ring. In addition, it is preferable that the 5- or 6-membered ring is formed at the central portion of the bond that connects the indoline ring and the indolenine ring. The ring formed in the central portion of the bond connecting the indoline

ring and the indolenine ring means a ring containing carbon atoms as ring-constituting atoms so that the numbers of bonded atoms from the indoline ring and the indolenine ring are the same.

In a case where one hydrogen atom is removed from any of $R^1$ to $R^4$, $R^{11}$ to $R^{13}$, $R^{22}$ to $R^{25}$, $R^{32}$ to $R^{35}$, $R^{41}$, or $R^{42}$ described above, the phosphor moiety M is a monovalent structural moiety.

Specifically, a monovalent structural moiety is provided in a case where one hydrogen atom is removed from a substituent which can be adopted as $R^1$ to $R^4$, $R^{11}$ to $R^{13}$, $R^{22}$ to $R^{25}$, $R^{32}$ to $R^{35}$, $R^{41}$, or $R^{42}$, or a hydrogen atom which can be adopted as $R^{11}$ to $R^{13}$, $R^{22}$ to $R^{25}$, or $R^{32}$ to $R^{35}$ is removed to provide a monovalent structural moiety which has a bonding site on the carbon atom to which $R^{11}$ to $R^{13}$, $R^{22}$ to $R^{25}$, or $R^{32}$ to $R^{35}$ has been bonded.

Among the above, it is preferable that the phosphor moiety M is to be a monovalent structural moiety by removing one hydrogen atom from the ring formed by the bonding of $R^{41}$ and $R^{42}$ described above or is to be a monovalent structural moiety by removing one hydrogen atom from a substituent which can be adopted as $R^{12}$ (preferably $R^{12}$ on the carbon atom at which the numbers of bonded atoms from the indoline ring and the indolenine ring are the same).

(iii) $R^{22}$ to $R^{25}$ and $R^{32}$ to $R^{35}$

$R^{22}$ to $R^{25}$ and $R^{32}$ to $R^{35}$ represent a hydrogen atom, an alkyl group, an alkoxy group, an aryl group, a sulfo group, a sulfamoyl group, a carboxy group, an alkoxycarbonyl group, an aryloxycarbonyl group, an acyloxy group, a carbamoyl group, an acylamino group, a nitro group, or a halogen atom. Regarding this $R^{22}$ to $R^{25}$ and $R^{32}$ to $R^{35}$, adjacent groups may be bonded to each other to form a fused ring.

The alkyl group, the alkoxy group, the aryl group, the sulfo group, the sulfamoyl group, the carboxy group, the alkoxycarbonyl group, the aryloxycarbonyl group, the acyloxy group, the carbamoyl group, the acylamino group, the nitro group, and the halogen atom, which can be adopted as $R^{22}$ to $R^{25}$ and $R^{32}$ to $R^{35}$, respectively have the same meanings as the alkyl group, the alkoxy group, the aryl group, the sulfo group, the sulfamoyl group, the carboxy group, the alkoxycarbonyl group, the aryloxycarbonyl group, the acyloxy group, the carbamoyl group, the acylamino group, the nitro group, and the halogen atom in the substituent group T which will be described later.

The fused ring formed by bonding adjacent groups among $R^{22}$ to $R^{25}$ and $R^{32}$ to $R^{35}$ to each other is not particularly limited. However, examples thereof include a naphthalene ring (a naphthalene ring that is formed together with a benzene ring to which $R^{22}$ to $R^{25}$ are bonded or a benzene ring to which $R^{32}$ to $R^{35}$ are bonded in a case where adjacent groups are bonded to each other to form the benzene ring). From the viewpoint of suppressing association, it is preferable that adjacent groups among $R^{22}$ to $R^{25}$ and $R^{32}$ to $R^{35}$ are not bonded to each other and do not form a fused ring.

From the viewpoint of improving water solubility and suppressing association, it is preferable that at least one of $R^{22}$, ..., or $R^{25}$ and at least one of $R^{32}$, ..., or $R^{35}$ have a hydrophilic group, and it is more preferable that at least one hydrophilic group is contained per the number of rings of one, to which $R^{22}$ to $R^{25}$ are bonded and rings to which $R^{32}$ to $R^{35}$ are bonded. For example, in a case where adjacent groups among $R^{22}$ to $R^{25}$ and $R^{32}$ to $R^{35}$ are bonded to each other to form a naphthalene ring as a fused ring, the number of rings to which $R^{22}$ to $R^{25}$ are bonded is two, and the number of rings to which $R^{32}$ to $R^{35}$ are bonded to each other is two, which means that it is more preferable that at least two of $R^{22}$ to $R^{25}$ and at least two of $R^{32}$ to $R^{35}$ have a hydrophilic group. The upper limit value thereof is not particularly limited as long as it is allowed in terms of structure, and it can be appropriately adjusted in accordance with the number of hydrophilic groups in the compound as a whole, which will be described later.

The hydrophilic group is not particularly limited; however, examples thereof include an alkoxy group having a substituent, a carboxy group, a sulfo group, and a phosphono group, where a sulfo group is preferable.

$R^{22}$ to $R^{25}$ and $R^{32}$ to $R^{35}$ are preferably a hydrogen atom, an alkyl group, a sulfo group, a nitro group, or a halogen atom, more preferably a hydrogen atom, an alkyl group, a sulfo group, or a halogen atom, and still more preferably a hydrogen atom, an alkyl group, or a sulfo group.

(iv) $R^{41}$ and $R^{42}$

$R^{41}$ and $R^{42}$ represent an alkyl group or $-(CH_2-CH_2-O)_b-R^{21}$. $R^{21}$ and b respectively have the same meanings as $R^{21}$ and b described above.

Examples of the substituent which may be contained in the alkyl groups as $R^{41}$ and $R^{42}$ include an alkoxy group, a carboxy group, an alkoxycarbonyl group, an acyloxy group, a carbamoyl group, an acylamino group, a sulfo group, and a phosphono group, as well as a group consisting of a combination of these substituents.

The alkyl group which can be adopted as $R^{41}$ and $R^{42}$ has the same meaning as the alkyl group in the substituent group T which will be described later.

The unsubstituted alkyl group preferably has 1 to 6 carbon atoms, more preferably has 1 to 4 carbon atoms, and still more preferably has 1 to 3 carbon atoms.

The alkyl group moiety of the alkyl group having a substituent preferably has 1 to 10 carbon atoms, more preferably has 1 to 8 carbon atoms, still more preferably has 1 to 7 carbon atoms, even still more preferably has 1 to 6

carbon atoms, and even further still more preferably has 1 to 5 carbon atoms. In addition, the number of atoms that constitute the longest chain of the alkyl group having a substituent is preferably 3 to 14, more preferably 3 to 12, and still more preferably 3 to 10.

The alkyl group having a substituent, which can be adopted as $R^{41}$ and $R^{42}$, is preferably an alkyl group having, as a substituent, at least one of an alkoxy group, a carboxy group, a sulfo group, or a phosphono group, and more preferably an alkyl group having, as a substituent, at least one of a carboxy group or a sulfo group, from the viewpoint of further improving water solubility. It is noted that it may be an alkyl group having a substituent consisting of a combination of the above-described preferred substituents (the alkoxy group, the carboxy group, the sulfo group, and the phosphono group) and a group other than these substituents.

In addition, the form of the alkyl group having a substituent, which can be adopted by $R^1$ to $R^4$, can be also preferably applied.

To $-(CH_2-CH_2-O)_b-R^{21}$ which can be adopted as $R^{41}$ and $R^{42}$, the description of $-(CH_2-CH_2-O)_b-R^{21}$ in $R^1$ to $R^4$ can be preferably applied.

$R^{41}$ and $R^{42}$ may be bonded to each other to form a ring.

Among the cyanine dyes represented by General Formula ($\alpha$), preferred examples of the structure in which $R^{41}$ and $R^{42}$ are bonded to each other to form a ring include a cyanine dye represented by General Formula ($\beta$).

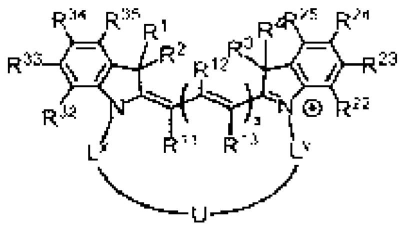

General Formula ($\beta$)

In the formula, $L^x$ to $L^y$ represent an alkylene group or $-(CH_2-CH_2-O)_b$-alkylene-*. * represents a bonding position to U.

The linking group U represents a divalent linking group having 1 to 100 atoms.

$R^1$ to $R^4$, $R^{11}$ to $R^{13}$, $R^{22}$ to $R^{25}$, $R^{32}$ to $R^{35}$, b, and a respectively have the same meanings as $R^1$ to $R^4$, $R^{11}$ to $R^{13}$, $R^{22}$ to $R^{25}$, $R^{32}$ to $R^{35}$, b, and a in General Formula ($\alpha$), and the same also applies to the preferred ranges thereof unless otherwise specified.

At least one of $R^1$, $R^2$, $R^3$, $R^4$, $L^x$, or U includes a structure represented by-$(CH_2-CH_2-O)_b$-. m has the same meaning as m described above.

However, the cyanine dye represented by Formula ($\beta$) is neutral.

The alkylene group which can be adopted as $L^x$ and $L^y$ corresponds to an alkylene group obtained by removing one hydrogen atom or one substituent from an alkyl group having a substituent which can be adopted as $R^{41}$ and $R^{42}$.

For the number of carbon atoms of the alkylene group moiety of the alkylene group which can be adopted as $L^x$ to $L^y$, the description of the number of carbon atoms of the alkyl group moiety of the alkyl group having a substituent, as $R^{41}$ and $R^{42}$, can be preferably applied.

The $-(CH_2-CH_2-O)_b$-alkylene-* which can be adopted as $L^x$ and $L^y$ corresponds to $-(CH_2-CH_2-O)_b$-alkylene- obtained by removing one hydrogen atom or one substituent from the alkyl group as $R^{21}$, among the $-(CH_2-CH_2-O)_b-R^{21}$ which can be adopted as $R^{41}$ and $R^{42}$ ($R^{21}$ represents an alkyl group having a substituent).

In the $-(CH_2-CH_2-O)_b$-alkylene-* which can be adopted as $L^x$ and $L^y$, b is preferably 1 to 10 and more preferably 1 to 8, and as the number of carbon atoms of the alkylene group moiety, the description of the number of carbon atoms of the alkyl group moiety of the alkyl group having a substituent in $R^{41}$ to $R^{42}$ can be preferably applied.

From the viewpoint of further improving the fluorescence intensity, it is preferable that both $L^x$ and $L^y$ include a structure represented by $-(CH_2-CH_2-O)_b$-.

The total number of atoms constituting the linking group U is 1 to 100, and it is preferably 10 to 90, more preferably 20 to 90, and still more preferably 30 to 80.

The linking group U is preferably a divalent linking group formed by bonding three or more selected from an alkylene group, -O-, $-NR^{50}-$, -COO-, $-CONR^{50}-$, and $-SO_2NR^{50}-$. $R^{50}$ represents a hydrogen atom or an alkyl group.

The number of carbon atoms in the alkylene moiety of the alkylene group which can be adopted as the linking group U is preferably 1 to 10, more preferably 1 to 8, still more preferably 1 to 7, particularly preferably 1 to 6, and most preferably 1 to 5.

In the present invention, "the number of carbon atoms in the alkylene moiety of the alkylene group" means the number of carbon atoms excluding the substituent moiety contained in the alkylene group.

As the alkyl group which can be adopted as $R^{50}$, the description of the alkyl group as $R^1$ to $R^4$ can be preferably applied.

$R^{50}$ is preferably a hydrogen atom.

The number of the above-described alkylene group, -O-, -$NR^{50}$-, -COO-, -$CONR^{50}$-, and -$SO_2NR^{50}$-, constituting the linking group U, is preferably 3 to 11, more preferably 3 to 7, still more preferably 3 to 5, and particularly preferably 3.

In the linking group U, the connecting portion to $L^x$ to $L^y$ is preferably -O-, -$NR^{50}$-, - COO-, -$CONR^{50}$-, or -$SO_2NR^{50}$-. That is, the linking group U is preferably bonded to the alkylene groups of $L^x$ to $L^y$ through -O-, -$NR^{50}$-, -COO-, -$CONR^{50}$-, or -$SO_2NR^{50}$-, which constitutes the linking group U. In the linking group U, it is more preferable that connecting portions to $L^x$ to $L^y$ are -O-, -$NR^{50}$-, -COO-, -$CONR^{50}$-, or -$SO_2NR^{50}$-, where the linking group U is a divalent linking group in which the connecting portions are connected to each other through an alkylene group.

It is preferable that the linking group U is to be a monovalent structural moiety, that is, a phosphor moiety M, by removing one hydrogen atom therefrom. In the linking group U, examples of the position where one hydrogen atom is removed include an alkylene group and an alkyl group as $R^{50}$, where an alkylene group is preferable.

In a case where in the linking group U, one hydrogen atom is removed in the alkylene group or the alkyl group as $R^{50}$, one hydrogen atom may be directly removed from the alkylene group or the alkyl group as $R^{50}$, and the linking group ZZZ may be bonded to the alkylene group or the alkyl group as $R^{50}$, thereby the linking group ZZZ serving as a bonding site.

Examples of the linking group ZZZ include an alkylene group, an alkenylene group, an alkynylene group, an arylene group, a heteroarylene group, -O-, -S-, >C=O, >$NR^{60}$, >S=O, >$S(=O)_2$, >$P(=O)OR^{70}$, -COO-, -$CONR^{60}$-, and -$(CH_2\text{-}CH_2\text{-}O)_p$-, as well as a group consisting of a combination of these substituents. The number of those to be combined is not particularly limited; however, it can be set to, for example, 2 to 20, and it is preferably 2 to 7 and more preferably 2 to 5.

$R^{60}$ and $R^{70}$ are a hydrogen atom or an alkyl group and are preferably a hydrogen atom. To the alkyl group which can be adopted as $R^{60}$ or $R^{70}$, the description of the alkyl group as $R^{50}$ can be preferably applied.

p represents the repetition number, and it is preferably 1 to 10, more preferably 1 to 8, and still more preferably 1 to 4.

(v) a

a is an integer of 1 to 3, where it is preferably an integer of 2 or 3.

In the cyanine dye represented by General Formula ($\alpha$), it is preferable that at least one of $R^1$, $R^2$, $R^3$, $R^4$, $R^{41}$, or $R^{42}$ includes a structure represented by -$(CH_2\text{-}CH_2\text{-}O)_b$-. b has the same meaning as b described above. It is conceived that this makes it possible for the fluorescent dye (F), which has a structural moiety consisting of a cyanine dye represented by General Formula ($\alpha$), to have a proper hydrophilicity and a proper excluded volume effect, whereby a fluorescent dye-labeled biomolecule to be obtained can exhibit an excellent fluorescence intensity.

In addition, from the viewpoint that sufficient hydrophilicity is imparted as the fluorescent dye (F), the cyanine dye represented by General Formula ($\alpha$) is such that the number of hydrophilic groups per one molecule of the cyanine dye represented by General Formula ($\alpha$) is preferably 2 or more, more preferably 2 to 8, still more preferably 2 to 6, and particularly preferably 3 to 6.

To the hydrophilic group, the description of the hydrophilic group which can be adopted by $R^{22}$ to $R^{25}$ and $R^{32}$ to $R^{35}$ described above can be applied.

The position of the hydrophilic group is not particularly limited unless specified otherwise, and examples of the group having the hydrophilic group preferably include $R^{11}$ to $R^{13}$, $R^{22}$ to $R^{25}$ $R^{32}$ to $R^{35}$, $R^{41}$, or $R^{42}$.

It is noted that in the structure represented by General Formula ($\alpha$) or ($\beta$), one hydrogen atom may be removed from any substituent to provide a monovalent structural moiety (the phosphor moiety M); however, it is preferable that, for example, one hydrogen atom is removed from the linking group U to provide a monovalent structural moiety, or one hydrogen atom is removed from a substituent, which can be adopted as $R^{12}$ (preferably $R^{12}$ on the carbon atom at which the numbers of bonded atoms from the indoline ring and the indolenine ring are the same), to provide a monovalent structural moiety.

Unless otherwise specified, the description of the substituent in General Formulae ($\alpha$) and ($\beta$) is a description of a substituent that is applied only to General Formulae ($\alpha$) and ($\beta$).

The physiologically active substance moiety which can be adopted as M can be used without particular limitation as long as it is a structural moiety consisting of a physiologically active substance. Examples of the physiologically active substance include a vitamin, a coenzyme, a hormone, an antibiotic, a neurotransmitter, and a cytokine. More specific examples thereof are calicheamicin, doxorubicin, daunorubicin, mitomycin C, bleomycin, cyclocytidine, vincristine, vinblastine, methotrexate, cisplatin or a derivative thereof, auristatin or a derivative thereof, maytansine or a derivative thereof, taxol or a derivative thereof, and camptothecin or a derivative thereof, which are described in paragraph [0095] of JP2021-020956A, and the description of paragraphs [0095] to [0099] of JP2021-020956A can be applied thereto.

The prodrug moiety which can be adopted as M can be used without particular limitation as long as it is a structural moiety consisting of a compound that is metabolized in vivo to be changed to a physiologically active substance. To the prodrug, for example, the description (a prodrug form of 2-pyrrolinodoxorubicin) in paragraph [0003] of JP2020-105187A can be applied.

The radioactive isotope-containing moiety which can be adopted as M can be used without particular limitation as long as it is a structural moiety containing a radioactive isotope that is capable of being used in the medical field. Examples of the radioactive isotope include iodine 131, indium 111, yttrium 90, lutetium 177, and copper 64, which are not limited thereto. The description of paragraph [0225] of JP2021-11483A can be applied thereto. Examples of the structural moiety containing a radioactive isotope include a structural moiety in which the radioactive isotope is bonded or coordinated to a nitrogen atom of an amino group or a tertiary amine, a sulfanyl group, an aryl group, a heteroaryl group, or the like. Examples of the structural moiety in which a nitrogen atom of a tertiary amine is coordinated to the radioactive isotope) include a structural moiety in which 1,4,7,10-tetraazacyclododecane-1,4,7,10-tetraacetic acid (DOTA) or the like is coordinated to the radioactive isotope to form a complex, and examples of the structural moiety in which a sulfanyl group is coordinated to the radioactive isotope include a structural moiety consisting of a complex such as copper (II) diacetylbis(N(4)-methylthiosemicarbazone).

<Compound represented by General Formula (III)>

The compound represented by General Formula (II) is preferably represented by General Formula (III).

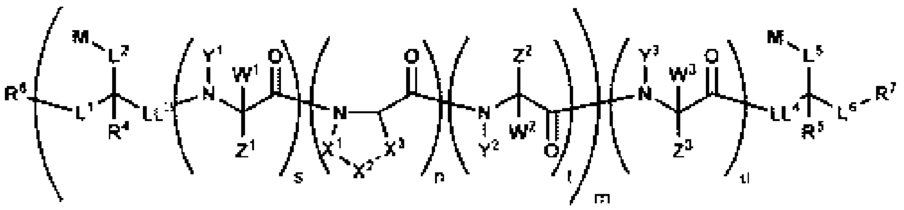

General Formula (III)

In the formula, $Y^1$ to $Y^3$, $Z^1$ to $Z^3$, and $W^1$ to $W^3$ represent a hydrogen atom, an alkyl group, an alkenyl group, an alkynyl group, an acyl group, an amino group, a hydroxy group, an alkoxy group, a sulfanyl group, an aryl group, a heteroaryl group, or an anionic group.

$LL^3$ and $LL^4$ represent a divalent linking group.

s, t, and u are an integer of 0 or more.

$R^4$ to $R^7$, $L^1$, $L^2$, $L^5$, $L^6$, $X^1$ to $X^3$, M, n, and m respectively have the same meanings as $R^4$ to $R^7$, $L^1$, $L^2$, $L^5$, $L^6$, $X^1$ to $X^3$, M, n, and m in General Formula (II) described above.

However, the structure parenthesized by s, t, or u is not allowed to be the structure represented by General Formula (I) described above. That is, $Y^1$ is not allowed to be bonded to $W^1$ or $Z^1$, $Y^2$ is not allowed to be bonded to $W^2$ or $Z^2$, or $Y^3$ is not allowed to be bonded to $W^3$ or $Z^3$ to form a structure represented by General Formula (I).

$Y^1$ to $Y^3$, $Z^1$ to $Z^3$, and $W^1$ to $W^3$ represent a hydrogen atom, an alkyl group, an alkenyl group, an alkynyl group, an acyl group, an amino group, a hydroxy group, an alkoxy group, a sulfanyl group, an aryl group, a heteroaryl group, or an anionic group.

The alkyl group, the alkenyl group, the alkynyl group, the acyl group, the amino group, the alkoxy group, the aryl group, the heteroaryl group, and the anionic group, which can be adopted as $Y^1$ to $Y^3$, $Z^1$ to $Z^3$, or $W^1$ to $W^3$, respectively have the same meanings as the alkyl group, the alkenyl group, the alkynyl group, the acyl group, the amino group, the alkoxy group, the aryl group, the heteroaryl group, and the anionic group in the substituent group T which will be described later, and the same also applies to the preferred ranges thereof.

The alkyl group, the alkenyl group, the alkynyl group, the acyl group, the amino group, the alkoxy group, the aryl group, and the heteroaryl group, which can be adopted as $Y^1$ to $Y^3$, $Z^1$ to $Z^3$, or $W^1$ to $W^3$, may be unsubstituted or may have a substituent, and examples of the substituent which may be contained therein include substituents in the substituent group T which will be described later. For example, an aryl group, a halogen atom, or an anionic group is preferable.

In addition, the substituent which may be contained in the above-described alkyl group, alkenyl group, alkynyl group, acyl group, amino group, alkoxy group, aryl group, and heteroaryl group, which can constitute $Y^1$ to $Y^3$, $Z^1$ to $Z^3$, or $W^1$ to $W^3$, may be substituted with a substituent selected from the substituent group T which will be described later, and for example, an anionic group is preferable.

$Y^1$ to $Y^3$ are preferably a hydrogen atom, an alkyl group, an aryl group, or a heteroaryl group, and more preferably a hydrogen atom or an alkyl group.

$W^1$ to $W^3$ are preferably a hydrogen atom, an alkyl group, an aryl group, or a heteroaryl group, and more preferably a hydrogen atom.

$Z^1$ to $Z^3$ are preferably an alkyl group, an aryl group, or a heteroaryl group, and more preferably an alkyl group.

In order to suppress the interaction between the structures represented by General Formula (I), the compound represented by General Formula (III) preferably has a group having a charge repulsive action, and from this viewpoint, at least one of $Z^1$, $Z^2$, or $Z^3$ is preferably a group including an anionic group, and more preferably an alkyl group including an

anionic group.

Here, the "at least one of $Z^1$, $Z^2$, or $Z^3$ is a group including an anionic group" means that at least one of the following conditions (Z1) to (Z3) is satisfied.

Condition (Z1): The s is an integer of 1 or more, and the $Z^1$ is a group including an anionic group.

Condition (Z2): The t is an integer of 1 or more, and the $Z^2$ is a group including an anionic group.

Condition (Z3): The u is an integer of 1 or more, and the $Z^3$ is a group including an anionic group.

In addition, the phrase "at least one of $Z^1$, $Z^2$, or $Z^3$ is an alkyl group including an anionic group" means that in a case where "a group including an anionic group" in the conditions (Z1) to (Z3) is read as "an alkyl group including an anionic group", at least one of the following condition (Z1), (Z2), or (Z3) is satisfied.

$LL^3$ and $LL^4$ represent a divalent linking group and are more preferably a divalent linking group obtained by combining one or two or more of an alkylene group, an alkenylene group, an alkynylene group, an arylene group, a heteroarylene group, -O-, >C=O, and $>NR^A$. $R^A$ represents a hydrogen atom or a substituent.

To the alkylene group, the alkenylene group, the alkynylene group, the arylene group, the heteroarylene group, and $>NR^A$, which can constitute $LL^3$ and $LL^4$, the description of the alkylene group, the alkenylene group, the alkynylene group, the arylene group, the heteroarylene group, and $>NR^A$, which can constitute $L^1$ to $L^7$ described above, can be preferably applied.

$LL^3$ is preferably >C=O, $>NR^A$, an alkylene group, an arylene group, or a heteroarylene group, and more preferably >C=O or $NR^A$. It is noted that $R^A$ is preferably a hydrogen atom.

$LL^4$ is preferably >C=O, $>NR^A$, an alkylene group, an arylene group, or a heteroarylene group, and more preferably >C=O or $>NR^A$. It is noted that $R^A$ is preferably a hydrogen atom.

s, t, and u are an integer of 0 or more.

The upper limit value of each of s, t, and u is not particularly limited and can be set to, for example, 20 or less, and it is preferably 10 or less and more preferably 5 or less. Among the above, s, t, and u are preferably an integer of 0 or 1.

It is noted that in a case where a structure can be classified into both a structure parenthesized by t and a structure parenthesized by u, it is preferentially classified into a structure parenthesized by u.

<Compound represented by General Formula (IV)>

The compound represented by General Formula (III) is preferably represented by General Formula (IV).

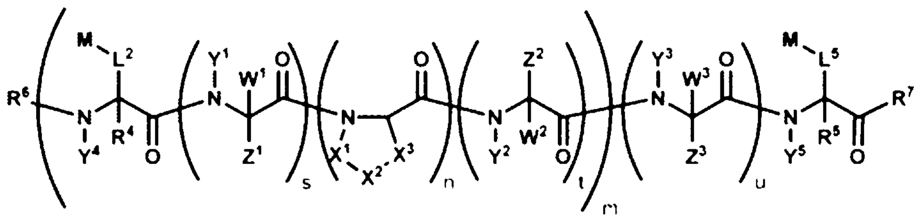

General Formula (IV)

In the formula, $Y^4$ and $Y^5$ represent a hydrogen atom, an alkyl group, an alkenyl group, an alkynyl group, an acyl group, an amino group, a hydroxy group, an alkoxy group, a sulfanyl group, an aryl group, a heteroaryl group, or an anionic group.

$R^4$ to $R^7$, $L^2$, $L^5$, $X^1$ to $X^3$, $Y^1$ to $Y^3$, $Z^1$ to $Z^3$, $W^1$ to $W^3$, M, n, m, s, t, and u respectively have the same meanings as $R^4$ to $R^7$, $L^2$, $L^5$, $X^1$ to $X^3$, $Y^1$ to $Y^3$, $Z^1$ to $Z^3$, $W^1$ to $W^3$, M, n, m, s, t, and u in General Formula (III) described above.

It is noted that in a case where the terminal of the compound on the $R^7$ side is a carboxy group or a substituent capable of being bonded to a biological substance of a type that is bonded through a carbonyl or a substituent capable of being bonded to a solid support of a type that is bonded through a carbonyl, the entire expression of "-C=O-$R^7$" in the formula may be interpreted as a carboxy group, a substituent capable of being bonded to a biological substance of a type that is bonded through a carbonyl, or a substituent capable of being bonded to a solid support of a type that is bonded through a carbonyl.

$Y^4$ and $Y^5$ represent a hydrogen atom, an alkyl group, an alkenyl group, an alkynyl group, an acyl group, an amino group, a hydroxy group, an alkoxy group, a sulfanyl group, an aryl group, a heteroaryl group, or an anionic group.

The alkyl group, the alkenyl group, the alkynyl group, the acyl group, the amino group, the alkoxy group, the aryl group, the heteroaryl group, and the anionic group, which can be adopted as $Y^4$ or $Y^5$, respectively have the same meanings as the alkyl group, the alkenyl group, the alkynyl group, the acyl group, the amino group, the alkoxy group, the aryl group, the heteroaryl group, and the anionic group in the substituent group T which will be described later, and the same also applies to the preferred ranges thereof.

It is noted that all the alkyl group, the alkenyl group, the alkynyl group, the acyl group, the amino group, the alkoxy group, the aryl group, and the heteroaryl group, which can be adopted as $Y^4$ or $Y^5$, may be an unsubstituted group or a

group having a substituent.

$Y^4$ and $Y^5$ are preferably a hydrogen atom or an alkyl group and more preferably a hydrogen atom.

<Compound represented by General Formula (V)>

The compound represented by General Formula (IV) is preferably represented by General Formula (V).

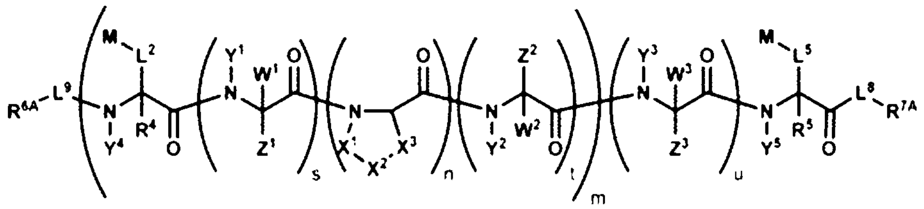 General Formula (V)

In the formula, $R^{6A}$ and $R^{7A}$ represent a hydrogen atom, a hydroxy group, a sulfanyl group, an alkyl group, an alkenyl group, an alkynyl group, an aryl group, an alkoxy group, a heteroaryl group, an amino group, an acyl group, an anionic group, a cationic group, or Q. However, at least one of $R^{6A}$ or $R^{7A}$ represents Q.

$L^8$ and $L^9$ represent a linking group. However, $L^9$ in a case where $R^{6A}$ is Q is a linking group in which the number of shortest-distance atoms that connect >$NY^4$ to $R^{6A}$ is 3 or more, and $L^8$ in a case where $R^{7A}$ is Q is a linking group in which the number of shortest-distance atoms that connect >C=O to $R^{7A}$ is 3 or more.

$R^4$, $R^5$, $L^2$, $L^5$, $X^1$ to $X^3$, $Y^1$ to $Y^5$, $Z^1$ to $Z^3$, $W^1$ to $W^3$, M, Q, n, m, s, t, and u respectively have the same meanings as $R^4$, $R^5$, $L^2$, $L^5$, $X^1$ to $X^3$, $Y^1$ to $Y^5$, $Z^1$ to $Z^3$, $W^1$ to $W^3$, M, Q, n, m, s, t, and u in General Formula (IV) described above.

$R^{6A}$ or $R^{7A}$ and $L^9$ or $L^8$ are each determined such that $R^{6A}$ or $R^{7A}$ is an unsubstituted group and $L^9$ or $L^8$ is the longest group. However, in a case where the group represented by - $L^9R^{6A}$ or -$L^8R^{7A}$ has an anionic group, a cationic group, or Q, it is determined such that the anionic group, the cationic group, or Q, which is located on the most terminal side (the $R^{6A}$ side in -$L^9R^{6A}$ or the $R^{7A}$ side in -$L^8R^{7A}$), is $R^{6A}$ or $R^{7A}$.

$R^{6A}$ and $R^{7A}$ represent a hydrogen atom, a hydroxy group, a sulfanyl group, an alkyl group, an alkenyl group, an alkynyl group, an aryl group, an alkoxy group, a heteroaryl group, an amino group, an acyl group, an anionic group, a cationic group, or Q. However, at least one of $R^{6A}$ or $R^{7A}$ represents Q.

The alkyl group, the alkenyl group, the alkynyl group, the aryl group, the alkoxy group, the heteroaryl group, the amino group, the acyl group, the anionic group, and the cationic group, which can be adopted as $R^{6A}$ and $R^{7A}$, respectively have the same meanings as the alkyl group, the alkenyl group, the alkynyl group, the aryl group, the alkoxy group, the heteroaryl group, the amino group, the acyl group, the anionic group, and the cationic group in the substituent group T which will be described later, and the same also applies to the preferred ranges thereof. However, all the groups are unsubstituted groups.

Q which can be adopted as $R^{6A}$ and $R^{7A}$ has the same meaning as Q described above, and the same applies to the preferred range thereof.

$R^{6A}$ is preferably an alkyl group, a sulfanyl group, an aryl group, a heteroaryl group, or Q, and more preferably an alkyl group or Q.

$R^{7A}$ is preferably an alkyl group, a sulfanyl group, an aryl group, a heteroaryl group, or Q, and more preferably an alkyl group or Q.

$L^8$ and $L^9$ represent a linking group.

The linking group which can be adopted as $L^8$ and $L^9$ is, for example, more preferably a divalent linking group obtained by combining one or two or more of an alkylene group, an alkenylene group, an alkynylene group, an arylene group, a heteroarylene group, -O-, >C=O, and >$NR^A$. $R^A$ represents a hydrogen atom or a substituent.

For the alkylene group, the alkenylene group, the alkynylene group, the arylene group, the heteroarylene group, and >$NR^A$, which can constitute $L^8$ and $L^9$, the description of the alkylene group, the alkenylene group, the alkynylene group, the arylene group, the heteroarylene group, and >$NR^A$, which can constitute $L^1$ to $L^7$ described above, can be preferably applied.

The substituent which may be contained in the alkylene group, the alkenylene group, the alkynylene group, the arylene group, and the heteroarylene group, which can constitute $L^8$ and $L^9$, is not particularly limited, and it is preferably selected from a substituent group T which will be described later. More preferred examples thereof include a halogen atom, an aryl group, and an alkyl group. In addition, the substituent which may be contained in the alkylene group, the alkenylene group, the alkynylene group, the arylene group, and the heteroarylene group, which can constitute $L^8$ or $L^9$, may be substituted with a substituent selected from the substituent group T which will be described later, and it is, for example, preferably an anionic group.

In addition, the number of substituents which may be contained in the alkylene group, the alkenylene group, the

alkynylene group, the arylene group, and the heteroarylene group, which can constitute $L^8$ or $L^9$, is not particularly limited as long as it can be adopted as a structure. The number thereof can be set to at least one or more, the upper limit thereof is not particularly limited, and for example, all hydrogen atoms in the alkylene group, the alkenylene group, the alkynylene group, the arylene group, and the heteroarylene group may be substituted with a substituent.

However, $L^9$ in a case where $R^{6A}$ is Q is a linking group in which the number of shortest-distance atoms that connect >$NY^4$ to $R^{6A}$ is 3 or more, and $L^8$ in a case where $R^{7A}$ is Q is a linking group in which the number of shortest-distance atoms that connect >C=O to $R^{7A}$ is 3 or more.

Regarding the divalent linking group $L^9$, "the number of shortest-distance atoms that connect >$NY^4$ to $R^{6A}$" means the number of atoms that constitute the shortest chain connecting >$NY^4$ to $R^{6A}$, and regarding the above-described divalent linking group $L^8$, "the number of shortest-distance atoms that connect >C=O to $R^{7A}$" means the number of atoms that constitute the shortest chain connecting >C=O to $R^{7A}$. It is noted that >$NY^4$ means >$NY^4$ that is directly bonded to $L^9$, and >C=O means >C=O that is directly bonded to $L^8$. For example, in a compound (4) that is used in Examples described later, since $L^8$ is $-NH(C_2H_4O)_4C_2H_4-$ and $R^{7A}$ is -COOH, the number of atoms that constitute the shortest chain connecting >C=O to $R^{7A}$ is 15.

In the compound represented by General Formula (V), at least one of $R^{6A}$ or $R^{7A}$ is Q. In a case where Q is linked to >C=O or >$NY^4$ by a linking group having the number of shortest-distance atoms of 3 or more, the distance from a bonding point between the main chain connecting $R^{6A}$ to $R^{7A}$ and M (that is, the carbon atom to which $L^5$ and $R^5$ are bonded or the carbon atom to which $L^2$ and $R^4$ are bonded) is increased, and the steric hindrance around Q is reduced, whereby the reactivity of the dye multimer in which the number of phosphor moieties M in the compound is 2 or more is improved with respect to the antibody, and the degree of fluorescence labeling (DOL) can be improved.

At least one of $R^{6A}$ or $R^{7A}$ described above is Q, where the number of shortest-distance atoms of the linking group that links this Q to >C=O or >$NY^4$ (the number of shortest-distance atoms that connect >$NY^4$ to $R^{6A}$ in a case where $R^{6A}$ is Q) and the number of shortest-distance atoms that connect >C=O to $R^{7A}$ in a case where $R^{7A}$ is Q) is preferably 3 to 60, more preferably 12 to 40, and still more preferably 15 to 40.

In the present invention, from the viewpoint of ease of synthesis, it is preferable that any one of $L^8$ or $L^9$ is a group including $-(L-O)_g-$, and it is more preferable that $L^8$ is a group including $-(L-O)_g-$.

$L^8$ is more preferably a linking group obtained by combining one or two or more of an alkylene group, -O-, >C=O, and >$NR^A$, and it is still more preferably an alkylene group, -O-, >C=O, >$NR^A$, a group in which -$NR^A$-alkylene group is bonded to a right side of a repeating unit (preferably having the repetition number of 1 to 20) represented by -[$NR^A$-alkylene-C(=O)]-, -$NR^A$-$(L-O)_g$-alkylene, or -C(=O)-$(L-O)_g$- alkylene.

$L^9$ is more preferably a linking group obtained by combining one or two or more of an alkylene group, -O-, >C=O, and >$NR^A$, and it is still more preferably an alkylene group, -O-, >C=O, >$NR^A$, a group in which -$NR^A$-alkylene group is bonded to a right side of a repeating unit (preferably having the repetition number of 1 to 20) represented by -[$NR^A$-alkylene-C(=O)]-, -$NR^A$-$(L-O)_g$-alkylene, or -C(=O)-$(L-O)_g$- alkylene.

<Compound represented by General Formula (VI)>

The compound represented by General Formula (II) is also preferably represented by General Formula (VI). The compound represented by General Formula (VI) corresponds to a compound in which $L^1$ and $L^4$ are >NH and $L^3$ and $L^6$ are >C=O, $L^7$ is a single bond, $R^4$ and $R^5$ are a hydrogen atom, and $L^1$ and $L^2$, and $L^4$ and $L^5$ are respectively bonded to each other to form a specific 5-membered ring in the compound represented by General Formula (II).

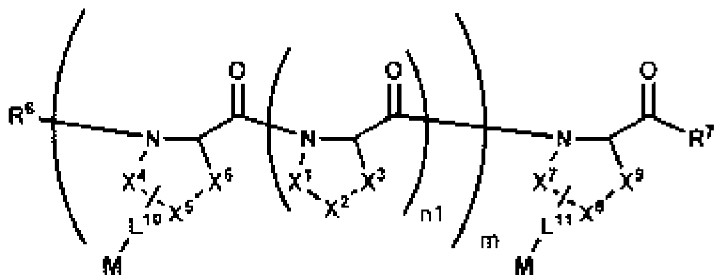
General Formula (VI)

In the formula, $X^4$ to $X^9$ represent -O-, -S-, >$NR^{101}$, or >$CR^{102}R^{103}$.

However, one of $X^4$, $X^5$, or $X^6$ is >$NR^{101}$ or >$CR^{102}R^{103}$, where $R^{101}$ is -$L^{10}$-M in a case where one of $X^4$, $X^5$, or $X^6$ is >$NR^{101}$ and $R^{102}$ or $R^{103}$ is -$L^{10}$-M in a case where one of $X^4$, $X^5$, or $X^6$ is >$CR^{102}R^{103}$. One of $X^7$, $X^8$, or $X^9$ is >$NR^{101}$ or >$CR^{102}R^{103}$, where $R^{101}$ is -$L^{11}$-M in a case where one of $X^7$, $X^8$, or $X^9$ is >$NR^{101}$ and $R^{102}$ or $R^{103}$ is -$L^{11}$-M in a case where one of $X^7$, $X^8$, or $X^9$ is >$CR^{102}R^{103}$.

$R^{101}$ to $R^{103}$, which are neither -$L^{10}$-M nor -$L^{11}$-M, represent a hydrogen atom, an alkyl group, an alkenyl group, an

alkynyl group, an aryl group, a heteroaryl group, an acyl group,$-NR^8R^9$, $-OR^{10}$, or an anionic group.

$L^{10}$ and $L^{11}$ represent a single bond or a divalent linking group.

n1 is an integer of 2 or more.

$R^6$ to $R^{10}$, $X^1$ to $X^3$, M, and m respectively have the same meanings as $R^6$ to $R^{10}$, $X^1$ to $X^3$, M, and m in General Formula (II) described above.

It is noted that in a case where the terminal of the compound on the $R^7$ side is a carboxy group or a substituent capable of being bonded to a biological substance of a type that is bonded through a carbonyl or a substituent capable of being bonded to a solid support of a type that is bonded through a carbonyl, the entire expression of "$-C=O-R^7$" in the formula may be interpreted as a carboxy group, a substituent capable of being bonded to a biological substance of a type that is bonded through a carbonyl, or a substituent capable of being bonded to a solid support of a type that is bonded through a carbonyl.

To $X^4$ to $X^6$, the description of $X^1$ to $X^3$ in General Formula (I) described above can be applied unless otherwise specified, except that one of $X^4$, $X^5$, or $X^6$ is $>NR^{101}$ or $>CR^{102}R^{103}$, where $R^{101}$ is $-L^{10}-M$ in a case where one of $X^4$, $X^5$, or $X^6$ is $>NR^{101}$ and $R^{102}$ or $R^{103}$ is $-L^{10}-M$ in a case where one of $X^4$, $X^5$, or $X^6$ is $>CR^{102}R^{103}$.

In addition, the description of $X^1$ to $X^3$ in General Formula (I) described above can be applied to $X^7$ to $X^9$ unless otherwise specified, except that one of $X^7$, $X^8$, or $X^9$ is $>NR^{101}$ or $>CR^{102}R^{103}$, where $R^{101}$ is $-L^{11}-M$ in a case where one of $X^7$, $X^8$, or $X^9$ is $>NR^{101}$ or $R^{102}$ or $R^{103}$ is $-L^{10}-M$ in a case where one of $X^7$, $X^8$, or $X^9$ is $>CR^{102}R^{103}$.

That is, the description of $X^1$ described above is applied to $X^4$ and $X^7$, the description of $X^2$ described above is applied to $X^5$ and $X^8$, and the description of $X^3$ described above is applied to $X^6$ and $X^9$, and the descriptions of $R^1$, $R^2$, and $R^3$ in General Formula (I) described above can be respectively applied to $R^{101}$, $R^{102}$, and $R^{103}$ which are neither $-L^{10}-M$ nor $-L^{11}-M$.

In $>NR^{101}$ and $>CR^{102}R^{103}$ among $X^4$ to $X^9$, which have neither $-L^{10}-M$ nor $-L^{11}-M$, $R^{101}$ is preferably an alkyl group, and $R^{102}$ and $R^{103}$ are preferably a hydrogen atom.

Regarding the two groups among $X^4$ to $X^6$, which do not have $-L^{10}-M$ described above, it is preferable that at least one thereof is $>CR^{102}R^{103}$, it is more preferable that at least one thereof is $>CR^{102}R^{103}$ and the remaining one is -O-, -S-, or $>CR^{102}R^{103}$, and it is still more preferable that both the two are $>CR^{102}R^{103}$.

Regarding the two groups among $X^7$ to $X^9$, which do not have $-L^{11}-M$ described above, it is preferable that at least one thereof is $>CR^{102}R^{103}$, it is more preferable that at least one thereof is $>CR^{102}R^{103}$ and the remaining one is -O-, -S-, or $>CR^{102}R^{103}$, and it is still more preferable that both the two are $>CR^{102}R^{103}$.

Among $X^4$ to $X^6$, $>NR^{101}$ or $>CR^{102}R^{103}$ which has $-L^{10}-M$ as any one of $R^{101}$ to $R^{103}$ is preferably a group represented by $>CR^{102}R^{103}$ where $R^{103}$ is $-L^{10}-M$, and it is more preferably a group represented by $>CR^{102}R^{103}$ where $R^{102}$ is a hydrogen atom and $R^{103}$ is $-L^{10}-M$.

Among $X^7$ to $X^9$, $>NR^{101}$ or $>CR^{102}R^{103}$ which has $-L^{11}-M$ as any one of $R^{101}$ to $R^{103}$ is preferably a group represented by $>CR^{102}R^{103}$ where $R^{103}$ is $-L^{11}-M$, and it is more preferably a group represented by $>CR^{102}R^{103}$ where $R^{102}$ is a hydrogen atom and $R^{103}$ is $-L^{11}-M$.

Among $X^4$ to $X^6$, the group having $-L^{10}-M$ is not particularly limited; however, it is preferably $X^5$.

Among $X^7$ to $X^9$, the group having $-L^{11}-M$ is not particularly limited; however, it is preferably $X^8$.

$L^{10}$ and $L^{11}$ are preferably a single bond, or a group obtained by combining one or two or more of an alkylene group, an alkenylene group, an alkynylene group, an arylene group, a heteroarylene group, -O-, -S-, >C=O, and $>NR^A$, more preferably a group obtained by combining one or two or more of an alkylene group, an alkenylene group, an alkynylene group, an arylene group, a heteroarylene group, -O-, -S-, >C=O, and $>NR^A$, and still more preferably a group represented by $* -L^{x1}-L^{y1}-**$.

To the alkylene group, the alkenylene group, the alkynylene group, the arylene group, the heteroarylene group, and $>NR^A$, which can constitute $L^{10}$ and $L^{11}$, the description of the alkylene group, the alkenylene group, the alkynylene group, the arylene group, the heteroarylene group, and $>NR^A$, which can constitute $L^2$ and $L^5$ described above, can be applied unless otherwise specified.

$L^{x1}$ is a single bond or a group obtained by combining one or two or more of an alkylene group, an alkenylene group, an alkynylene group, an arylene group, and a heteroarylene group, and $L^{y1}$ is a single bond, -O, -, -S-, >C=O, or $>NR^A$. It is noted that in $*-L^{x1}-L^{y1}-**$, * represents a bonding site to $X^4$ to $X^9$, and ** represents a bonding site to M.

Among the groups represented by $* -L^{x1}-L^{y1}-**$, $L^{10}$ and $L^{11}$ are preferably a group in which $L^{y1}$ is a single bond, -S-, >C=O, or $>NR^A$, more preferably a group in which $L^{y1}$ is >C=O or $>NR^A$, and still more preferably a group in which $L^{x1}$ is a single bond and $L^{y1}$ is >C=O or $>NR^A$.

It is noted that in the compound represented by General Formula (VI), it suffices that the number of shortest-distance atoms in the linking chain (each linking chain of the linking chain including $L^{10}$ and the linking chain including $L^{11}$) that connects M to any one of $X^4$ to $X^9$ is, for example, 1 to 60, where 1 to 40 is preferable. In a case where M is a phosphor moiety, the above-described number of shortest-distance atoms means the number of atoms that constitute the shortest chain in the linking chain that connects a conjugated structural moiety for exhibiting fluorescence to any one of $X^4$ to $X^9$ in the phosphor moiety M.

It is noted that in the compound represented by General Formula (VI), it is also preferable that the structure represented by $-(CH_2-CH_2-O)_b-$ described above (b is also as described above) is provided at any portion of the linking chain represented by "-linking group ZZZ-$L^{10}$-" in the structure represented by "the conjugated structural moiety of M - the linking group ZZZ-$L^{10}$- described above" and any portion of the linking chain represented by "-linking group ZZZ-$L^{11}$-" in the structure represented by "the conjugated structural moiety of M - the linking group ZZZ-$L^{11}$- described above".

n1 is an integer of 2 or more.

In the compound represented by General Formula (VI), the linker main chain connecting the two phosphor moieties is rigid and the dye association can be further suppressed as compared with the compound represented by any one of General Formulae (III) to (V) described above. Therefore, the lower limit value of n1 is preferably an integer of 3 or more, and it is more preferably an integer of 5 or more from the viewpoint that a sufficient effect of improving the fluorescence intensity is obtained. The same applies even in a case where the number of phosphor moieties is two or more and a case where the number thereof is 2 or more.

The upper limit value of n1 is, for example, preferably an integer of 36 or less, more preferably an integer of 24 or less, and still more preferably an integer of 18 or less. That is, n1 is preferably an integer of 3 to 36 and more preferably an integer of 5 to 24.

<Compound represented by General Formula (VII)>

The compound represented by General Formula (VI) is preferably represented by General Formula (VII).

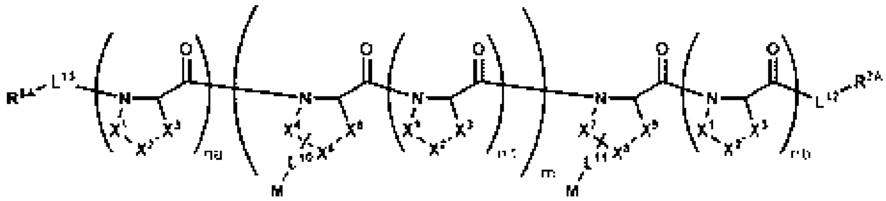

General Formula (VII)

In the formula, $R^{6A}$ and $R^{7A}$ represent a hydrogen atom, a hydroxy group, a sulfanyl group, an alkyl group, an alkenyl group, an alkynyl group, an aryl group, an alkoxy group, a heteroaryl group, an amino group, an acyl group, an anionic group, a cationic group, or Q. However, at least one of $R^{6A}$ or $R^{7A}$ represents Q.

$L^{12}$ and $L^{13}$ represent a linking group.

na and nb are an integer of 0 or more.

$L^{10}$, $L^{11}$, $X^1$ to $X^9$, M, Q, n1, and m respectively have the same meanings as $L^{10}$, $L^{11}$, $X^1$ to $X^9$, M, Q, n1, and m in General Formula (VI) described above.

It is noted that in a case where the terminal of the compound on the $R^{7A}$ side is a carboxy group or a substituent capable of being bonded to a biological substance of a type that is bonded through a carbonyl or a substituent capable of being bonded to a solid support of a type that is bonded through a carbonyl, the entire expression of "-C=O-$L^{12}$-$R^{7A}$" in the formula may be interpreted as a carboxy group, a substituent capable of being bonded to a biological substance of a type that is bonded through a carbonyl, or a substituent capable of being bonded to a solid support of a type that is bonded through a carbonyl.

Unless otherwise specified, $R^{6A}$ and $R^{7A}$ have the same meanings as $R^{6A}$ and $R^{7A}$ in General Formula (V) described above. That is, the description of $R^{6A}$ and $R^{7A}$ in General Formula (V) can be applied to $R^{6A}$ and $R^{7A}$.

$R^{6A}$ or $R^{7A}$ and $L^{12}$ or $L^{13}$ are each determined such that $R^{6A}$ or $R^{7A}$ is an unsubstituted group and $L^{12}$ or $L^{13}$ is the longest group. However, in a case where the group represented by $-L^{13}R^{6A}$ or $-L^{12}R^{7A}$ has an anionic group, a cationic group, or Q, it is determined such that the anionic group, the cationic group, or Q, which is located on the most terminal side (the $R^{6A}$ side in $-L^{13}R^{6A}$ or the $R^{7A}$ side in $-L^{12}R^{7A}$), is $R^{6A}$ or $R^{7A}$.

$L^{12}$ and $L^{13}$ represent a linking group.

The linking group which can be adopted as $L^{12}$ and $L^{13}$ is, for example, more preferably a divalent linking group obtained by combining one or two or more of an alkylene group, an alkenylene group, an alkynylene group, an arylene group, a heteroarylene group, -O-, >C=O, and >$NR^A$. $R^A$ represents a hydrogen atom or a substituent.

To the alkylene group, the alkenylene group, the alkynylene group, the arylene group, the heteroarylene group, and >$NR^A$, which can constitute $L^{12}$ and $L^{13}$, the description of the alkylene group, the alkenylene group, the alkynylene group, the arylene group, the heteroarylene group, and >$NR^A$, which can constitute $L^8$ or $L^9$ in General Formula (V) described above, can be applied.

In the compound represented by General Formula (VII), it is preferable that (A) na is an integer of 1 or more and $R^{6A}$ is Q, and/or (B) nb is an integer of 1 or more and $R^{7A}$ is Q.

However, the number of shortest linking atoms of $L^{13}$ is 7 or less in a case of the (A), and the number of shortest

linking atoms of $L^{12}$ is 7 or less in a case of the (B).

Regarding the divalent linking group $L^{13}$, "the number of shortest linking atoms in $L^{13}$" means the number of atoms that constitute the shortest chain connecting N directly bonded to $L^{13}$ to $R^{6A}$, in a case where na is an integer of 1 or more, where the N is one of those indicated in the structure parenthesized in $(\ )_{na}$, and it means the number of atoms that constitute the shortest chain connecting N directly bonded to $L^{13}$ to $R^{6A}$, in a case where na is 0, where the N is one of those indicated in the structure parenthesized in $(\ )_{m}$.

In addition, Regarding the divalent linking group $L^{12}$, "the number of shortest linking atoms in $L^{12}$" means the number of atoms that constitute the shortest chain connecting >C=O directly bonded to $L^{12}$ to $R^{7A}$, in a case where nb is an integer of 1 or more, where the >C=O is one of those indicated in the structure parenthesized in $(\ )_{nb}$, and it means the number of atoms that constitute the shortest chain connecting >C=O to $R^{7A}$, which is indicated on the left side of the structure parenthesized in $(\ )_{nb}$.in General Formula (VII), in a case where nb represents 0.

For example, in compounds (1) to (3) that is used in Examples described later, since $L^{12}$ is $-NHC_2H_4-$ and $R^{7A}$ is -COOH, the number of shortest linking atoms of $L^{12}$ is 3.

In the compound represented by General Formula (VII), at least one of $R^{6A}$ or $R^{7A}$ is Q. In particular, it is conceived that in a case of satisfying (A) and/or (B) described above, the mobility of the linker main chain is reduced, and thus the dye association can be further suppressed.

The number of shortest linking atoms of $L^{12}$ and the number of shortest linking atoms of $L^{13}$, which are described, are preferably 1 to 5 and more preferably 1 to 4.

In the present invention, from the viewpoint of ease of synthesis, it is also preferable that any one of $L^{12}$ or $L^{13}$ is a group including $-(L-O)_g-$, and it is also more preferable that $L^{12}$ is a group including $-(L-O)_g-$.

$L^{12}$ is more preferably a linking group obtained by combining one or two or more of an alkylene group, -O-, >C=O, and $>NR^A$, more preferably an $-NR^A$-alkylene group or an $-NR^A-(L-O)_g$-alkylene group, and still more preferably an $-NR^A$-alkylene group.

$L^{13}$ is more preferably a linking group obtained by combining one or two or more of an alkylene group, -O-, >C=O, and $>NR^A$, and still more preferably $>NR^A$ or >C=O.

na and nb are an integer of 0 or more.

In a case where $R^{6A}$ is Q, na is preferably an integer of 0 to 20, more preferably an integer of 2 to 20, and still more preferably an integer of 4 to 18.

In a case where $R^{7A}$ is Q, nb is preferably an integer of 0 to 20, more preferably an integer of 2 to 20, and still more preferably an integer of 4 to 18. It is noted that the lower limit value of nb may be 0, and in this case, nb is preferably an integer of 0 to 20 and more preferably an integer of 0 to 18.

In a case where $R^{6A}$ is not Q, na is preferably an integer of 0 to 20, more preferably an integer of 0 to 10, and still more preferably an integer of 0 to 5.

In a case where $R^{7A}$ is not Q, nb is preferably an integer of 0 to 20, more preferably an integer of 0 to 10, and still more preferably an integer of 0 to 5.

In a case where a compound represented by any one of General Formulae (II) to (VII) among the fluorescent dyes (F) is obtained by using a peptide synthesis method, the structure is, in general, such that the C-terminal structure is on the right side of the paper surface, and the N-terminal structure is on the left side of the paper surface.

The fluorescent dye (F) preferably contains at least one substituent represented by Q, that is, at least one of a carboxy group, a substituent capable of being bonded to a biological substance, or a substituent capable of being bonded to a solid support.

The fluorescent dye such as the fluorescent dye (F) can be bonded to a biological substance by the carboxy group or a substituent capable of being bonded to a biological substance described later, whereby a targeted fluorescent dye-labeled biomolecule can be obtained. It is noted that a substituent capable of being bonded to a biological substance can be easily derived from a carboxy group by a conventional method.

In addition, the fluorescent dye can be bonded to a solid support such as microparticles by the carboxy group or a substituent capable of being bonded to a solid support described later, whereby a targeted labeled microparticles can be obtained. The microparticle is not particularly limited; however, examples thereof include small particles that are useful for bonding to the fluorescent dye, including a glass bead, a non-polymer bead such as a magnetic bead, and a polymer bead. In a certain embodiment, the microparticle includes a polystyrene bead. The small particle is not particularly limited as long as it has a size that is commonly used in the fluorescence labeling; however, the average particle diameter thereof is generally 10 nm to 10 $\mu$m. It is noted that a substituent capable of being bonded to a solid support can be easily derived from a carboxy group by a conventional method.

In the present invention, for convenience, the substituent capable of being bonded to a biological substance and the substituent capable of being bonded to a solid support do not include a carboxy group, where "the substituent capable of being bonded to a biological substance" includes a substituent capable of being bonded to a biological substance, which is derived from a carboxy group, and "the substituent capable of being bonded to a solid support" includes a substituent capable of being bonded to a solid support, which is derived from a carboxy group. However, as described above, it is also

possible to carry out bonding to a biological substance or a solid support by a carboxy group.

In the fluorescent dye (F), a position where the substituent represented by Q is provided is not particularly limited; however, the substituent represented by Q is preferably provided in a structure other than the structure represented by General Formula (I) and the phosphor moiety and more preferably provided in at least one of $R^6$ or $R^7$ in the compound represented by General Formula (II).

It suffices that the number of substituents represented by Q in the fluorescent dye (F) is at least 1 or more in total, and it is preferably 1 to 3, more preferably 1 or 2, and still more preferably 1, from the viewpoint of the quantification of the target substance to be detected.

In addition, from the viewpoint of imparting sufficient hydrophilicity as a compound, the fluorescent dye (F) also preferably has an anionic group be described later at a position other than the phosphor moiety, and for example, it preferably has one or more anionic groups, more preferably 1 to 8 anionic groups, and still more preferably 1 to 6 anionic groups.

The position of the anionic group is not particularly limited unless specified otherwise, and examples of the group having the anionic group preferably include $Z^1$ to $Z^3$ or $X^1$ to $X^3$ in the compound represented by General Formula (III).

Specific examples of the fluorescent dye (F) will be shown below; however, the present invention is not limited to these compounds. In the following specific examples, the sulfo group and the phosphonooxy group may adopt a salt structure in which a hydrogen ion is dissociated. In the following specific examples, Dye indicates a phosphor moiety.

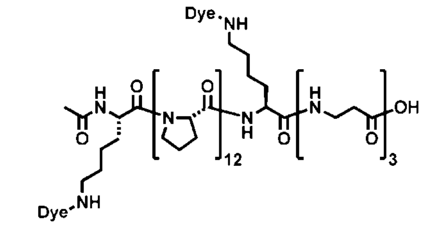

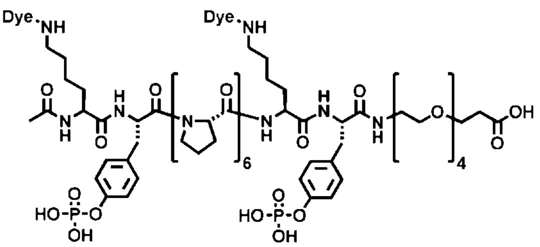

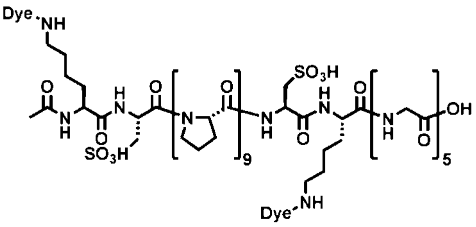

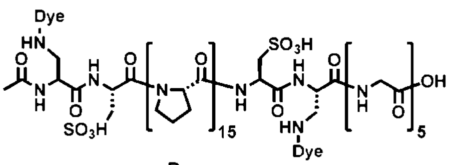

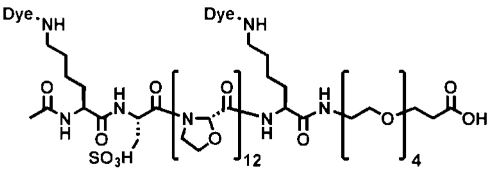

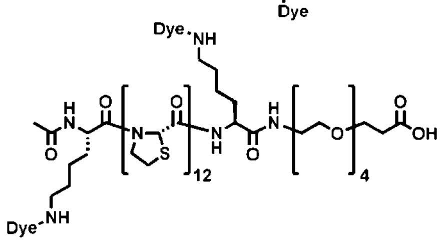

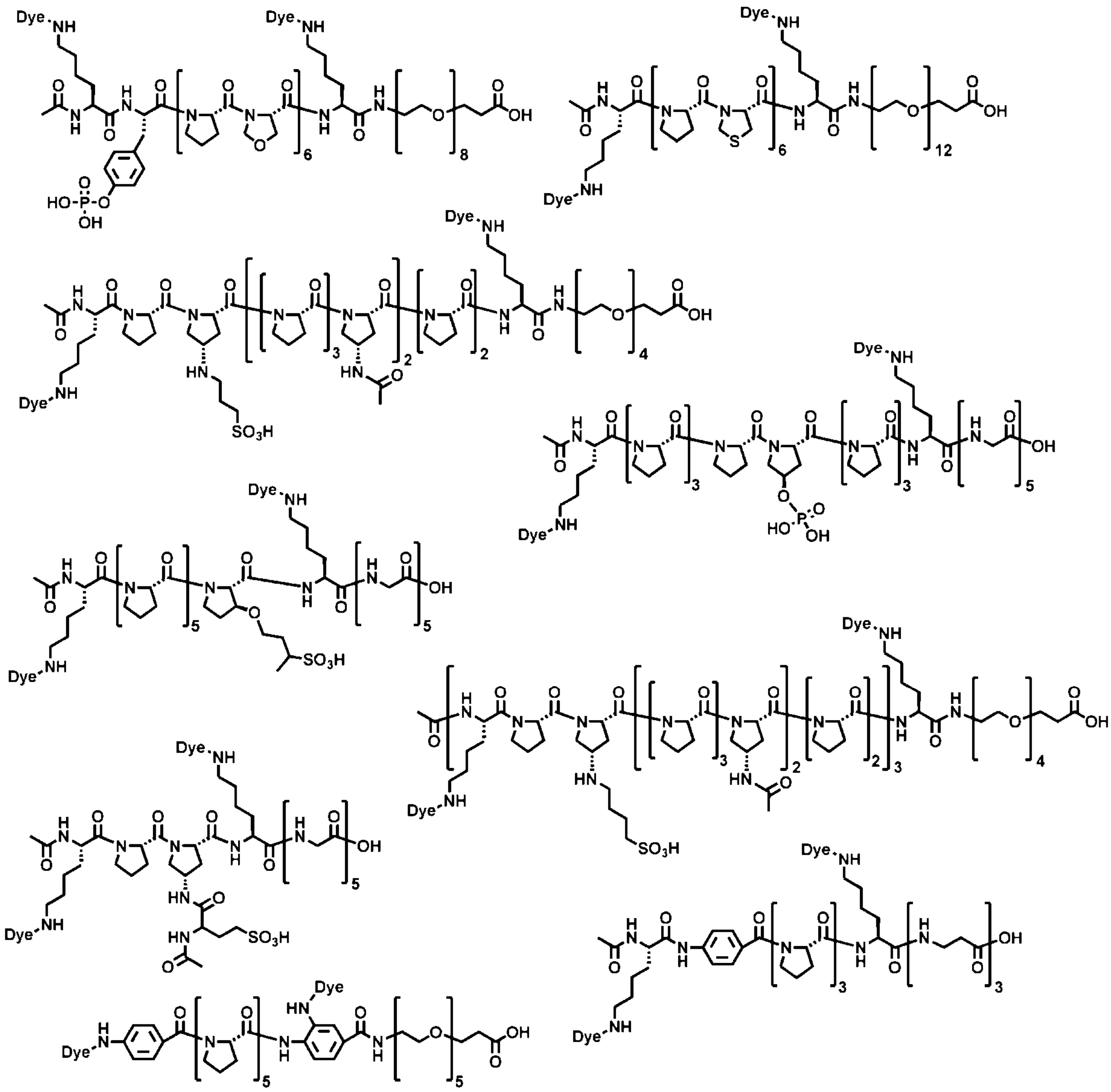

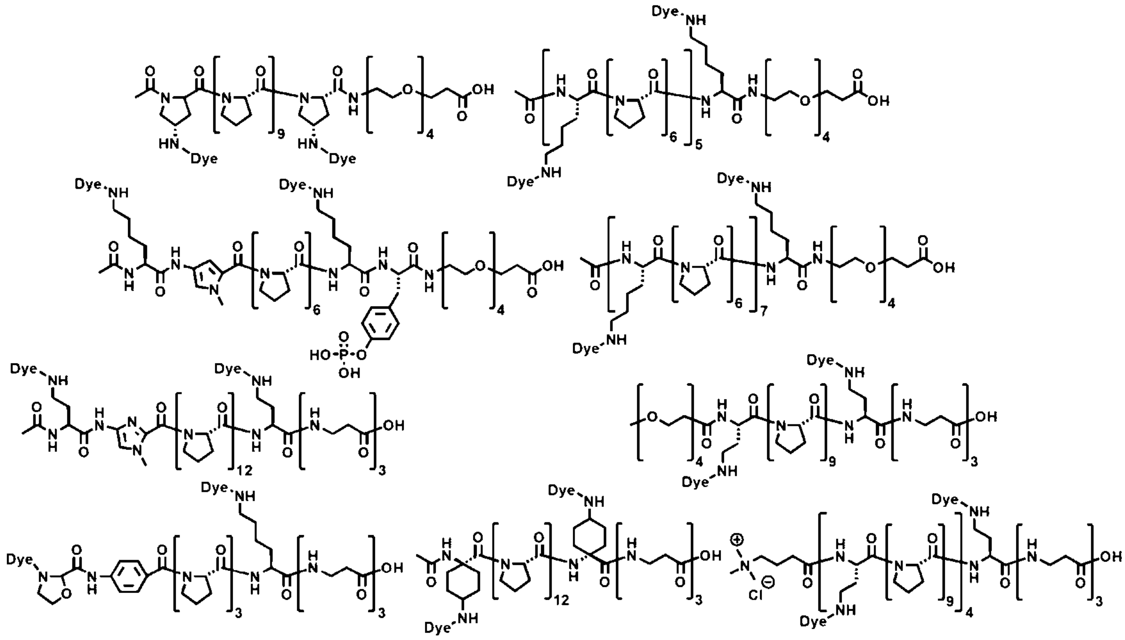

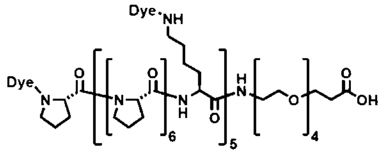

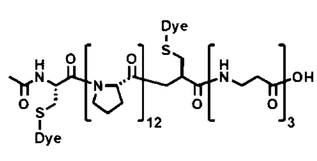

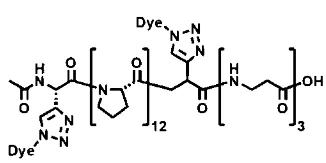

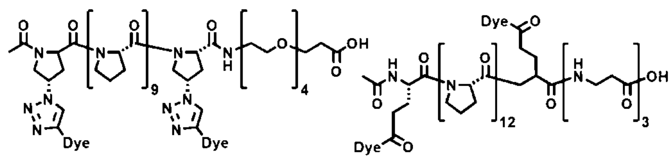

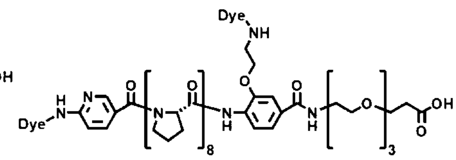

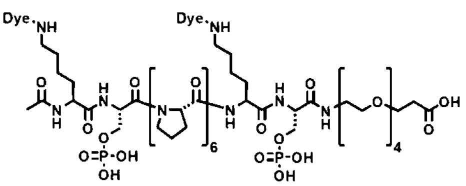

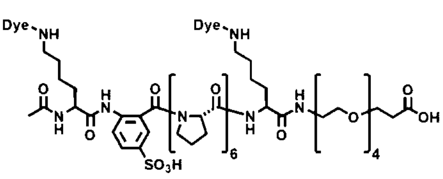

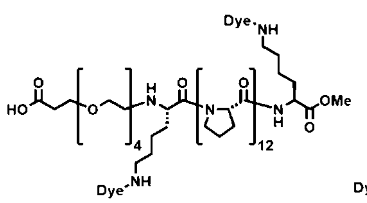

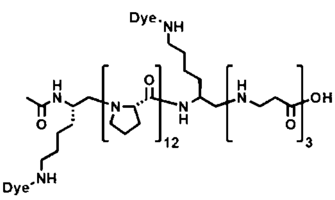

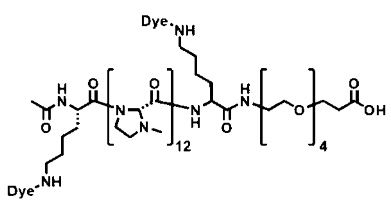

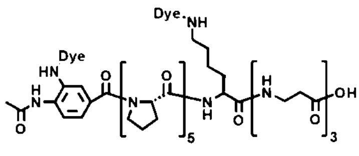

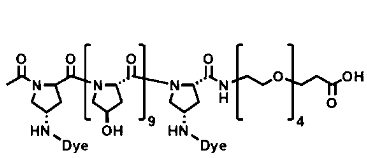

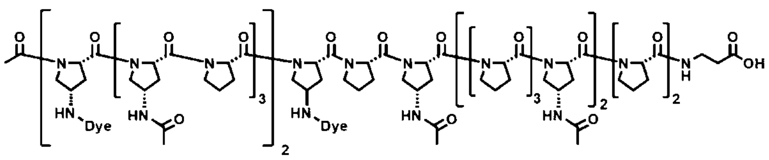

The fluorescent dye such as the fluorescent dye (F) can be bonded to a biological substance such as a protein (including a peptide), an amino acid, a nucleic acid, a nucleotide, a sugar chain, or a lipid, by at least one substituent capable of being bonded to a biological substance, where the substituent is contained in the compound, and the compound can be used as a fluorescent dye-labeled biomolecule.

The substituent capable of being bonded to a biological substance can be used without particular limitation as long as it is a group for acting (including adhering) or bonding to a biological substance, and examples thereof include the substituents described in WO2002/026891A. Specifically, the description of the electrophilic group and the nucleophilic group described in Table 2 of WO2002/026891A and the reactive group Rx described on page 18, line 16 to page 19, line 13 of WO2002/026891A can be applied to the present invention.

Specific examples of "the substituent capable of being bonded to a biological substance" include the following structures.

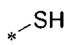
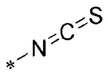
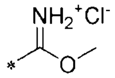
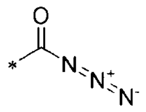
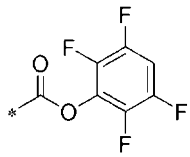
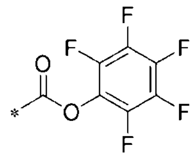

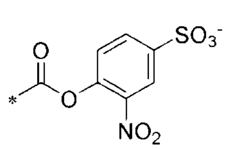
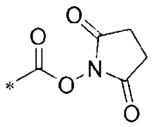
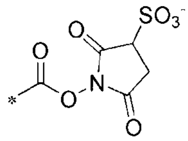
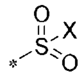
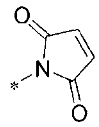
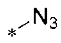
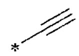

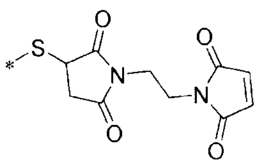
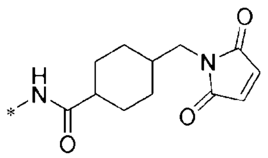
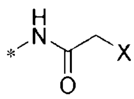
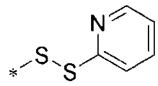
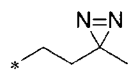

X means a halogen atom such as an iodine atom or a bromine atom. * represents a bonding site.

In addition to the above, it is possible to use a peptide structure (a polyamino acid structure), a long-chain alkyl group, or the like can be used as the "substituent capable of being bonded to a biological substance".

Among them, preferred examples thereof include an N-hydroxysuccinimide ester structure (an NHS ester structure), a succinimide structure, a maleimide structure, an azide group, an acetylene group, a peptide structure (a polyamino acid structure), a long-chain alkyl group (preferably having 12 to 30 carbon atoms), and a quaternary ammonium group.

Among the fluorescent dyes (F), specific examples of the compound having at least one substituent capable of being bonded to a biological substance also include, as a specific example, a form in which the carboxy group in the above-described exemplary fluorescent dye (F) is appropriately replaced with the substituent capable of being bonded to a biological substance described above. It is noted that the present invention is not limited to these compounds. For example, in the specific examples thereof, a group having a dissociative hydrogen atom such as a carboxy group or a sulfo group may adopt a salt structure by a hydrogen atom being dissociated therefrom.

The fluorescent dye such as the fluorescent dye (F) can be bonded to a solid support such as the above-described microparticles by a substituent capable of being bonded to at least one solid support, the substituent being contained in the compound, and it can be used as a solid support reagent.

The substituent that can be bonded to a solid support can be used without particular limitation as long as it is a group for acting on (including adhering to) or bonding to a solid support, and examples thereof preferably include the substituents described as the above-described substituent capable of being bonded to a biological substance. Among them, preferred examples thereof include an N-hydroxysuccinimide ester (NHS ester) structure, a succinimide structure,

and a maleimide structure.

Among the fluorescent dyes (F), specific examples of the compound having at least one substituent capable of being bonded to a solid support also include, as a specific example, a form in which the carboxy group in the above-described exemplary fluorescent dye (F) is appropriately replaced with the substituent capable of being bonded to a solid support described above. It is noted that the present invention is not limited to these compounds. For example, in the specific examples thereof, a group having a dissociative hydrogen atom such as a carboxy group or a sulfo group may adopt a salt structure by a hydrogen atom being dissociated therefrom.

The fluorescent dye (F) can be synthesized by a conventional method. For example, it can be synthesized based on the peptide synthesis such as solid phase peptide synthesis, and a method that uses an automatic peptide synthesizer described in WO2018/174078A can also be preferably applied. The phosphor moiety, the physiologically active substance moiety, the prodrug moiety, and the radioactive isotope-containing moiety can also be synthesized based on a conventional method and introduced into the fluorescent dye (F).

A compound having a substituent capable of being bonded to a biological substance can also be synthesized according to a conventional method. For example, Bioconjugate Techniques (Third Edition, written by Greg T. Hermanson) can be referred to.

<Fluorescent dye-labeled biomolecule>

The fluorescent dye-labeled biomolecule is a substance in which a fluorescent dye is bonded to a biological substance. In particular, since the fluorescent dye (F) has fluorescence due to the phosphor moiety and exhibits an excellent fluorescence intensity, it can be preferably used for a fluorescent dye-labeled biomolecule. The bond between the fluorescent dye and a biological substance may have a form in which the fluorescent dye and the biological substance are directly bonded or a form of being linked via a linking group.

Preferred examples of the biological substance include a protein (including a peptide), an amino acid, a nucleic acid, a nucleotide, a sugar chain, and a lipid. Preferred examples of the protein include an antibody, and preferred examples of the lipid include a phospholipid, a fatty acid, and sterol, where a phospholipid is more preferable.

Among the above biological substances, the clinically useful substance is not particularly limited; however, examples thereof include immunoglobulins such as immunoglobulin (Ig) G, IgM, IgE, IgA, and IgD; blood plasma proteins such as complement, C-reactive protein (CRP), ferritin, $\alpha_1$ microglobulin, $\beta_2$ microglobulin, and antibodies thereof; tumor markers such as $\alpha$-fetoprotein, carcinoembryonic antigen (CEA), prostate acid phosphatase (PAP), carbohydrate antigen (CA) 19-9, and CA-125, and antibodies thereof; hormones such as luteinizing hormone (LH), follicle-stimulating hormone (FSH), human ciliated gonadotropin (hCG), estrogen, and insulin, and antibodies thereof; and viral infection-related substances of viruses such HIV and ATL, hepatitis B virus (HBV)-related antigens (HBs, HBe, and HBc), human immunodeficiency virus (HIV), adult T-cell leukemia (ATL), and antibodies thereof.

The examples thereof further include bacteria such as Corynebacterium diphtheriae, Clostridium botulinum, mycoplasma, and Treponema pallidum, and antibodies thereof; protozoa such as Toxoplasma, Trichomonas, Leishmania, Trypanosoma, and malaria parasites, and antibodies thereof; embryonic stem (ES) cells such as ELM3, HM1, KH2, v6.5, v17.2, v26.2 (derived from mice, 129, 129/SV, C57BL/6, and BALB/c), and antibodies thereof; antiepileptic drugs such as phenytoin and phenobarbital; cardiovascular drugs such as quinidine and digoxin; anti-asthma drugs such as theophylline; drugs such as antibiotics such as chloramphenicol and gentamicin, and antibodies thereof; and enzymes, extracellular toxins (for example, streptolysin O), and the like, and antibodies thereof. In addition, antibody fragments such as Fab'2, Fab, and Fv can also be used.

Examples of the specific form in which the fluorescent dye and a biological substance interact with each other to be bonded to each other includes the forms described below.

Examples thereof include

i) a non-covalent bond (for example, a hydrogen bond and an ion bond including chelate formation) or a covalent bond between a peptide in a fluorescent dye and a peptide in a biological substance,

ii) a van der Waals force between a long-chain alkyl group in a fluorescent dye and a lipid bilayer, a lipid, and the like in a biological substance,

iii) an amide bond formed by a reaction between an N-hydroxysuccinimide ester (NHS ester) in a fluorescent dye and an amino group in a biological substance,

iv) a thioether bond formed by a reaction between a maleimide group in a fluorescent dye and a sulfanyl group (-SH) in a biological substance, and

v) a formation of a triazole ring by a Click reaction between an azide group in a fluorescent dye and an acetylene group in a biological substance or a Click reaction between an acetylene group in a fluorescent dye and an azide group in a biological substance.

Provided that in the form of the above i), the peptide in the fluorescent dye is not particularly limited as long as it is a peptide that is capable of forming a non-covalent bond or a covalent bond with a peptide in the biological substance. Preferred examples of the position where such a peptide is provided include $R^6$ or $R^7$ in General Formula (II) as described above.

In addition to the forms i) to v) described above, the bonding can be formed, for example, in the form described in Lucas C. D. de Rezende and Flavio da Silva Emery. A Review of the Synthetic Strategies for the Development of BODIPY Dyes for Conjugation with Proteins, Orbital: The Electronic Journal of Chemistry, 2013, Vol 5, No. 1, p. 62-83. In addition, the method described in the same document can be appropriately referred to for the preparation of the fluorescent dye-labeled biomolecule.

Among the fluorescent dye such as the fluorescent dye (F), examples of the fluorescent dye-labeled biomolecule, which is obtained from a fluorescent dye having a substituent capable of being bonded to a biological substance and a biological substance that is bonded to the compound by an interaction includes the compound in which a moiety other than the substituent capable of being bonded to a biological substance is replaced with the fluorescent dye (F) in the compound according to the embodiment of the present invention, and a product thereof, in the description of the compound example and the product in paragraph [0038] of JP2019-172826A. However, the present invention is not limited to these fluorescent dye-labeled biomolecules and the like.

The fluorescent dye-labeled biomolecule, which is obtained from the fluorescent dye (F), can exhibit an excellent fluorescence intensity and stably detect fluorescence emitted from the fluorescent dye-labeled biomolecule excited by light irradiation. Therefore, the fluorescent dye-labeled biomolecule obtained from the fluorescent dye (F) can be suitably used in combination with the fluorescence intensity enhancer according to the embodiment of the present invention.

In addition, commercially available primary antibodies and secondary antibodies can also be used without particular limitation as the fluorescent dye-labeled biomolecule. Examples thereof include Alexa Fluor Plus 405, Alexa Fluor Plus 488, Alexa Fluor Plus 555, Alexa Fluor Plus 594, Alexa Fluor Plus 647, Alexa Fluor Plus 680, and Alexa Fluor Plus 800, all of which are trade names of products manufactured by Thermo Fisher Scientific Inc.

In addition, a fluorescent dye-labeled biomolecule obtained from a commercially available fluorescent dye can also be used without particular limitation.

<Fluorescence detection using fluorescent dye-labeled biomolecule>

The fluorescence detection performed using a fluorescent dye-labeled biomolecule generally includes the following steps (i) to (iii) or (iv) to (vii). The fluorescence detection including the steps (i) to (iii) corresponds to the direct method using a primary antibody fluorescently labeled with the fluorescent dye, and the fluorescence detection including the steps (iv) to (vii) corresponds to the indirect method using a secondary antibody fluorescently labeled with the fluorescent dye.

(i) The step of preparing each of the following (a) and (b)

(a) A sample containing a targeted biological substance (hereinafter, also referred to as a "target biological substance")
(b) A fluorescent dye-labeled biomolecule (hereinafter, also referred to as a "fluorescent dye-labeled biomolecule A") in which a biological substance capable of binding to the target biological substance in the above (a) (hereinafter, also referred to as a "primary biological substance") and a fluorescent dye are bound to each other

(ii) The step of preparing a conjugate (hereinafter, also referred to as a "fluorescently labeled conjugate A") in which the target biological substance in the above (a) is bonded to the primary biological substance in the fluorescent dye-labeled biomolecule A in the above (b)
(iii) The step of irradiating the fluorescently labeled conjugate A with light having the range of the wavelength which is absorbed by the fluorescent dye-labeled biomolecule A, and detecting the fluorescence emitted by the fluorescent dye-labeled biomolecule A
(iv) The step of preparing each of the following (c) to (e)

(c) A sample containing a target biological substance
(d) A biological substance capable of binding to the target biological substance in the above (c) (hereinafter, also referred to as a "primary biological substance")
(e) A fluorescent dye-labeled biomolecule (hereinafter, also referred to as a "fluorescent dye-labeled biomolecule B") in which a biological substance capable of binding to the primary biomolecule of the above (d) (hereinafter, also referred to as a "secondary biological substance") and a fluorescent dye are bound to each other

(v) The step of preparing a conjugate (hereinafter, also referred to as a "conjugate b") in which the target biological substance in the above (c) is bonded to the primary biological substance of the above (d)

(vi) The step of preparing a conjugate (hereinafter, also referred to as a "fluorescently labeled conjugate B2") in which the primary biological substance in the conjugate b is bonded to the secondary biological substance in the fluorescent dye-labeled biomolecule B

(vii) The step of irradiating the fluorescently labeled conjugate B2 with light having the range of the wavelength which is absorbed by the fluorescent dye-labeled biomolecule B, and detecting the fluorescence emitted by the fluorescent dye-labeled biomolecule B

Examples of the biological substance (the primary biological substance) capable of binding to the target biological substance and the biological substance (the secondary biological substance) capable of binding to the primary biological substance include the biological substances in the fluorescent dye-labeled biomolecule. The above biological substance can be appropriately selected in accordance with the target biological substance (a biological substance in the test object) or the primary biological substance, and a biological substance capable of specifically binding to the biological substance in the test object or to the primary biological substance can be selected.

Examples of the protein among the target biological substances include a protein, which is a so-called disease marker. The disease marker is not particularly limited; however, examples thereof include $\alpha$-fetoprotein (AFP), protein induced by vitamin K absence or antagonist II (PIVKA-II), breast carcinoma-associated antigen (BCA)225, basic fetoprotein (BFP), carbohydrate antigen (CA) 15-3, CA19-9, CA72-4, CA125, CA130, CA602, CA54/61 (CA546), carcinoembryonic antigen (CEA), DUPAN-2, elastase 1, immunosuppressive acidic protein (IAP), NCC-ST-439, $\gamma$-seminoprotein ($\gamma$-Sm), prostate specific antigen (PSA), prostatic acid phosphatase (PAP), nerve specific enolase (NSE), Iba1, amyloid $\beta$, tau, flotillin, squamous cell carcinoma associated antigen (SCC antigen), sialyl LeX-i antigen (SLX), SPan-1, tissue polypeptide antigen (TPA), sialyl Tn antigen (STN), cytokeratin (CYFRA), pepsinogen (PG), C-reactive protein (CRP), serum amyloid A protein (SAA), myoglobin, creatine kinase (CK), troponin T, and ventricular muscle myosin light chain I.

The target biological substance may be a bacterium. Examples of the bacterium include a bacterium to be subjected to a cellular and microbiological test, which is not particularly limited. Specific examples thereof include Escherichia coli, Salmonella, Legionella, and bacteria causing problems in public health.

The target biological substance may be a virus. Although the virus is not particularly limited, examples the antigens of the virus include hepatitis virus antigens such as hepatitis C and B virus antigens, p24 protein antigen of HIV virus, and pp65 protein antigen of cytomegalovirus (CMV), and E6 and E7 protein antigens of human papillomavirus (HPV).

In the above (i) or (iv), the sample containing the target biological substance is not particularly limited and can be prepared according to a conventional method.

In addition, the fluorescent dye-labeled biomolecule is not particularly limited and can be prepared by bonding a biological substance capable of binding to a target biological substance to the fluorescent dye according to a conventional method. The form of the bond and the reaction to form the bond are as described above in the fluorescent dye-labeled biomolecule.

In the above (v), the target biological substance may be directly bonded to the primary biological substance or may be bonded through another biological substance which is different from the target biological substance and the primary biological substance. In addition, in the above (vi), the primary biological substance in the conjugate b may be directly bonded to the secondary biological substance in the fluorescent dye-labeled biomolecule B or may be bonded through another biological substance which is different from the primary biological substance and the secondary biological substance.

The fluorescent dye-labeled biomolecule obtained from the fluorescent dye can be used as a fluorescent dye-labeled antibody in both the direct method and the indirect method, and is preferably used as a fluorescent dye-labeled antibody in the indirect method.

In the above (ii) or (v) and the above (vi), the binding of the fluorescent dye-labeled biomolecule and the like to the target biological substance is not particularly limited and can be carried out according to a conventional method.

In the above (iii) or (vii), the wavelength of light with which the fluorescent dye-labeled biomolecule is irradiated for excitation is not particularly limited as long as the wavelength is capable of exciting the fluorescent dye-labeled biomolecule. Generally, the wavelength for excitation is preferably 300 to 1,000 nm and more preferably 400 to 800 nm.

The light source used in the above (iii) or (vii) is not particularly limited as long as it emits a wavelength capable of exciting a fluorescent dye-labeled biomolecule, and for example, various laser light sources can be used. In addition, by using various optical filters, the emission wavelength from the light source can be adjusted to have a preferred wavelength range, or can be adjusted to detect only the fluorescence from the fluorescent dye-labeled biomolecule.

Other matters in the above (i) to (vii) are not particularly limited, and conditions of a method, a reagent, a device, and the like, which are generally used in the fluorescence detection using fluorescence labeling, can be appropriately

selected.

In addition, also regarding the steps other than the above (i) to (vii), conditions of a method, a reagent, a device, and the like, which are generally used, can be appropriately selected in accordance with various methods using fluorescence labeling.

For example, in the multicolor WB using the fluorescent dye-labeled biomolecule, it is possible to detect a target biological substance with excellent fluorescence intensity by preparing a blotted membrane according to a method generally used for a target biological substance (protein separation by electrophoresis, blotting to a membrane, and blocking of a membrane) and using a fluorescent dye-labeled biomolecule as a fluorescent dye-labeled antibody (preferably, as a secondary antibody in a fluorescent dye-labeled antibody obtained from a fluorescent dye (F)). In the dot blotting using the fluorescent dye-labeled biomolecule, as in the case of the multicolor WB, it is possible to detect a target biological substance with excellent fluorescence intensity by preparing a blotted nitrocellulose membrane, a blotted PVDF (polyvinylidene fluoride) membrane, or the like according to a method generally used for a target biological substance and using the fluorescent dye-labeled biomolecule as a fluorescent dye-labeled antibody (preferably, as a secondary antibody in a fluorescent dye-labeled antibody obtained from a fluorescent dye (F)).

- Substituent group T -

In the present invention, the preferred substituents include those selected from the following substituent group T.

In addition, in the present invention, in a case where the substituent is simply described as a substituent, the description of the corresponding substituent in the substituent group T can be referred to and applied. For example, in a case where only "alkyl group" is described, the description of "alkyl group" in the substituent group T can be referred to and applied. The same applies to other substituents other than the "alkyl group".

In addition, in the present invention, examples of a substituent which may be contained in a certain substituent such as an "alkyl group", include a substituent selected from the following substituent group T. In addition, in a case where a certain substituent such as an "alkyl group" has a substituent and further has a substituent, examples of the substituent which is contained in a certain substituent include a substituent obtained by combining two or more substituents selected from the following substituent group T.

Furthermore, in the present invention, in a case where an alkyl group is described separately from a cyclic (cyclo) alkyl group, the alkyl group is meant to include a linear alkyl group and a branched alkyl group. On the other hand, in a case where an alkyl group is not described separately from a cyclic alkyl group, and unless otherwise specified, the alkyl group is meant to include a linear alkyl group, a branched alkyl group, and a cycloalkyl group. The same applies to groups (alkoxy group, alkylthio group, alkenyloxy group, and the like) containing a group capable of having a cyclic structure (alkyl group, alkenyl group, alkynyl group, and the like) and compounds containing a group capable of having a cyclic structure. In a case where a group is capable of forming a cyclic skeleton, the lower limit of the number of atoms of the group forming the cyclic skeleton is 3 or more and preferably 5 or more, regardless of the lower limit of the number of atoms specifically described below for the group that can adopt this structure,

In the following description of the substituent group T, a group having a linear or branched structure and a group having a cyclic structure, such as an alkyl group and a cycloalkyl group, are sometimes described separately for clarity.

The groups included in the substituent group T include the following groups.

Examples thereof include an alkyl group (preferably having 1 to 30 carbon atoms, more preferably having 1 to 20 carbon atoms, still more preferably having 1 to 12 carbon atoms, even still more preferably having 1 to 8 carbon atoms, yet even still more preferably having 1 to 6 carbon atoms, and particularly preferably having 1 to 3 carbon atoms), an alkenyl group (preferably having 2 to 30 carbon atoms, more preferably having 2 to 20 carbon atoms, still more preferably having 2 to 12 carbon atoms, even still more preferably having 2 to 6 carbon atoms, and yet even still more preferably having 2 to 4 carbon atoms), an alkynyl group (preferably having 2 to 30 carbon atoms, more preferably having 2 to 20 carbon atoms, still more preferably having 2 to 12 carbon atoms, even still more preferably having 2 to 6 carbon atoms, and yet even still more preferably having 2 to 4 carbon atoms), a cycloalkyl group (preferably having 3 to 20 carbon atoms), a cycloalkenyl group (preferably having 5 to 20 carbon atoms), an aryl group (may be a monocyclic group or a fused ring group (preferably a fused group of 2 to 6 rings); in a case of a fused ring group, the fused ring group consists of a 5-membered to 7-membered ring; preferably having 6 to 40 carbon atoms, more preferably 6 to 30 carbon atoms, still more preferably 6 to 26 carbon atoms, and particularly preferably 6 to 10 carbon atoms), a heterocycle group (having at least one nitrogen atom, an oxygen atom, a sulfur atom, a phosphorus atom, a silicon atom, or selenium atom as a ring-constituting atom; may be a monocyclic ring or a fused ring (preferably a fused group in which 2 to 6 rings); in a case of a monocyclic group, the monocyclic ring is preferably a 5-membered to 7-membered ring and more preferably a 5-membered or 6-membered ring; preferably having 2 to 40 carbon atoms and more preferably having 2 to 20 carbon atoms; and the heterocyclic group includes an aromatic heterocyclic group (heteroaryl group) and an aliphatic heterocyclic group (aliphatic heterocyclic group)), an alkoxy group (preferably having 1 to 20 carbon atoms and more preferably having 1 to 12 carbon atoms), an alkenyloxy group (preferably having 2 to 20 carbon atoms and more preferably having 2 to 12 carbon atoms), an alkynyloxy

group (preferably having 2 to 20 carbon atoms and more preferably having 2 to 12 carbon atoms), a cycloalkyloxy group (preferably having 3 to 20 carbon atoms), an aryloxy group (preferably having 6 to 40 carbon atoms, more preferably having 6 to 26 carbon atoms, and still more preferably having 6 to 14 carbon atoms), a heterocyclic oxy group (preferably having 2 to 20 carbon atoms), a polyalkyleneoxy group,

an alkoxycarbonyl group (preferably having 2 to 20 carbon atoms), a cycloalkoxycarbonyl group (preferably having 4 to 20 carbon atoms), an aryloxycarbonyl group (preferably having 6 to 20 carbon atoms), an amino group (preferably having 0 to 20 carbon atoms; including an unsubstituted amino group ($-NH_2$), a (mono- or di-) alkylamino group, a (mono- or di-) alkenylamino group, a (mono- or di-) alkynylamino group, a (mono- or di-) cycloalkylamino group, a (mono- or di-) cycloalkenylamino group, a (mono- or di-) arylamino group, and a (mono- or di-) heterocyclic amino group; and the above-described groups that substitute an unsubstituted amino group have the same meanings as the corresponding groups in the substituent group T), a sulfamoyl group (preferably having 0 to 20 carbon atoms; and preferably an alkyl, cycloalkyl, or aryl sulfamoyl group), an acyl group (preferably having 1 to 20 carbon atoms and more preferably having 2 to 15 carbon atoms; and including -C(=O)H, an alkylcarbonyl group, a cycloalkylcarbonyl group, an arylcarbonyl group, and a heterocyclic carbonyl group), an acyloxy group (preferably having 1 to 20 carbon atoms), a carbamoyl group (preferably having 1 to 20 carbon atoms; and preferably an alkyl, cycloalkyl, or aryl carbamoyl group),

an acylamino group (preferably having 1 to 20 carbon atoms), a sulfonamide group (preferably having 0 to 20 carbon atoms; and preferably an alkyl, cycloalkyl, or aryl sulfonamide group), an alkylthio group (preferably having 1 to 20 carbon atoms and more preferably having 1 to 12 carbon atoms), a cycloalkylthio group (preferably having 3 to 20 carbon atoms), an arylthio group (preferably having 6 to 40 carbon atoms, more preferably having 6 to 26 carbon atoms, and still more preferably having 6 to 14 carbon atoms), a heterocyclic thio group (preferably having 2 to 20 carbon atoms), an alkyl, cycloalkyl, or aryl sulfonyl group (preferably having 1 to 20 carbon atoms),

a silyl group (preferably having 1 to 30 carbon atoms and more preferably 1 to 20 carbon atoms; preferably a silyl group at which an alkyl, aryl, alkoxy, or aryloxy has been substituted), a silyloxy group (preferably having 1 to 20 carbon atoms: preferably a silyloxy group at which an alkyl, aryl, alkoxy, or aryloxy has been substituted), a hydroxy group, a cyano group, a nitro group, a halogen atom (for example, a fluorine atom, a chlorine atom, a bromine atom or an iodine atom), an oxygen atom (specifically replacing $>CH_2$ which constitutes a ring with >C=O), a carboxy group ($-CO_2H$), a phosphono group [$-PO(OH)_2$], a phosphonooxy group [$-O-PO(OH)_2$], a sulfo group ($-SO_3H$), a boric acid group [$-B(OH)_2$], onio group (referred to as a cationic group; including an ammonio group containing a cyclic ammonio, a sulfonio group, and a phosphonio group; and preferably having 0 to 30 carbon atoms and more preferably having 1 to 20 carbon atoms), a sulfanyl group (-SH), a guanidino group ($-NHC(=NH)NH_2$), an amino acid residue, or a polyamino acid residue.

(Anionic group)

In the present invention, the anionic group may be any group having an anion. Examples of such an anionic group include a carboxy group, a phosphono group (a phosphonate group, $-PO(OH)_2$), a phosphonooxy group (a phosphate group, $-OPO(OH)_2$), and a sulfo group, where a phosphono group, a phosphonooxy group, or a sulfo group is preferable, and a phosphonooxy group or a sulfo group is more preferable.

The anionic group may have an ionic structure in which a hydrogen ion is dissociated, or may have a salt structure. To the monovalent or polyvalent cation in a case where the anionic group has a salt structure, the description of the monovalent or polyvalent cation in the description of the salt structure described above can be preferably applied.

(Cationic group)

In the present invention, the cationic group may be any group having a cation. Examples of such a cationic group include a group having a quaternary ammonium ion (a quaternary ammonio group) and a group having a quaternary phosphonium ion (a quaternary phosphonio group), where a group having a quaternary ammonium ion is preferable. It is noted that preferred examples of the substituent possessed by $N^+$ in the group having a quaternary ammonium ion and the substituent possessed by $P^+$ in the group having a quaternary phosphonium ion include an alkyl group and an aryl group, where groups in which all the substituents possessed by $N^+$ and $P^+$ are alkyl groups are preferable.

The cationic group may have a salt structure in addition to the ionic structure. Examples of the monovalent or polyvalent anion in a case where the cationic group has a salt structure include halide ions such as $F^-$ and $Cl^-$, and monovalent or polyvalent organic anions such as $BF_4^-$, $PF_6^-$, and a bis(trifluoromethylsulfonyl)imide ion.

(Polyalkyleneoxy group)

In the present invention, the polyalkyleneoxy group may be any group represented by $-(L-O)_gR^E$.

The L represents an alkylene group obtained by removing one hydrogen atom from an alkyl group in the substituent group T described above, where it preferably has 2 to 4 carbon atoms, more preferably has 2 or 3 carbon

atoms, and still more preferably has 2 carbon atoms. The number of carbon atoms contained in the shortest chain that links two carbon atoms which are bonding sites of the group is preferably 0 to 2, more preferably 0 or 1, and still more preferably 0. That is, L is most preferably an ethylene group.

The g means an average repetition number (simply, also referred to as a repetition number), which is preferably 1 to 24, more preferably 1 to 12, and still more preferably 4 to 12. Even in a case where the repetition number is small, for example, even in a case where g is 1, a proper hydrophilicity and a proper excluded volume effect can be exhibited.

The $R^E$ represents a hydrogen atom or an alkyl group. For the alkyl group which can be adopted as $R^E$, the description of the alkyl group in the above-described substituent group T can be preferably applied, and among the above, an alkyl group having 1 to 3 carbon atoms is preferable. The alkyl group which can be adopted as $R^E$ may have a substituent.

In addition, examples of the group obtained by combining a plurality of substituents selected from the substituent group T include the above-described alkyl group, alkenyl group, alkynyl group, cycloalkyl group, cycloalkenyl group, aryl group, heterocyclic group, alkoxy group, alkenyloxy group, alkynyloxy group, cycloalkyloxy group, aryloxy group, heterocyclic oxy group, alkoxycarbonyl group, cycloalkoxycarbonyl group, aryloxycarbonyl group, amino group, sulfamoyl group, acyl group, acyloxy group, carbamoyl group, acylamino group, sulfonamide group, alkylthio group, cycloalkylthio group, arylthio group, heterocyclic thio group, and an alkyl, cycloalkyl, or aryl sulfonyl group, which have, as a substituent, an anionic group (a carboxy group, a phosphono group, or a sulfo group), a cationic group (an onio group), an amino acid residue, a polyamino acid residue, or a $-(CH_2-CH_2-O)_b$-alkyl group (b has the same meaning as b in $R^1$ to $R^4$ in General Formula ($\alpha$) described above).

The substituent selected from the substituent group T is more preferably an alkyl group, an alkenyl group, a cycloalkyl group, an aryl group, a heterocyclic group, an alkoxy group, a cycloalkoxy group, an aryloxy group, an acyl group, an alkoxycarbonyl group, a cycloalkoxycarbonyl group, an amino group, an acylamino group, a carbamoyl group, a cyano group, a halogen atom, an anionic group, or a cationic group, and particularly preferably an alkyl group, an alkenyl group, an aryl group, a heterocyclic group, an alkoxy group, an acyl group, an alkoxycarbonyl group, an amino group, an acylamino group, a carbamoyl group, a cyano group, an anionic group, or a cationic group.

In addition to the group obtained by combining a plurality of substituents selected from the substituent group T described above, the substituent selected from the substituent group T also includes a group obtained by combining a plurality of the above groups, unless otherwise specified. For example, in a case where a compound, a substituent, or the like contains an alkyl group, an alkenyl group, or the like, the alkyl group, the alkenyl group, or the like may be substituted or unsubstituted. In addition, in a case where a compound, a substituent, or the like contains an aryl group, a heterocyclic group, or the like, the aryl group, the heterocyclic group, or the like may be a monocyclic ring or a fused ring moiety, and may be substituted or unsubstituted.

## Examples

Hereinafter, the present invention will be described in more detail based on Examples, but the present invention is not limited thereto. It is noted that room temperature means 25°C.

The compounds (1) to (4) corresponding to the fluorescent dye (F) are shown below.

It is noted that in each compound, the sulfo group or the phosphonooxy group may include a salt structure (for example, a potassium salt, a sodium salt, a triethylammonium (TEA) salt, or an N,N-diisopropylethylammonium (DIPEA) salt), even unless otherwise specified.

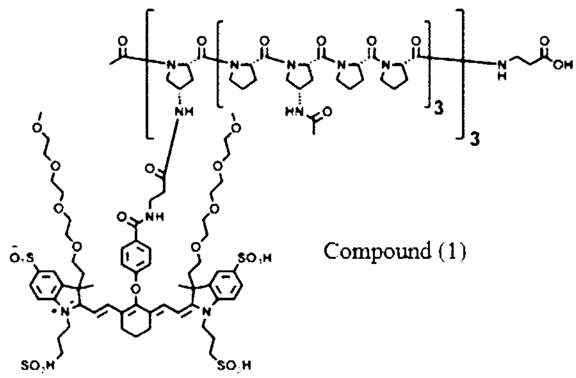

Compound (1)

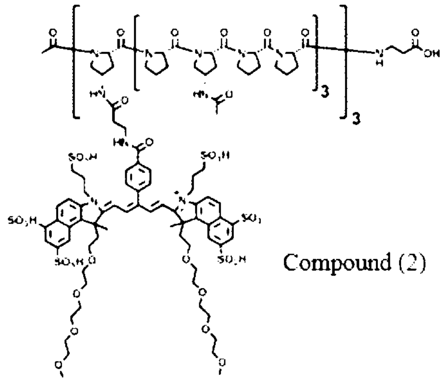

Compound (2)

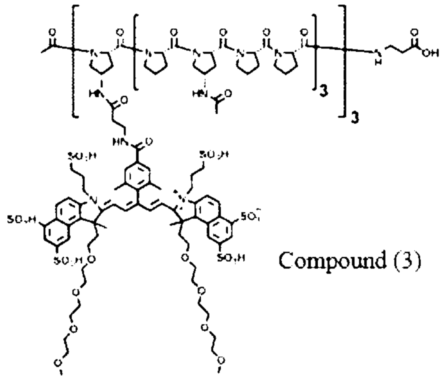

Compound (3)

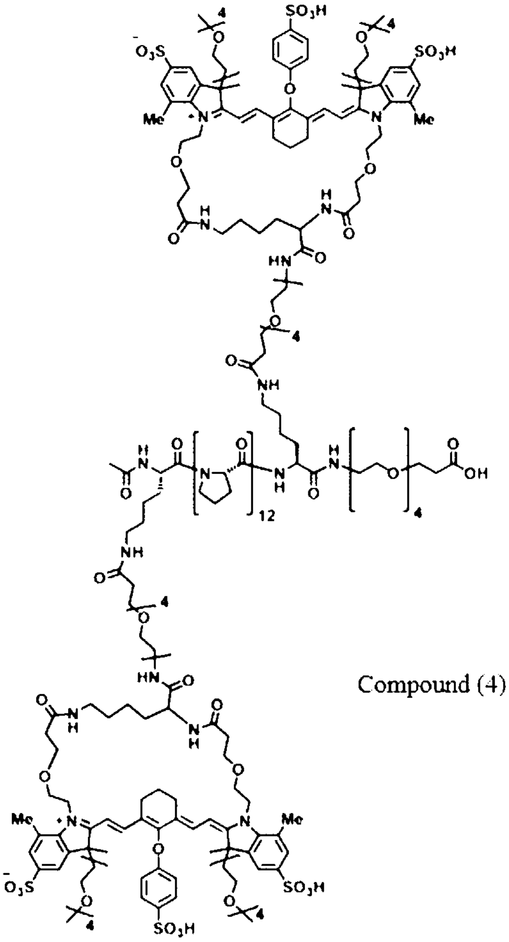

Compound (4)

The method of synthesizing each compound and a labeled antibody will be described in detail below; however, the starting materials, the dye intermediates, and the synthetic routes are not limited thereto.

It is noted that the abbreviations used in the synthesis of the respective compounds described below are as follows.

DBU: 1,8-diazabicyclo[5.4.0]-7-undecene
PyAOP: (7-azabenzotriazole-1-yloxy)tripyrrolidinophosphonium hexafluorophosphate
DIC: diisopropylcarbodiimide
EDCI: 1-ethyl-3-(3-dimethylaminopropyl)carbodiimide hydrochloride
HOBt: 1-hydroxybenzotriazole
Pd(dppf)$Cl_2$: [1,1'-bis(diphenylphosphino)ferrocene]palladium (II) dichloride
Pd(Amphos)$Cl_2$: bis(di-tert-butyl(4-dimethylaminophenyl)phosphine)dichloropalladium (II)
DMT-MM: 4-(4,6-dimethoxy-1,3,5-triazin-2-yl)-4-methylmorpholinium chloride

NMP: N-methyl-2-pyrrolidone
DMF: dimethylformamide
DMAP: 4-dimethylaminopyridine
DIPEA: N-diisopropylethylamine
TFA: Trifluoroacetic acid
THF: tetrahydrofuran
TFE: 2,2,2-trifluoroethanol
Me: methyl group
Ms: mesyl group
Et: ethyl group
Ac: acetyl group
Ts: p-toluenesulfonyl group
Trt: trityl group (triphenylmethyl group)
Fmoc: 9-fluorenylmethyloxycarbonyl group
Boc: tert-butoxycarbonyl group
Ala: alanine
Lys: lysine
Pro: proline
Resin: resin
$mPEG_p$: indicating the following structures, where p in $PEG_p$ is the average repetition number. * represents a bonding site.

$EO_p$: indicating the following structure, where p in $EO_p$ is the average repetition number. Provided that $EO_m$ is bonded to a nitrogen atom or an oxygen atom on the carbon atom side. * represents a bonding site.

$$EO_p = *\!-\!\left(CH_2-CH_2-O\right)_p\!-\!* \qquad mPEG_p = *\!-\!\left(CH_2-CH_2-O\right)_p\!-CH_3$$

In addition, %v/v means a percentage in terms of volume.

The MS spectrum was measured by ACQUITY SQD LC/MS System [product name, manufactured by Waters Corporation, ionization method: electrospray Ionization (ESI)] or LCMS-2010EV [product name, manufactured by Shimadzu Corporation, ionization method: an ionization method simultaneously carrying out ESI and atmospheric pressure chemical ionization (APCI)].

It is noted that in the synthesis of each compound, the synthesis of the peptide chain was carried out according to the general method of the peptide solid phase method described in WO2018/174078A.

[General method of solid phase peptide synthesis method using automatic peptide synthesizer]

Solid phase peptide synthesis was carried out using an automatic peptide synthesizer (product name: Syrol, manufactured by biotage, LLC). The synthesizer was set with Rink Amide-ChemMatrix (registered trade name, manufactured by Biotage, LLC), an N-methyl-2-pyrrolidone (NMP) solution of Fmoc amino acid (0.5 mol/L), an NMP solution of cyano-hydroxyimino-acetic acid ethyl ester (1.0 mol/L) and diisopropylethylamine (0.1 mol/L), an NMP solution of diisopropylcarbodiimide (1.0 mol/L), an NMP solution of piperidine (20% v/v), and an NMP solution of acetic anhydride (20 %v/v), and synthesis was carried out according to the manual. A cycle consisting of Fmoc deprotection (20 minutes), washing with NMP, condensation of Fmoc amino acids (1 hour), and washing with NMP as one cycle was repeated to elongate the peptide chain.

[Synthesis of compound (M2-1)]

A compound (M2-1) was synthesized based on the following scheme. The results of the MS measurement of the compound (M2-13) were as follows.
MS (ESI m/z): $(M + H^+)^+ = 1,384$, $(M - H^+)^- = 1,382$

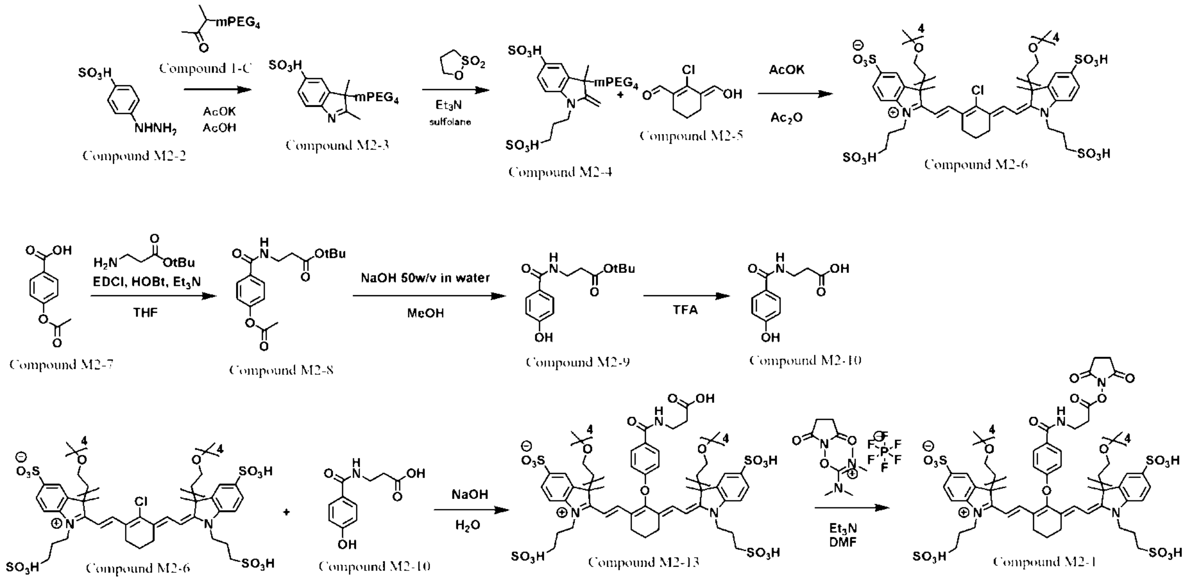


<Synthesis of compounds (1) and (1-NHS)>

1) Synthesis of compound (1-8)

A compound (1-8) was synthesized according to the following scheme.

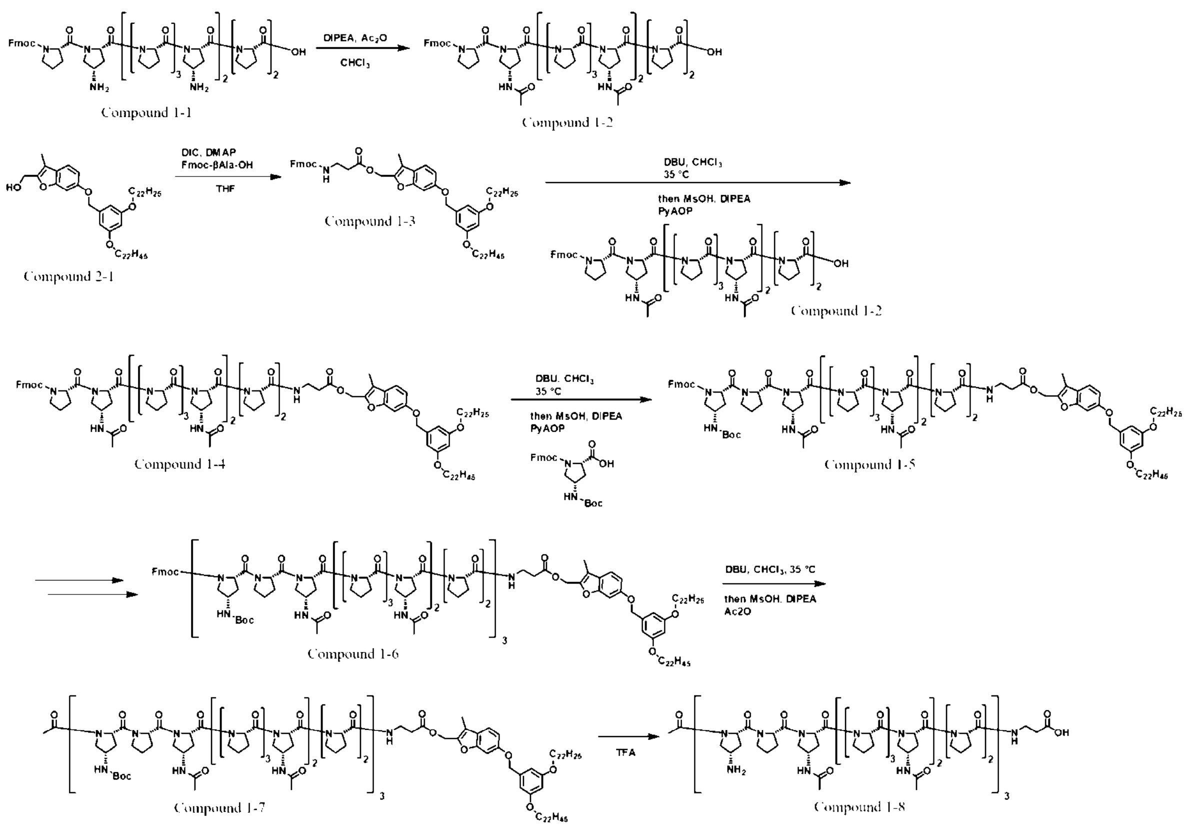


(i) Synthesis of compound (1-1)

Solid phase peptide synthesis was carried out using H-Pro-Trt(2-Cl)-Resin (manufactured by Watanabe Chemical Industries, Ltd., 0.93 mmol/g, 53.8 mg) as a starting raw material. N-(9-fluorenylmethoxycarbonyl)-L-proline (Fmoc-Pro-OH) and (2S,4S)-(tert-butoxycarbonyl)-4-amino-1-(9H-fluoren-9-ylmethoxy)carbonyl)-pyrrolidine-2-carboxylic acid (Fmoc-L-Pro(4-NHBoc)-OH(2S,4S)) were subjected to elongation, and the elongation that was carried out by using N-(9-fluorenylmethoxycarbonyl)-L-proline (Fmoc-Pro-OH) was repeated for 3 cycles. (2S,4S)-(tert-butoxycarbonyl)-4-amino-1-(9H-fluoren-9-ylmethoxy)carbonyl)-pyrrolidine-2-carboxylic acid (Fmoc-L-Pro(4-NHBoc)-OH (2S,4S)) was subjected to elongation, and the elongation using N-(9-fluorenylmethoxycarbonyl)-L-proline (Fmoc-Pro-OH) was repeated for 3 cycles. (2S,4S)-(tert-butoxycarbonyl)-4-amino-1-(9H-fluoren-9-ylmethoxy)carbonyl)-pyrrolidine-2-carboxylic acid (Fmoc-L-Pro(4-NHBoc)-OH(2S,4S)) and N-(9-fluorenylmethoxycarbonyl)-L-proline (Fmoc-Pro-OH) were subjected to elongation. After completion of the elongation, the resin was washed with dichloromethane, and then the solvent was distilled off under reduced pressure. 2.0 mL of a mixed solution of TFA:triisopropylsilane:water = 95:2.5:2.5 was added thereto, and the peptide was cut out and deprotected. After 2 hours, the resin was filtered out, and methyl-t-butyl ether (12 mL) was added to the filtrate to generate a solid. The solid was precipitated by centrifugation, and then the supernatant was removed. The solid was washed with methyl-t-butyl ether, and then the solvent was distilled off under reduced pressure to obtain 51.2 mg of a white solid compound (1-1).

(ii) Synthesis of compound (1-2)

352 mg of the compound (1-1), 3.5 mL of chloroform ($CHCl_3$), 423 μL of N-diisopropylethylamine (DIPEA), and 115 μL of acetic anhydride ($Ac_2O$) were placed in an eggplant flask having a capacity of 50 mL, and stirring was carried out at room temperature for 1 hour. Thereafter, the reaction solution was concentrated and purified by preparative HPLC, and freeze drying was followed to obtain 314 mg of a compound (1-2).

(iii) Synthesis of compound (1-3)

500 mg of the compound (4-1) described in WO2020/175473A as the compound (2-1), 5 mL of tetrahydrofuran (THF), 339.3 mg of N-[(9H-fluoren-9-ylmethoxy)carbonyl]-β-alanine (Fmoc-βAla-OH), 171 mL of diisopropylcarbodiimide, and 13.3 mg of 4-dimethylaminopyridine were placed in an eggplant flask having a capacity of 50 mL, and stirring was carried out at room temperature for 2 hours. Acetonitrile (50 mL) was added thereto to filter the precipitated solid, which was subsequently subjected to drying under reduced pressure to obtain 593 mg of a compound (1-3).

(iv) Synthesis of compound (1-4)

108 mg of the compound (1-3), 2.2 mL of chloroform ($CHCl_3$), and 26.8 μL of 1,8-diazabicyclo[5.4.0]-7-undecene (DBU) were placed in an eggplant flask having a capacity of 50 mL, and stirring was carried out at 35°C for 1 hour. 11.7 μL of methanesulfonic acid (MsOH), 93.3 μL of N,N-diisopropylethylamine (DIPEA), 169 mg of the compound (1-2), and 141 mg of (7-azabenzotriazole-1-yloxy)tripyrrolidinophosphonium hexafluorophosphate (PyAOP) were placed therein, and stirring was carried out at 35°C for 3 hours. Acetonitrile (10 mL) was added thereto to filter the precipitated solid, which was subsequently subjected to drying under reduced pressure to obtain 219 mg of a compound (1-4).

(v) Synthesis of compound (1-5)

233 mg of the above-described compound (1-5) was synthesized from 219 mg of the compound (1-4) in the same manner, except that in the synthesis of the compound (1-4) described above, (2S,4S)-(tert-butoxycarbonyl)-4-amino-1-(9H-fluoren-9-ylmethoxy)carbonyl)-pyrrolidine-2-carboxylic acid (Fmoc-L-Pro(4-NHBoc)-OH(2S,4S)) was used instead of the compound (1-2) and the compound (1-4) was used instead of the compound (1-3).

(vi) Synthesis of compound (1-6)

The same operation as the above-described synthesis of the compounds (1-4) and (1-5) was repeated twice to synthesize 190 mg of a compound (1-6) from 100 mg of the compound (1-5).

(vii) Synthesis of compound (1-7)

40.2 mg of the compound (1-6), 5 mL of chloroform ($CHCl_3$), and 3.1 μL of 1,8-diazabicyclo[5.4.0]-7-undecene (DBU) were placed in an eggplant flask having a capacity of 100 mL, and stirring was carried out at 35°C for 1 hour. 1.35 μL of methanesulfonic acid (MsOH), 7.2 μL of N,N-diisopropylethylamine (DIPEA), and 1.9 μL of acetic anhydride ($Ac_2O$) were placed therein, and stirring was carried out at 35°C for 1 hour. A solid precipitated by adding acetonitrile (50 mL) was subjected to filtration and drying under reduced pressure to obtain 33.7 mg of a compound (1-7).

(viii) Synthesis of compound (1-8)

30.1 mg of the compound (1-7) and 1.0 mL of a mixed solution of TFA:triisopropylsilane:water = 95:2.5:2.5 were added to an eggplant flask having a capacity of 10 mL, and stirring was carried out at room temperature for 1 hour. 10 mL of methanol (MeOH) was added to the reaction solution to generate a solid to be removed, and then the solid was removed by filtration. The filtrate was concentrated and purified by preparative HPLC, and freeze drying was followed to obtain 10.6 mg of a compound (1-8).

2) Synthesis of compound (1) and (1-NHS)

Compounds (1) and (1-NHS) were synthesized based on the following scheme.

Although the compound (1-8), and the compounds (1) and (1-NHS) have different notation formats for the repeating structure of the polypeptide chain, the compounds have the same structure as the repeating structure of the polypeptide chain.

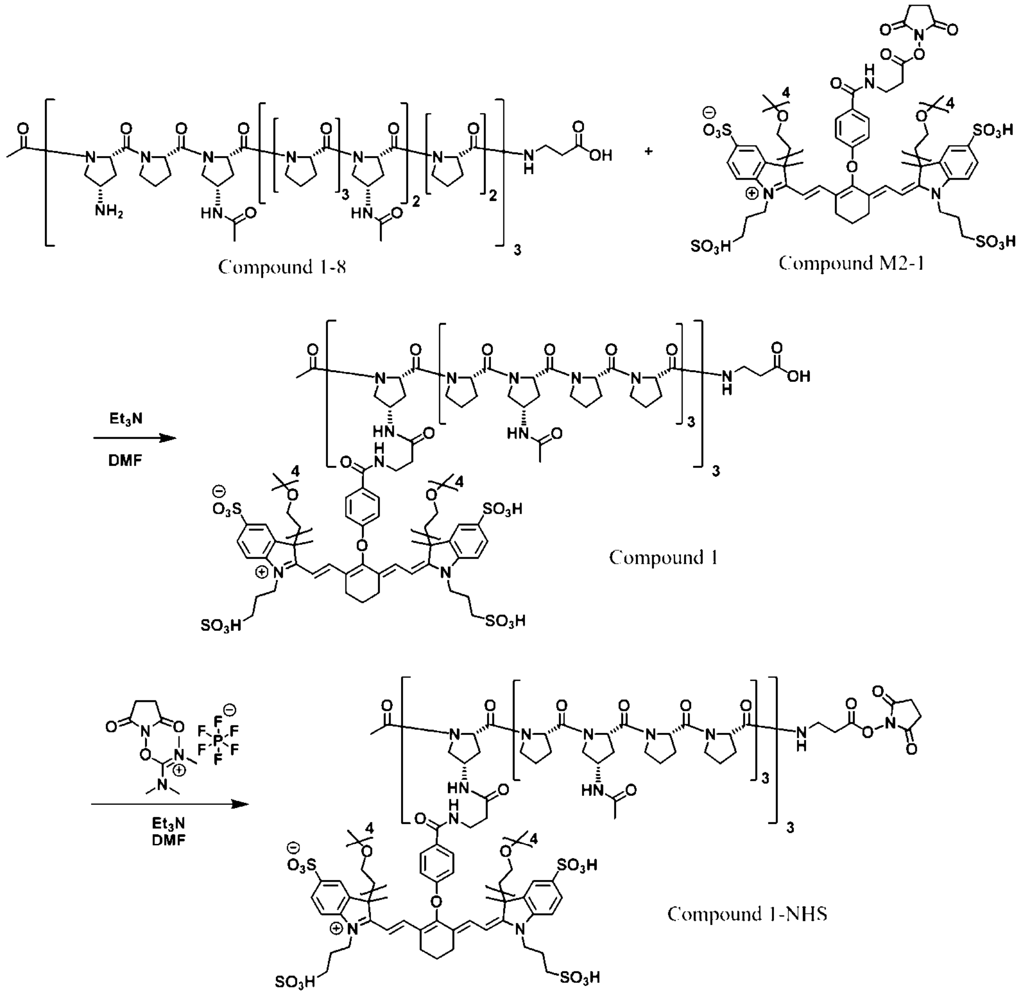


Compound 1-8

Compound M2-1

Compound 1

Compound 1-NHS

(i) Synthesis of compound (1)

2.33 mg of the compound (1-8), 110 μL of N,N-dimethylformamide (DMF), 7.3 μL of triethylamine ($Et_3N$), and 3.1 mg of the compound (M2-1) were placed in an eggplant flask having a capacity of 10 mL, and stirring was carried out at room temperature for 1 hour. Thereafter, the reaction solution was concentrated and purified by preparative HPLC, and freeze drying was followed to obtain 0.86 mg of a compound (1). The results of the MS measurement of the compound (1) were as follows.

MS (ESI m/z): $(M + H^+)^+$ = 8,571, $(M - H^+)^-$ = 8,569

(ii) Synthesis of compound (1-NHS)

126 μL of N,N-dimethylformamide (DMF), 2.64 mg of N,N,N',N'-tetramethyl-O-(N-succinimidyl)uronium hexafluorophosphate, and 2.1 μL of triethylamine ($Et_3N$) were added to 1.26 mg of the compound (1), and stirring was carried out for 1 hour. Then, the solvent was distilled off under reduced pressure, ethyl acetate was added, the supernatant was removed, and vacuum drying was carried out to obtain a compound (1-NHS).

<Synthesis of compound (2)>

1) Synthesis of compound (M3-1)

A compound (M3-1) was synthesized according to the following scheme.

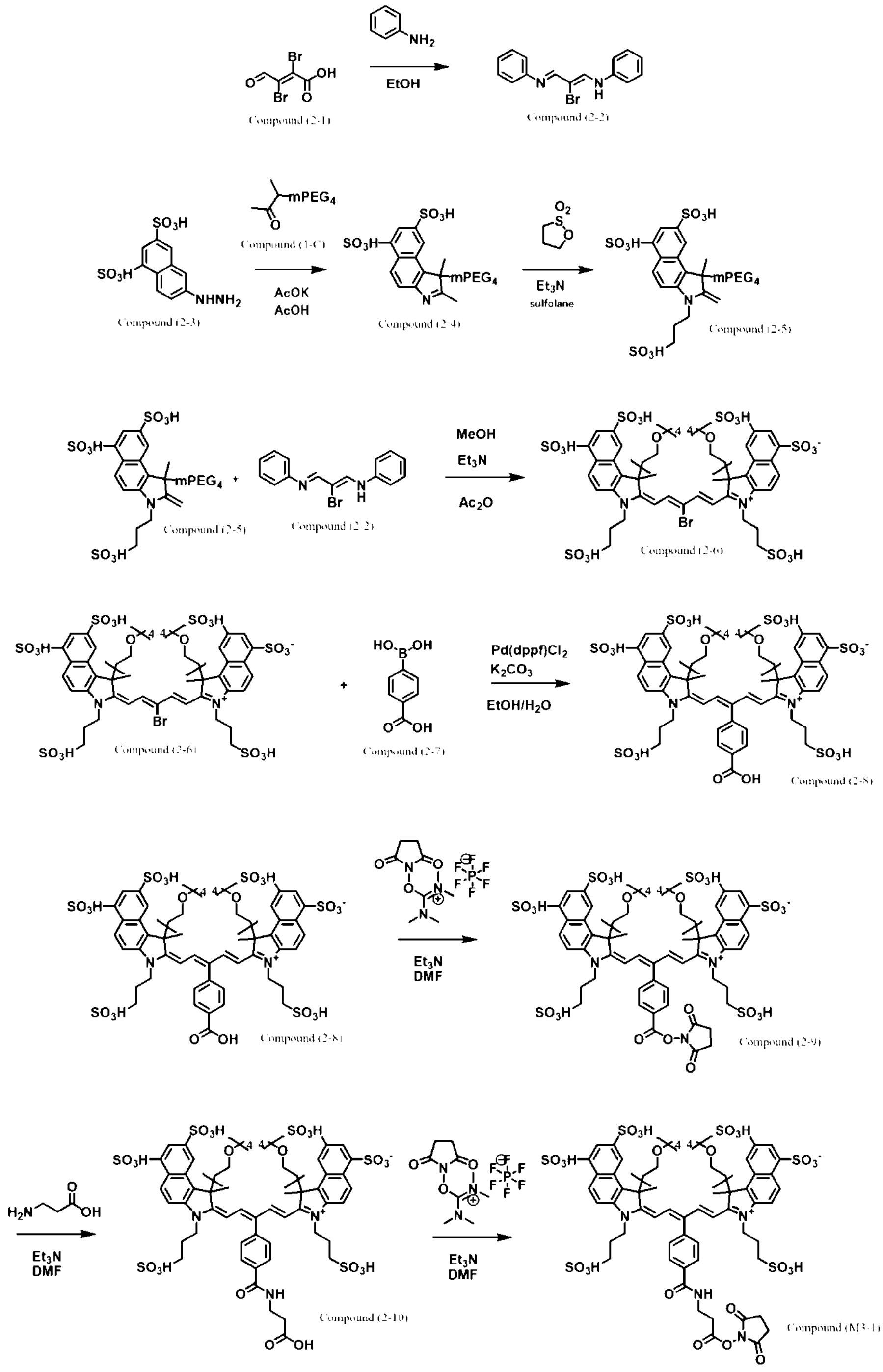

Compound (2-1)
Compound (2-2)
Compound (1-C)
Compound (2-3)
Compound (2-4)
Compound (2-5)
Compound (2-5)
Compound (2-2)
Compound (2-6)
Compound (2-6)
Compound (2-7)
Compound (2-8)
Compound (2-8)
Compound (2-9)
Compound (2-10)
Compound (M3-1)

(i) Synthesis of compound (2-2)

2.9 g of the compound (2-1) and 15 mL of ethanol were placed in an eggplant flask having a capacity of 100 mL, 15 mL of ethanol in which 2.1 mL of aniline was dissolved was added dropwise thereto at 0°C, and the mixture was stirred at 60°C for 4 hours under a nitrogen atmosphere. The solvent was distilled off under reduced pressure to adjust the solvent amount to 15 mL, and air cooling was carried out to 0°C. The precipitated yellow crystals were filtered to obtain 2.6 g of a compound (2-2).

(ii) Synthesis of compound (2-4)

100 mg of the compound (2-3), 0.3 mL of acetic acid (AcOH), 124 mg of the compound (1-C), and 31 mg of potassium acetate (AcOK) were placed in an eggplant flask having a capacity of 100 mL, and stirring was carried out at 130°C for 1 hour in a nitrogen atmosphere. The solvent was distilled off under reduced pressure, the purification was carried out by reverse phase column chromatography (eluent: acetonitrile/water = from 0/100 to 20/80) to obtain 58 mg of a compound (2-4).

(iii) Synthesis of compound (2-5)

470 mg of the compound (2-4), 0.15 mL of 1,3-propane sultone, 2 mL of sulfolane, and 254 mg of potassium acetate (AcOK) were placed in an eggplant flask having a capacity of 50 mL, and stirring was at 110°C for 1 hour. 20 mL of ethyl acetate was added to the reaction solution to cause precipitation. The precipitate was purified by reverse phase column chromatography (eluent: acetonitrile/water = from 0/100 to 30/70) to obtain 475 mg of a compound (2-5).

(iv) Synthesis of compound (2-6)

171 mg of the compound (2-5), 48 mg of the compound (2-2), 71 μL of triethylamine ($Et_3N$), 5 μL of methanol (MeOH), and 0.48 mL of acetic anhydride ($Ac_2O$) were added in a test tube, and stirring was carried out at 50°C for 2 hours in a nitrogen atmosphere. After the reaction was settled, distilled water was added, and purification was carried out by reverse phase column chromatography (eluent: acetonitrile/water = from 0/100 to 25/75) to obtain 150 mg of a compound (2-6).

(v) Synthesis of compound (2-8)

10 mg of the compound (2-6), 10 mg of potassium carbonate ($K_2CO_3$), 300 μL of ethanol (EtOH), and 100 μL of distilled water were added in a test tube, and stirring was carried out at 50°C. To this solution, a solution obtained by mixing 6 mg of the compound (2-7) and [1,1'-bis(diphenylphosphino)ferrocene]palladium (II) dichloride ($Pd(dppf)Cl_2$) in 200 μL of distilled water was added dropwise, and then stirring was carried out at 50°C for 30 minutes. The reaction solution was cooled to room temperature and purified by preparative HPLC, and freeze drying was followed to obtain 3.6 mg of a compound (2-8). The results of the MS measurement of the compound (2-8) were as follows.
MS (ESI m/z):$(M + H^+)^+$ = 1,491, $(M - H^+)^-$ = 1,489

(vi) Synthesis of compound (2-9)

3.5 mg of a compound (2-9) was synthesized from 3.6 mg of the compound (2-8) in the same manner as in the synthesis of the compound (M2-1). The results of the MS measurement of the compound (2-9) were as follows.
MS (ESI m/z):$(M + H^+)^+$ = 1,588, $(M - H^+)^-$ = 1,586

(vii) Synthesis of compound (2-10)

7.53 mg of the compound (2-9), 261 μL of N,N-dimethylformamide (DMF), 3.6 μL of triethylamine ($Et_3N$), and 11.3 mg of β-alanine were placed in a test tube, and the mixture was stirred at room temperature for 3 hours. Thereafter, the reaction solution was concentrated and purified by preparative HPLC, and freeze drying was followed to obtain 4.49 mg of a compound (2-10). The results of the MS measurement of the compound (2-10) were as follows.
MS (ESI m/z):$(M + H^+)^+$ = 1,562, $(M - H^+)^-$ = 1,560

(viii) Synthesis of compound (M3-1)

4.21 mg of a compound (M3-1) was synthesized from 4.49 mg of the compound (2-10) in the same manner as in

the synthesis of the compound (M2-1). The results of the MS measurement of the compound (M3-1) were as follows.
MS (ESI m/z):$(M + H^+)^+$ = 1,659, $(M - H^+)^-$ = 1,657

2) Synthesis of compound (2)

1.64 mg of a compound (2) was synthesized from 3.0 mg of the compound (1-8) in the same manner as the compound (1) according to the following scheme. The results of the MS measurement of the compound (2) were as follows.
MS (ESI m/z):$(M + H^+)^+$ = 9,112, $(M - H^+)^-$ = 9,110

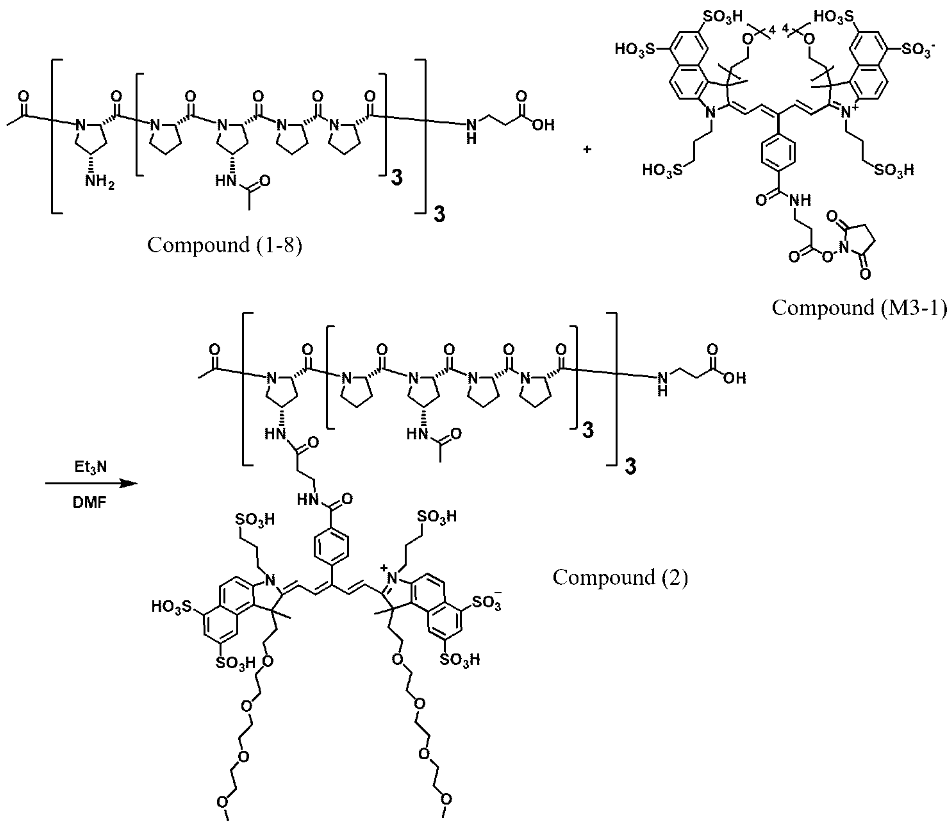


Compound (1-8)

Compound (M3-1)

Compound (2)

<Synthesis of compound (3)>

1) Synthesis of compound (M4-1)

A compound (M4-1) was synthesized in the same manner as the synthesis of the compound (M3-1) according to the following scheme. The results of the MS measurement of the compound (M4-1) were as follows.
MS (ESI m/z):$(M + H^+)^+$ = 1,688, $(M - H^+)^-$ = 1,686

A compound (3-2) was synthesized as follows. 300 mg of the compound (3-1), 185 mg of tetrahydroxydiboron, 1.9 mg of bis(di-tert-butyl(4-dimethylaminophenyl)phosphine)dichloropalladium (II), 457 μL of N,N-diisopropylethylamine (DIPEA), 3 mL of tetrahydrofuran (THF), and 1.5 mL of methanol (MeOH) were placed in a three-necked eggplant flask having a capacity of 50 mL, and stirring was carried out at 55°C for 3 hours in a nitrogen atmosphere. The solvent was distilled off under reduced pressure, and purification was carried out by reverse phase column chromatography to obtain 254 mg of a compound (3-2).

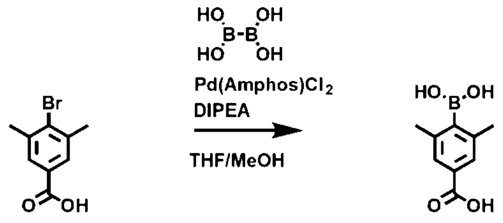


Compound (3-1) Compound (3-2)

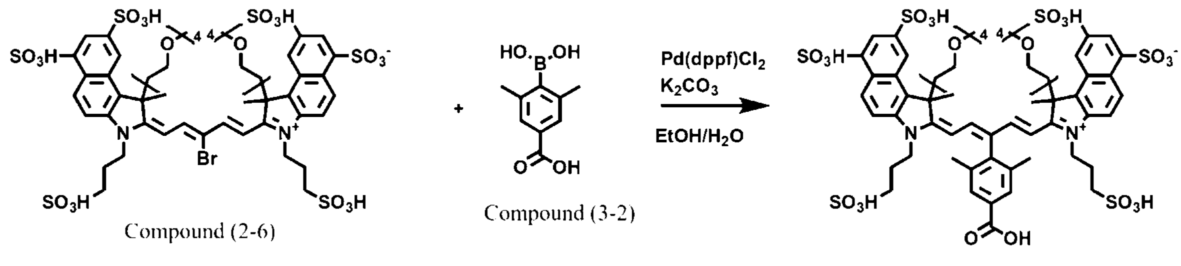


Compound (2-6) Compound (3-2) Compound (3-3)

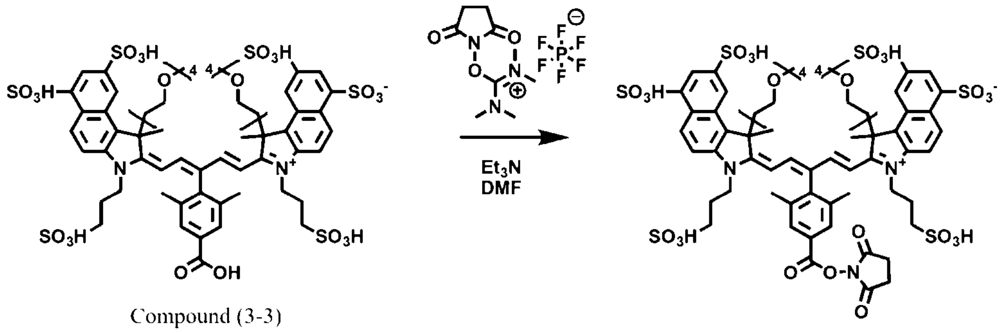


Compound (3-3) Compound (3-5)

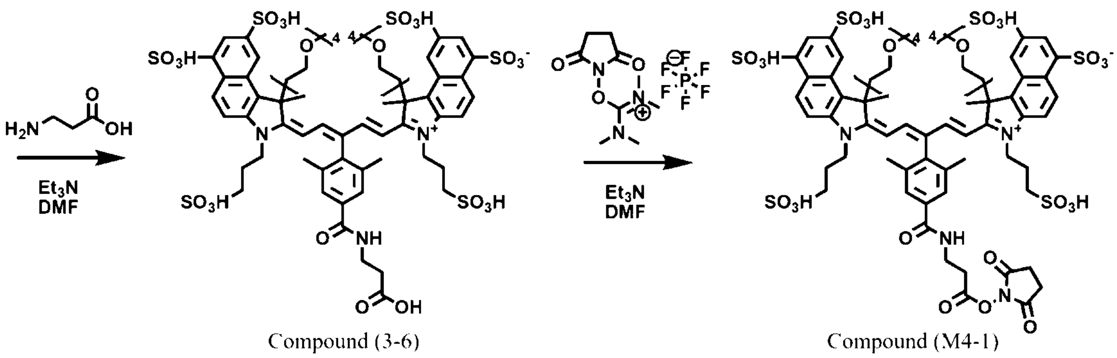


Compound (3-6) Compound (M4-1)

2) Synthesis of compound (3)

1.57 mg of a compound (3) was synthesized from 3.0 mg of the compound (1-8) in the same manner as the synthesis of the compound (1) according to the following scheme. The results of the MS measurement of the compound (3) were as follows.

MS (ESI m/z):$(M + H^+)^+$ = 9,194, $(M - H^+)^-$ = 9,196

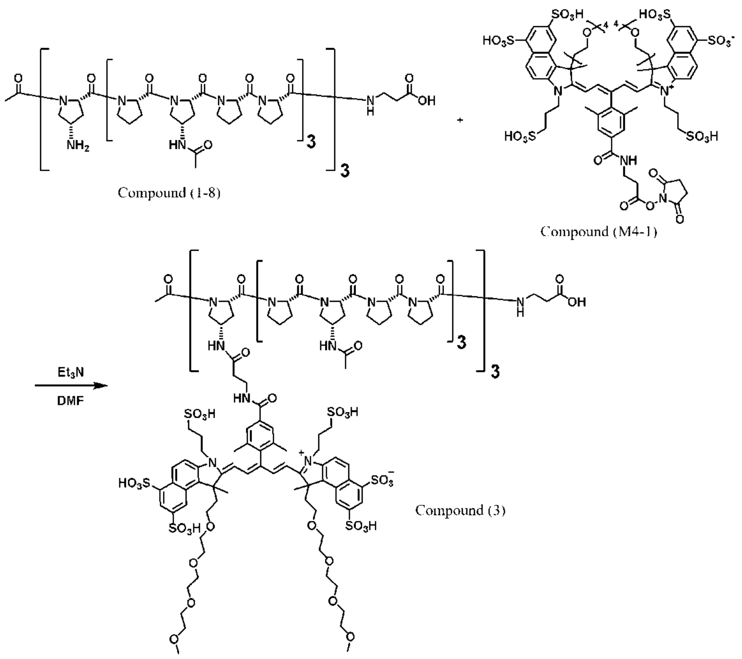


Compound (1-8)

Compound (M4-1)

Compound (3)

<Synthesis of compound (4)>

1) Synthesis of compound (4-7)

A compound (4-7) was synthesized according to the following scheme.

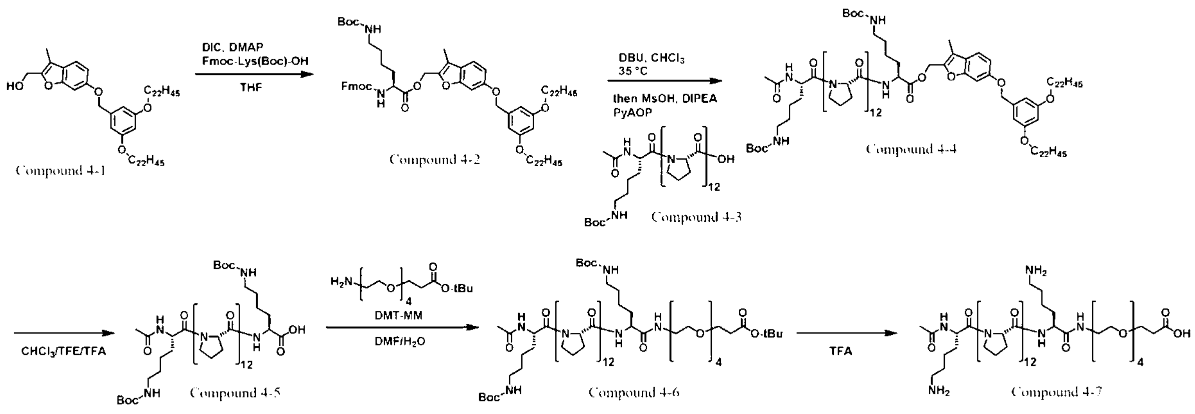


Compound 4-1

Compound 4-2

Compound 4-3

Compound 4-4

Compound 4-5

Compound 4-6

Compound 4-7

(i) Synthesis of compound (4-2)

300 mg of the compound (4-1) described in WO2020/175473A as the compound (4-1), 3 mL of tetrahydrofuran (THF), 229.8 mg of Nε-(tert-butoxycarbonyl)-Nα-[(9H-fluoren-9-ylmethoxy)carbonyl)]-L-lysine (Fmoc-Lys(Boc)-OH), 76.8 μL of diisopropylcarbodiimide, and 8.0 mg of 4-dimethylaminopyridine were placed in an eggplant flask having a capacity of 50 mL, and stirring was carried out at room temperature for 2 hours. Acetonitrile (30 mL) was added thereto to filter the precipitated solid, which was subsequently subjected to drying under reduced pressure to obtain 350 mg of a compound (4-2).

(ii) Synthesis of compound (4-3)

Solid phase peptide synthesis was carried out using H-Pro-Trt(2-Cl)-Resin (manufactured by Watanabe Chemical Industries, Ltd., 0.94 mmol/g, 53.2 mg) as a starting raw material. The elongation that was carried out by using N-(9-fluorenylmethoxycarbonyl)-L-proline (Fmoc-Pro-OH) was repeated for 11 cycles to elongate Nε-(tert-butoxycarbonyl)-Nα-[(9H-fluoren-9-ylmethoxy)carbonyl)]-L-lysine (Fmoc-Lys(Boc)-OH). Subsequently, an NMP solution of

piperidine (20% v/v) was added thereto to carry out a reaction for 20 minutes, thereby carrying out the deprotection of the Fmoc group, and then, an NMP solution of acetic anhydride (20% v/v) was added thereto to carry out a reaction for 10 minutes, thereby subjecting the N-terminal amino group to acetylation. After completion of the elongation, the resin was washed with dichloromethane, and then the solvent was distilled off under reduced pressure. 2.0 mL of a mixed solution of hexafluoro-2-propanol (HFIP):dichloromethane ($CH_2Cl_2$) = 1:4 was added thereto, and the peptide was cut out. After 30 minutes, the resin was filtered out to concentrate the filtrate, which was subsequently purified by reverse phase column chromatography to obtain 59.3 mg of a white solid compound (4-3).

(iii) Synthesis of compound (4-4)

20 mg of the compound (4-2), 400 μL of chloroform ($CHCl_3$), and 4.4 μL of 1,8-diazabicyclo[5.4.0]-7-undecene (DBU) were placed in an eggplant flask having a capacity of 10 mL, and stirring was carried out at 35°C for 1 hour. 1.9 μL of methanesulfonic acid (MsOH), 15.3 μL of N,N-diisopropylethylamine (DIPEA), 25.5 mg of the compound (4-3), and 23.1 mg of (7-azabenzotriazole-1-yloxy)tripyrrolidinophosphonium hexafluorophosphate (PyAOP) were placed therein, and stirring was carried out at 35°C for 1 hour. A solid precipitated by adding acetonitrile (4 mL) was subjected to filtration and drying under reduced pressure to obtain 34.0 mg of a compound (4-4).

(iv) Synthesis of compound (4-5)

30.0 mg of the compound (4-4), 600 μL of chloroform ($CHCl_3$), 60 μL of 2,2,2-trifluoroethanol (TFE), and 6 μL of trifluoroacetic acid (TFA) were placed in an eggplant flask having a capacity of 10 mL, and stirring was carried out at room temperature for 4 hours. Thereafter, the reaction solution was filtered, and 6 mL of methanol (MeOH) was added to the filtrate to generate a precipitate, which was subsequently centrifuged. The recovered compound was purified by preparative HPLC and subjected to freeze drying to obtain 16.5 mg of a compound (4-5).

(v) Synthesis of compound (4-6)

10 mg of the compound (4-5), 200 μL of water, 200 μL of N,N-dimethylformamide (DMF),3.9 mg of tert-butyl 1-amino-3,6,9,12-tetraoxadecane-15-ate, and 6.7 mg of 4-(4,6-dimethoxy-1,3,5-triazin-2-yl)-4-methylmorpholinium chloride (DMT-MM) were placed in an eggplant flask having a capacity of 10 mL, and stirring was carried out at room temperature for 2 hours. Thereafter, the reaction solution was concentrated and purified by preparative HPLC, and freeze drying was followed to obtain 8.9 mg of a compound (4-6).

(vi) Synthesis of compound (4-7)

7.0 mg of the compound (4-6) and 200 μL of trifluoroacetic acid (TFA) were placed in an eggplant flask having a capacity of 10 mL, and stirring was carried out at room temperature for 1 hour. Thereafter, the reaction solution was concentrated and purified by preparative HPLC, and freeze drying was followed to obtain 5.9 mg of a compound (4-7).

2) Synthesis of compound (M1-1)

A compound (M1-1) was synthesized based on the following scheme. It is noted that the compound (1-C) is the same as the compound (1-C) in the synthesis of a compound (M2-1) described above. The results of the MS measurement of the compound (M1-8) were as follows.
MS (ESI m/z): $(M + H^+)^+$ = 1,723, $(M - H^+)^-$ = 1,721

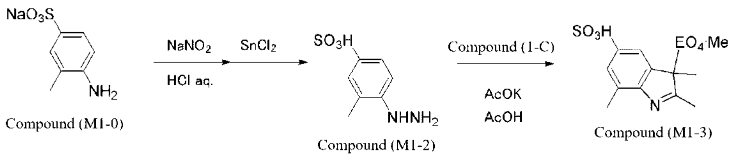

Compound (M1-0) Compound (M1-2) Compound (M1-3)

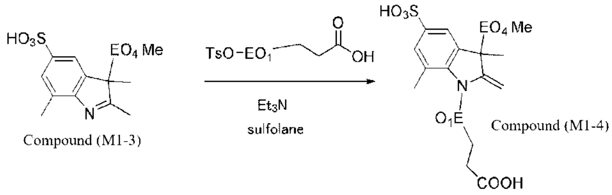


Compound (M1-3) Compound (M1-4)

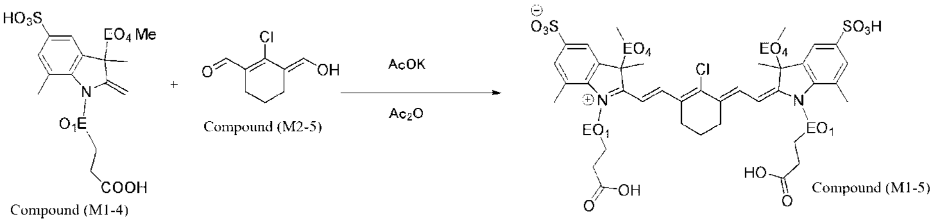


Compound (M1-4) Compound (M2-5) Compound (M1-5)

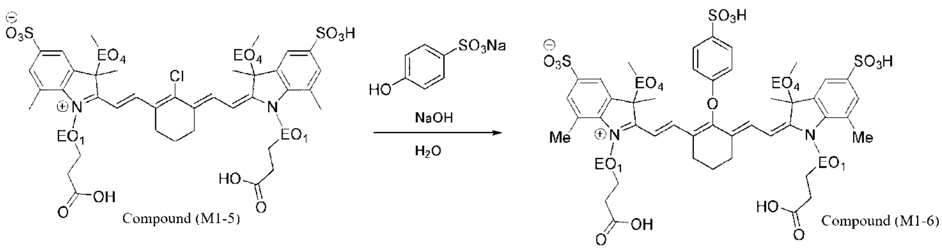


Compound (M1-5) Compound (M1-6)

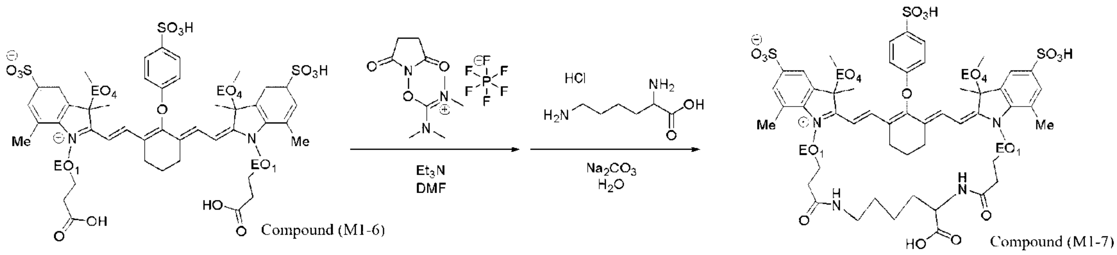


Compound (M1-6) Compound (M1-7)

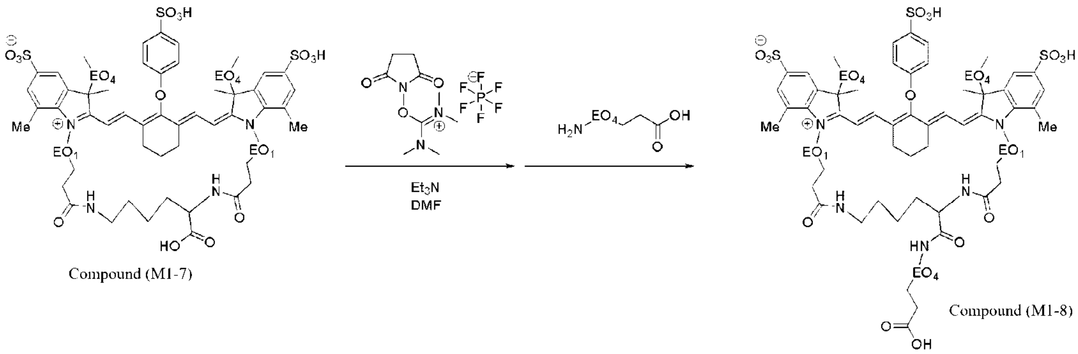


Compound (M1-7) Compound (M1-8)

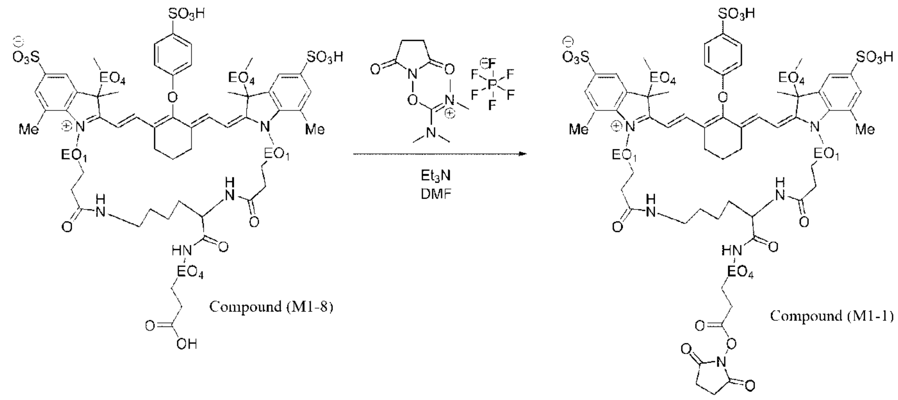


Compound (M1-8)

Compound (M1-1)

3) Synthesis of compound (4)

3.2 mg of a compound (4) was synthesized from 2.0 mg of the compound (4-7) in the same manner as the synthesis of the compound (1) according to the following scheme. The results of the MS measurement of the compound (4) were as follows.

MS (ESI m/z): $(M + H^+)^+ = 5{,}136$, $(M - H^+)^- = 5{,}134$

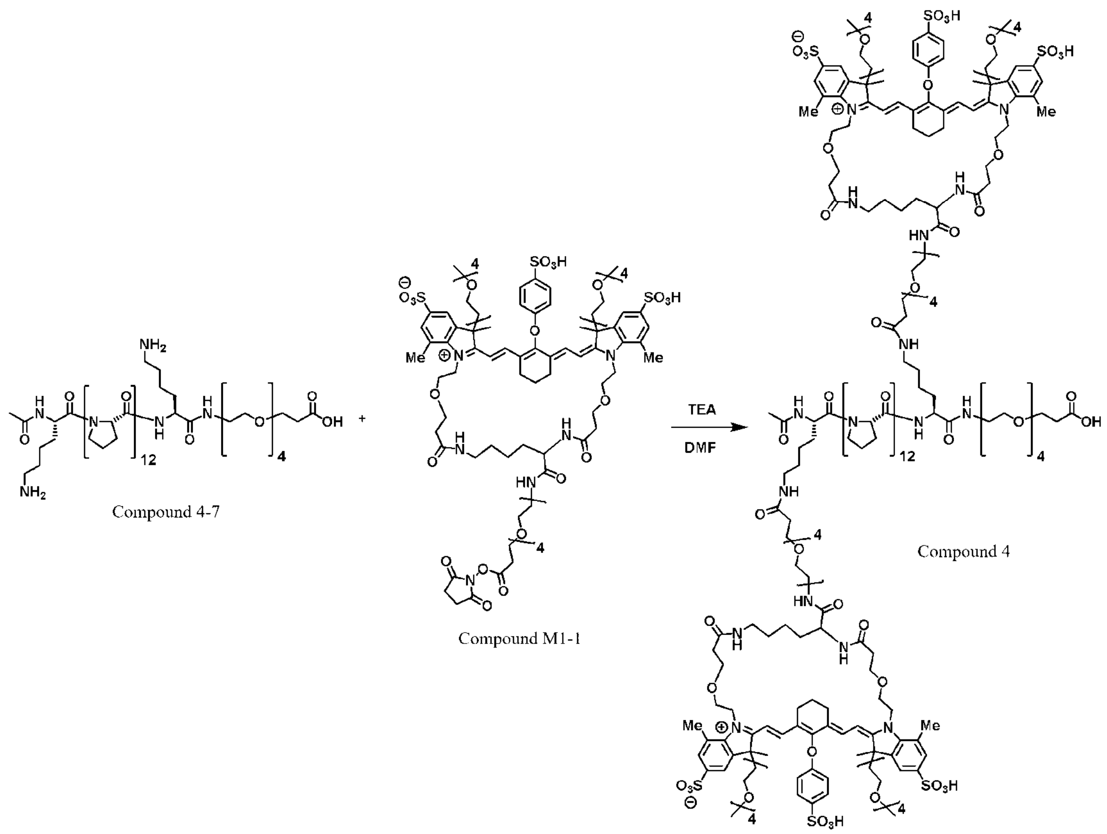


Compound 4-7

Compound M1-1

Compound 4

<Synthesis of Compounds (2-NHS), (3-NHS), and (4-NHS)>

The compound (2-NHS), the compound (3-NHS), and the compound (4-NHS) in which the terminal carboxy group in the peptide chain of the compound (2), the compound (3), or the compound (4) was converted into an NHS ester structure were synthesized in the same manner, except that in the synthesis of the above-described compound (1-NHS), the compound (2), the compound (3), or the compound (4) was used instead of the compound (1).

<Example 1>

The degree of fluorescence labeling and the fluorescence intensity in Western blotting were evaluated for each of the above-described compounds (1-NHS) to (4-NHS).

[1] Production of fluorescent dye-labeled antibody

104 μL of an anti-rabbit IgG antibody (2.3 mg/mL) and 10.4 μL of a carbonate buffer were added to a microtube, the resultant mixture was shaken and stirred. Then, a dimethyl sulfoxide solution of the compound (1-NHS) was added thereto such that the molar equivalent ratio (30 equivalent ratios) of the compound (1-NHS) with respect to 1 equivalent of the antibody was as shown in Table A, and further, the resultant mixture was shaken and stirred. After being allowed to stand at 4°C for 24 hours, the reaction solution was subjected to purification, by using a centrifugal ultrafiltration filter (product name: Amicon Ultra UFC 510096, manufactured by Merck KGaA) and a PBS solution (phosphate-buffered saline), to obtain an IgG antibody (2.0 mg/mL) labeled with the compound (1). The antibodies labeled with the compounds (2) to (4) were obtained in the same manner as above except that the compounds (2-NHS) to (4-NHS) were used instead of the compound (1-NHS) such that the molar equivalent ratios (20, 30, or 40 equivalent ratios) were shown in Table A. The degree of fluorescence labeling (DOL) of the obtained fluorescent dye-labeled antibody was calculated according to the method described below. The results are summarized in Table A.

As the method for calculating the degree of fluorescence labeling, such a general method as described below was used. The description in [ ] indicates a unit, and [-] means that there is no unit. In the present test, the protein means an anti-rabbit IgG antibody in the compounds (1) to (4).

$$\text{Degree of fluorescence labeling} = \text{fluorescent dye concentration}/\text{protein concentration}$$

The fluorescent dye concentration means the total molar concentration [M] of the labeled fluorescent dye, and the protein concentration means the molar concentration [M] of the fluorescently labeled protein. They are respectively calculated according to the following expressions.

$$\text{Fluorescent dye concentration} = \text{Dye}_{max}/\varepsilon_{dye}$$

$$\text{Protein concentration} = (\text{IgG}_{280} - (\text{Dye}_{max} \times \text{CF}))/\varepsilon_{protein}$$

Each symbol in the above expressions is as follows.

$Dye_{max}$: Absorption [-] of fluorescent dye at maximum absorption wavelength
$\varepsilon_{dye}$: Molar absorption coefficient [$M^{-1}cm^{-1}$] of fluorescent dye
$IgG_{280}$: Absorption [-] of fluorescently labeled protein at 280 nm
$Dye_{280}$: Absorption [-] of fluorescent dye at 280 nm
$\varepsilon_{protein}$: Molar absorption coefficient [$M^{-1}cm^{-1}$] of protein
Correction Factor (CF): $Dye_{280}/Dye_{max}$ [-]

[Table A]

| No. | Fluorescent dye-labeled antibody | 20 equivalent ratios | 30 equivalent ratios | 40 equivalent ratios |
|---|---|---|---|---|
| 001 | Compound (1)-IgG | - | 4.3 | - |
| 002 | Compound (2)-IgG | - | 5.5 | - |
| 003 | Compound (3)-IgG | - | - | 5.7 |
| 004 | Compound (4)-IgG | 5.8 | - | - |

(Note in table)

In the column of the fluorescent dye-labeled antibody, the notation of the compound (Z)-IgG means an IgG-antibody labeled with the compound (Z-NHS). Here, Z means the number of each compound. The same applies to the

following tables.

From the results in Table A above, the following facts can be seen.

The compounds (1) and (2) had degrees of fluorescence labeling of 4.3 and 5.5, in a case where the compounds were added at a molar equivalent ratio of 30 equivalents with respect to 1 equivalent of the antibody, the compound (3) had a degree of fluorescence labeling of 5.7 in a case where the compound was added at a molar equivalent ratio of 40 equivalents with respect to 1 equivalent of the antibody, and the compound (4) had a degree of fluorescence labeling of 5.8 in a case where the compound was added at a molar equivalent ratio of 20 equivalents with respect to 1 equivalent of the antibody, which indicated that the binding properties to the antibody were at a sufficient level without any practical problem.

[2] Evaluation of fluorescence intensity in Western blotting

(1) Production of dried membrane

Transferrin (manufactured by Merck KGaA, mass of about 76 kDa) was diluted with a Fluorescent Compatible Sample Buffer (4X, non-reducing) (manufactured by Thermo Fisher Scientific Inc.) to 1 ng/μL, 0.3 ng/μL, or 0.1 ng/μL, followed by heating treatment at 95°C for 5 minutes. The above-described transferrin sample and a PageRuler Prestained NIR Protein Ladder (manufactured by Thermo Fisher Scientific Inc.) were loaded on Novex 4-20% Tris-Glycine Mini Gels (manufactured by Thermo Fisher Scientific Inc.), and then electrophoresis was carried out at a constant voltage of 225 V for 45 minutes. The gel after electrophoresis and a nitrocellulose membrane (manufactured by Cytiva) were superimposed, protein transfer was carried out at a constant voltage of 12 V for 1 hour, and then the membrane was immersed in a Western Blot Blocking Buffer (Fish Gelatin) (manufactured by TAKARA Bio Inc.) and allowed to stand at 4°C for 12 hours. After washing with a TBS-T buffer (manufactured by FUJIFILM Wako Pure Chemical Corporation), the membrane was immersed in a solution containing a primary antibody for transferrin (manufactured by abcam.plc) (diluted 5,000 times), shaking was carried out for 1 hour, the solution containing the primary antibody was discarded, and then the membrane was washed with the TBS-T buffer (manufactured by FUJIFILM Wako Pure Chemical Corporation). A 25,000-fold diluted solution of the fluorescent dye-labeled antibody prepared above or a 25,000-fold diluted solution of a commercially available fluorescent secondary antibody described later was adjusted, the membrane was immersed in the adjusted solution and shaken for 1 hour in a light-shielded state, and the solution of the fluorescent dye-labeled antibody was discarded. Thereafter, the membrane was washed by repeating shaking with 20 mL of TBS-T (manufactured by FUJIFILM Wako Pure Chemical Corporation, Tween 20 content of 0.1 (W/V)%) for 10 minutes three times, and then washed by shaking with 20 mL of TBS in which the water-soluble compound shown in Tables 1 to 5 was dissolved at an adding amount shown in Tables 1 to 5, for 10 minutes once. After washing, the membrane was shielded from light for 1 hour in a constant-temperature tank at 40°C and then dried.

It is noted that (W/V)% means a mass-to-volume percentage.

(2) Measurement of fluorescence intensity

(i) Case where Compound (1)-IgG (diluted 25,000-fold) is used as fluorescent dye-labeled antibody

For the dried membrane produced above, Imaging was carried out using an Amersham Typhoon scanner (trade name, manufactured by Cytiva), and with excitation light of 785 nm under the uniform measurement conditions, the integrated value of the fluorescence intensity of a fraction of 3.24 $mm^2$ in a fluorescence wavelength range of 810 to 840 nm with respect to a region where the band of the protein was detected was measured and defined as a "signal fluorescence intensity". In addition, an integrated value of the fluorescence intensity of a fraction of 3.24 $mm^2$ in a fluorescence wavelength range of 810 to 840 nm in a region in which the band of the protein was not detected in the lower part of the measurement region of the signal fluorescence intensity (on the low mass side with respect to the measurement region of the signal fluorescence intensity) was measured and defined as a "noise fluorescence intensity".

Even in a case where the compound (4)-IgG or Alexa Fluor Plus 800 (trade name, manufactured by Thermo Fisher Scientific Inc., fluorescent secondary antibody) (both of which were diluted 25,000-fold) was used as the fluorescent dye-labeled antibody, the measurement was carried out in the same manner as in a case where the compound (1)-IgG (diluted 25,000-fold) was used.

(ii) Case where the compound (2)-IgG, the compound (3)-IgG, or Alexa Fluor Plus 680 (trade name, manufactured by Thermo Fisher Scientific Inc., fluorescent secondary antibody) (both of which were diluted 25,000-fold) was used as the fluorescent dye-labeled antibody

For the dried membrane produced above, Imaging was carried out using an Amersham Typhoon scanner (trade name, manufactured by Cytiva), and with excitation light of 685 nm under the uniform measurement conditions, the

integrated value of the fluorescence intensity of a fraction of 3.24 $mm^2$ in a fluorescence wavelength range of 710 to 730 nm with respect to a region where the band of the protein was detected was measured and defined as a "signal fluorescence intensity". In addition, an integrated value of the fluorescence intensity of a fraction of 3.24 $mm^2$ in a fluorescence wavelength range of 710 to 730 nm in a region in which the band of the protein was not detected in the lower part of the measurement region of the signal fluorescence intensity (on the low mass side with respect to the measurement region of the signal fluorescence intensity) was measured and defined as a "noise fluorescence intensity".

(3) Evaluation of fluorescence intensity

In each fluorescent dye-labeled antibody, the signal fluorescence intensity of No. c11, c21, c31, c41, c51, or c61, in which TBS containing no water-soluble compound was used for washing instead of TBS in which the water-soluble compound was dissolved, was set as a reference value, the ratio to this reference value (signal fluorescence intensity of fluorescent dye-labeled antibody/reference value) was calculated, and the evaluation was performed based on the following evaluation standard.

In addition, in each fluorescent dye-labeled antibody, the noise fluorescence intensity of No. c11, c21, c31, c41, c51, or c61, in which TBS containing no water-soluble compound was used for washing instead of TBS in which the water-soluble compound was dissolved, was set as a reference value, the ratio to this reference value (noise fluorescence intensity of fluorescent dye-labeled antibody/reference value) was calculated, and the evaluation was performed based on the following evaluation standard.

It is noted that in any of the evaluations, the evaluations were performed by comparing the state in which the dilution concentration of transferrin (manufactured by Merck KGaA) (1 ng/10 μL, 0.3 ng/10 μL, or 0.1 ng/10 μL) was uniform. There was no difference in the value of the ratio to the reference value depending on the dilution concentration of transferrin (manufactured by Merck KGaA).

The results are summarized in Tables 1 to 6.

- Evaluation standard for signal fluorescence intensity (integrated value) -

A: The ratio of signal fluorescence intensity to the reference value is 2.0 times or more.

B: The ratio of signal fluorescence intensity to the reference value is 1.9 times or more and less than 2.0 times.

C: The ratio of signal fluorescence intensity to the reference value is 1.8 times or more and less than 1.9 times.

D: The ratio of signal fluorescence intensity to the reference value is 1.7 times or more and less than 1.8 times.

E: The ratio of signal fluorescence intensity to the reference value is 1.6 times or more and less than 1.7 times.

F: The ratio of signal fluorescence intensity to the reference value is 1.5 times or more and less than 1.6 times.

G: The ratio of signal fluorescence intensity to the reference value is 1.4 times or more and less than 1.5 times.

H: The ratio of signal fluorescence intensity to the reference value is 1.3 times or more and less than 1.4 times.

I: The ratio of signal fluorescence intensity to the reference value is 1.2 times or more and less than 1.3 times.

J: The ratio of signal fluorescence intensity to the reference value is 1.1 times or more and less than 1.2 times.

- Evaluation standard for noise fluorescence intensity (integrated value) -

A: The ratio of noise fluorescence intensity to the reference value is less than 1.2 times.

B: The ratio of noise fluorescence intensity to the reference value is 1.2 times or more and less than 1.3 times.

C: The ratio of noise fluorescence intensity to the reference value is 1.3 times or more and less than 1.4 times.

D: The ratio of noise fluorescence intensity to the reference value is 1.4 times or more and less than 1.5 times.

E: The ratio of noise fluorescence intensity to the reference value is 1.5 times or more and less than 1.6 times.

F: The ratio of noise fluorescence intensity to the reference value is 1.6 times or more and less than 1.7 times.

[Table 1]

| No. | Fluorescent dye-labeled antibody | Water-soluble compound | Melting point | Adding amount [wt%] | Signal fluorescence intensity | Noise fluorescence intensity |
|---|---|---|---|---|---|---|
| 101 | Compound (2)-IgG | Polyoxyethylene polyoxypropylene glycol (160 E.O.) (30 P.O.) | 55°C | 20 | H | C |
| 102 | | Propionic acid amide | 78°C | | G | A |
| 103 | | D-sorbitol | 95°C | | E | A |
| 104 | | Trehalose dihydrate | 97°C | | E | A |
| 105 | | Ribitol | 103°C | | E | A |
| 106 | | Resorcinol | 111°C | | E | B |
| 107 | | n-Butylamide | 115°C | | E | E |
| 108 | | D-mannitol | 166°C | | E | A |
| 109 | | Sucrose | 186°C | | E | A |
| 110 | | Polyvinylpyrrolidone K30 | 225°C | | C | F |
| 111 | | Betaine | 310°C | | E | A |
| 112 | | Dextran 40000 | 483°C | | E | A |
| 113 | | Polysucrose 400 | 200°C or higher | | E | A |
| 114 | | Sodium 1-naphthalenesulfonate | 300°C | | I | A |
| 115 | | Sodium benzenesulfonate | 450°C | | I | A |
| 116 | | D-sorbitol/betaine = 1:1 | 95°C/310°C | | E | A |
| c11 | | - | - | - | 1.0 (Reference value) | 1.0 (Reference value) |
| c12 | | 1,6-Hexanediol | 41°C | 20 | 1.0 | 1.0 |

[Table 2]

| No. | Fluorescent dye-labeled antibody | Water-soluble compound | Adding amount [wt%] | Content [wt%] | Signal fluorescence intensity | Noise fluorescence intensity |
|---|---|---|---|---|---|---|
| 201 | Compound (3)-IgG | D-sorbitol | 3 | 2.9 | J | A |
| 202 | | | 5 | 4.8 | G | A |
| 203 | | | 10 | 9.0 | F | A |
| 204 | | | 20 | 16.7 | E | A |
| 205 | | | 30 | 23.1 | B | B |
| 206 | | | 40 | 28.6 | A | B |
| 207 | | | 50 | 33.3 | A | B |
| c21 | | - | - | - | 1.0 (Reference value) | 1.0 (Reference value) |

[Table 3]

| No. | Fluorescent dye-labeled antibody | Water-soluble compound | Adding amount [wt%] | Signal fluorescence intensity | Noise fluorescence intensity |
|---|---|---|---|---|---|
| 301 | Compound (1)-IgG | D-sorbitol | 10 | C | A |
| c31 | | - | - | 1.0 (Reference value) | 1.0 (Reference value) |

[Table 4]

| No. | Fluorescent dye-labeled antibody | Water-soluble compound | Adding amount [wt%] | Signal fluorescence intensity | Noise fluorescence intensity |
|---|---|---|---|---|---|
| 401 | Compound (4)-IgG | D-sorbitol | 10 | C | A |
| c41 | | - | - | 1.0 (Reference value) | 1.0 (Reference value) |

[Table 5]

| No. | Fluorescent dye-labeled antibody | Water-soluble compound | Adding amount [wt%] | Signal fluorescence intensity | Noise fluorescence intensity |
|---|---|---|---|---|---|
| 501 | Alexa Fluor Plus 800 | D-sorbitol | 10 | D | A |
| c51 | | - | - | 1.0 (Reference value) | 1.0 (Reference value) |

[Table 6]

| No. | Fluorescent dye-labeled antibody | Water-soluble compound | Adding amount [wt%] | Signal fluorescence intensity | Noise fluorescence intensity |
|---|---|---|---|---|---|
| 601 | Alexa Fluor Plus 680 | D-sorbitol | 10 | H | A |
| c61 | | - | - | 1.0 (Reference value) | 1.0 (Reference value) |

<Note in table>

(Fluorescent dye-labeled antibody)

Compound (Z)-IgG: it is the same as the above-described compound (Z)-IgG

Alexa Fluor Plus 680: trade name, manufactured by Thermo Fisher Scientific Inc., fluorescent secondary antibody

Alexa Fluor Plus 800: trade name, manufactured by Thermo Fisher Scientific Inc., fluorescent secondary antibody

(Water-soluble compound)

Polyoxyethylene polyoxypropylene glycol (160 E.O.) (30 P.O.): trade name, manufactured by FUJIFILM Wako Pure Chemical Corporation

Resorcinol: trade name, manufactured by FUJIFILM Wako Pure Chemical Corporation, 1,3-dihydroxybenzene

Polyvinylpyrrolidone K30: trade name, manufactured by FUJIFILM Wako Pure Chemical Corporation

Betaine: trade name, manufactured by FUJIFILM Wako Pure Chemical Corporation, trimethylglycine

Dextran 40000: trade name, manufactured by FUJIFILM Wako Pure Chemical Corporation

Polysucrose 400: trade name, manufactured by FUJIFILM Wako Pure Chemical Corporation, water-soluble polymer obtained by copolymerization of sucrose and epichlorohydrin

It is noted that all the compounds described in the column of water-soluble compound are compounds which dissolve in an amount of 1 g or more in 100 mL of water under a condition of 25°C.

Adding amount: means a proportion (displayed as 100%) of an adding amount of the water-soluble compound to the TBS solution before the addition of the water-soluble compound, based on the mass, and a unit thereof is % by mass (wt%).

Content: means a proportion (displayed as 100%) of a content of a water-soluble compound in a water-soluble compound-containing TBS solution based on the mass, and a unit thereof is % by mass (wt%).

Melting point: measured as follows by simultaneous thermogravimetric and differential thermal measurement (TG-DTA).

As a simultaneous thermogravimetric and differential thermal measurement device, a simultaneous differential thermal and thermogravimetric measurement device (model number: STA7200) manufactured by Hitachi High-Tech Science Corporation was used.

First, the measurement sample was held until the device was stabilized at the expected temperature of 30°C, then heated to 200°C at a heating rate of 20°C/min, a TG curve and a DTA curve were created.

The temperature at an intersection point between the straight line obtained by extending the baseline on the low temperature side to the high temperature side and a tangent line drawn at a point where the gradient of the curve of the melting point in a stepwise change section in the DTA curve was defined as the melting point.

It is noted that for water-soluble compounds other than polysucrose 400 among the water-soluble compounds having a melting point of higher than 200°C, a value measured by changing the upper limit temperature of heating from 200°C to a temperature at which the melting point can be measured is described.

From the results in Tables 1 to 6 above, the following facts can be seen.

In Comparative Example of No. c12 in which TBS containing 1,6-hexanediol which is a water-soluble compound having a melting point of 41°C was used for washing the membrane, both the signal fluorescence intensity and the noise fluorescence intensity were the same value as those in No. c11 (standard) in which TBS containing no water-soluble compound was used for washing the membrane, and the effect of enhancing the fluorescence intensity was not obtained.

On the other hand, in Examples of Nos. 101 to 116 in which a water-soluble compound having a melting point of 50°C or higher was used, the signal fluorescence intensity was enhanced by at least 1.2 times or more and the increase in noise fluorescence intensity was suppressed to 1.7 times or less as compared with No. c11 (standard) in which TBS containing no water-soluble compound was used for washing the membrane, the increase in signal fluorescence intensity was large with respect to the increase in noise fluorescence intensity, and an excellent effect of enhancing fluorescence intensity was exhibited. Similarly, even in a case where a different fluorescent dye-labeled antibody or a commercially available fluorescent secondary antibody was used, in the examples in which the water-soluble compound having a melting point of 50°C or higher was used, an excellent effect of enhancing fluorescence intensity (Nos. 201 to 207 with

respect to No. c21 (standard), No. 301 with respect to No. c31 (standard), No. 401 with respect to No. c41 (standard), No. 501 with respect to No. c51 (standard), and No. 601 with respect to No. c61 (standard)) as compared with the standard in which TBS containing no water-soluble compound was used for washing the membrane.

Among these, it can be seen that in a case where the water-soluble compound does not contain a salt structure, the signal fluorescence intensity can be further increased (see Nos. 101 to 113 and 116 with respect to Nos. 114 and 115), in a case where the melting point of the water-soluble compound is 80°C or higher, the signal fluorescence intensity can be further increased (see Nos. 103 to 113 and 116 with respect to Nos. 101 and 102), and in a case where the water-soluble compound is a polyol compound or a betaine compound, the noise fluorescence intensity can be suppressed (see Nos. 103 to 106, 108, 109, 111 to 113, and 116 with respect to Nos. 107 and 110).

In addition, it can be seen that in a case where the content of the water-soluble compound is 4.5% by mass or more, the signal fluorescence intensity can be further increased (see Nos. 202 to 207 with respect to No. 201), and in a case where the content of the water-soluble compound is 20% by mass or more, the signal fluorescence intensity can be further increased (see Nos. 205 to 207 with respect to Nos. 201 to 204).

Although the present invention has been described with reference to the embodiments, it is the intention of the inventors of the present invention that the present invention should not be limited by any of the details of the description unless otherwise specified and rather be construed broadly within the spirit and scope of the invention appended in WHAT IS CLAIMED IS.

The present application claims the priority of JP2022-209083A filed in Japan on December 26, 2022, the contents of which are incorporated herein by reference, as a part of the description of the present specification.

## Claims

**1.** A fluorescence intensity enhancer comprising:
a water-soluble compound having a melting point of 50°C or higher.

**2.** The fluorescence intensity enhancer according to claim 1,
wherein a content of the water-soluble compound is 4.5% by mass or more.

**3.** The fluorescence intensity enhancer according to claim 1,
wherein the water-soluble compound does not contain a salt structure.

**4.** The fluorescence intensity enhancer according to claim 1,
wherein the water-soluble compound is a polyol compound or a betaine compound.

**5.** The fluorescence intensity enhancer according to claim 2,
wherein the content of the water-soluble compound is 20% by mass or more.

**6.** The fluorescence intensity enhancer according to claim 1,
wherein the melting point is 80°C or higher.

**7.** The fluorescence intensity enhancer according to claim 1,
wherein the fluorescence intensity enhancer is in a solution form.

**8.** The fluorescence intensity enhancer according to claim 1,
wherein the fluorescence intensity enhancer is used for fluorescence imaging.

**9.** A fluorescence intensity enhancing method of a fluorescently labeled target biological substance, the fluorescence intensity enhancing method comprising:
allowing the fluorescence intensity enhancer according to any one of claims 1 to 8 to act on a fluorescently labeled target biological substance.

**10.** A fluorescence intensity enhancing method of a fluorescently labeled target biological substance, the fluorescence intensity enhancing method comprising:
allowing the fluorescence intensity enhancer according to any one of claims 1 to 8 to act on a fluorescently labeled target biological substance to enhance a fluorescence intensity from a fluorescence label.

**11.** A kit for fluorescence detection, comprising:

(a) a fluorescence intensity enhancer containing a water-soluble compound having a melting point of 50°C or higher; and
(b) a reagent for producing a fluorescent dye-labeled biological molecule, which contains a fluorescent dye, or a fluorescent dye-labeled biomolecule.

**INTERNATIONAL SEARCH REPORT**

International application No.
**PCT/JP2023/046400**

**A. CLASSIFICATION OF SUBJECT MATTER**

***G01N 21/78***(2006.01)i
FI: G01N21/78 C

According to International Patent Classification (IPC) or to both national classification and IPC

**B. FIELDS SEARCHED**

Minimum documentation searched (classification system followed by classification symbols)

G01N21/62-21/83

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2017/010152 A1 (SONY CORPORATION) 19 January 2017 (2017-01-19) paragraphs [0015], [0023], [0024], [0030] | 1-11 |
| A | JP 2012-103014 A (TOKYO METROPOLITAN INSTITUTE OF MEDICAL SCIENCE) 31 May 2012 (2012-05-31) | 1-11 |
| A | JP 2014-231488 A (KATAYAMA KAGAKU KOGYO KK) 11 December 2014 (2014-12-11) | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 March 2024** | **12 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/046400**

| Patent document cited in search report | Publication date (day/month/year) | Patent family member(s) | Publication date (day/month/year) |
| --- | --- | --- | --- |
| WO 2017/010152 A1 | 19 January 2017 | US 2018/0202933 A1<br>paragraphs [0050], [0051], [0062], [0063], [0073]<br>EP 3324174 A1 | |
| JP 2012-103014 A | 31 May 2012 | (Family: none) | |
| JP 2014-231488 A | 11 December 2014 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2016534147 A
- JP 2022501402 A
- JP 5133957 A
- JP H05133957 A
- WO 2019230963 A
- WO 2021100814 A
- WO 2021125295 A
- WO 2021215514 A
- WO 2022025210 A
- WO 2022191123 A
- JP 2019172826 A
- JP 2022131357 A
- JP 2021020956 A
- JP 2020105187 A
- JP 2021011483 A
- WO 2002026891 A
- WO 2018174078 A
- WO 2020175473 A
- JP 2022209083 A

### Non-patent literature cited in the description

- **GREG T. HERMANSON**. Bioconjugate Techniques
- **LUCAS C. D. DE REZENDE** ; **FLAVIO DA SILVA EMERY.** A Review of the Synthetic Strategies for the Development of BODIPY Dyes for Conjugation with Proteins. *Orbital: The Electronic Journal of Chemistry*, 2013, vol. 5 (1), 62-83